# EUROPÄISCHE PATENTSCHRIFT

(11) **EP 0 590 520 B1**
(45) Veröffentlichungstag und Bekanntmachung des Hinweises auf die Patenterteilung: **12.06.1996**
(21) Anmeldenummer: 93115361.3
(22) Anmeldetag: 23.09.1993
(51) Int. Cl.: C07D 213/69, A61K 31/44

(54) **Acylsulfonamido-und Sulfonamidopyridin-2-carbonsäureester sowie ihre Pyridin-N-oxide, Verfahren zur ihrer Herstellung und ihre Verwendung als Arzneimittel**
Acylsulphonamido and sulphonamidopyridine-2-carboxylic acid esters, pyridine-n-oxides thereof, a process for their preparation and their use as medicaments
Esters carboxliques d'acylsulfonamides et de 2- pyridine- sulfonamides ainsi que les dérivés pyridiniques n- oxydes, un procédé pour leur préparation et leur utilisation comme médicament

(30) Priorität: 02.10.1992 DE 4233124
(43) Veröffentlichungstag der Anmeldung: 06.04.1994
(73) Patentinhaber: HOECHST AKTIENGESELLSCHAFT, D-65929 Frankfurt am Main (DE)
(72) Erfinder: Weidmann, Klaus Dr., D-61476 Kronberg/Taunus (DE); Bickel, Martin Dr., D-61348 Bad Homburg (DE); Gunzler-Pukall, Volkmar Dr., D-35039 Marburg (DE); Baringhaus, Karl-Heinz Dr., D-65835 Liederbach (DE)

(56) Entgegenhaltungen:
- EP-A- 0 176 741
- EP-A- 0 278 452
- EP-A- 0 278 453
- EP-A- 0 278 454
- EP-A- 0 281 943
- EP-A- 0 463 592
- EP-A- 0 479 177
- J.MED.CHEM,1992,35,pp800-804

## Beschreibung

Die Erfindung betrifft Acylsulfonamido- und Sulfonamidopyridin-2-carbonsäureester und ihre Pyridin-N-oxide sowie ihre Verwendung als Arzneimittel gegen fibrotische Erkrankungen.

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma] sowie die Atherosklerose.

Es ist bekannt, daß das Enzym Prolinhydroxylase durch Pyridin-2,4- und -2,5-dicarbonsäure effektiv gehemmt wird (K. Majamaa et al., Eur. J. Biochem. 138 (1984) 239 bis 245). Diese Verbindungen sind in der Zellkultur allerdings nur in sehr hohen Konzentrationen als Hemmstoffe wirksam (Tschank, G. et al., Biochem. J. 238 (1987) 625 bis 633).

In der DE-A 34 32 094 werden Pyridin-2,4- und -2,5-dicarbonsäurediester mit 1 bis 6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die die Kollagenbiosynthese im Tiermodell wirksam hemmen.

In der EP-A-0 463 592 sind 2,4 und 2,5 Diamide des Pyrdin-N-oxids und in der EP-A-0 281 943 2,4-Diester des Pyridins mit einem zusätzlichen Substituenten in Position 5 offenbart, die als Inhibitoren der Prolyl-4-hydroxylase wirken.

Ebenso werden 5-Acylsulfonamide des 2-Carboxy-Pyridins als Inhibitoren der Prolyl-4-hydroxylase beschrieben (J. Med. Chem. 1992, 35, 800-804).

Es bestand nunmehr die Aufgabe nach Verbindungen zu suchen, die stärker antifibrotisch sind als die bisher bekannten Verbindungen.

Gelöst wird die Aufgabe durch das Bereitstellen von Acylsulfonamido- und Sulfonamidopyridin-2-carbonsäurestern sowie ihrer Pyridin-N-oxide der allgemeinen Formel I 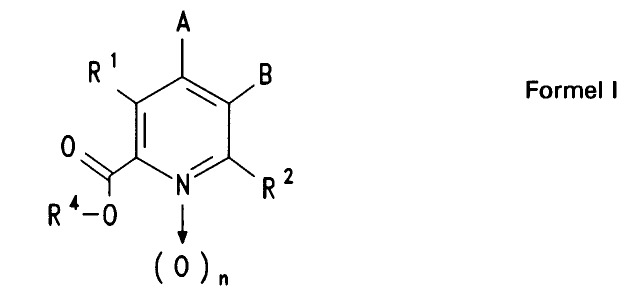
worin
A = R³ und B = X-NR⁵R⁶ oder
B = R³ und A = X-NR⁵R⁶ und
- X: eine Einfachbindung oder -CO-
bedeutet und
- R¹, R² und R³: gleich oder verschieden sind und Wasserstoff, (C₁-C₆)-Alkyl, (C₁-C₆)-Alkoxy, Halogen, insbesondere Fluor, Chlor oder Brom, Nitril, Hydroxy, Amino, ggf. mono- oder disubstituiert mit (C₁-C₄)-Alkyl, Hydroxy-(C₁-C₄)-alkyl, (C₁-C₆)-Alkylcarbonyloxy,
- R⁴: den Rest eines Alkohols R⁴OH bedeutet, worin R⁴ insbesondere (C₁-C₁₀)-Acyloxy-(C₁-C₆)-alkyl, vorzugsweise (C₁-C₁₀)-Alkanoyloxy-(C₁-C₆)-alkyl, Benzyloxy-(C₁-C₆]-alkyl, Benzyloxycarbonyloxy-(C₁-C₆)-alkyl oder Alkoxycarbonyloxy-(C₁-C₆)-alkyl, einen verzweigten oder unverzweigten oder cyclischen aliphatischen (C₁-C₁₆)-Alkyl-Rest, oder einen verzweigten oder unverzweigten ggf. cyclischen (C₁-C₁₆)-Alkenyl-Rest, einen (C₁-C₁₆)-Alkinyl-Rest, oder einen (C₁-C₁₆)-Alkeninyl-Rest bedeutet, die jeweils eine oder mehrere Mehrfachbindungen enthalten können,
einen (C₆-C₁₆)-Aryl-Rest, einen (C₇-C₁₆)-Aralkyl-Rest oder einen 5-oder 6-gliedrigen, vorzugsweise Stickstoff-enthaltenden Heteroaryl-oder einen 5- oder 6-gliedrigen, vorzugsweise Stickstoff-enthaltenden Heteroaralkyl-Rest bedeutet, wobei die vorstehenden Reste insbesondere einen oder mehrere Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂]ₓC_{f}H_{(2f + 1-g)}F_{g}, -OCF₂Cl, - OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-6₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)Aralkyloxy-(C₁-C₁₀-)alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy,
N-((C₁-C₁₀)-Alkoxy-(C₁-C₁₀)-alkyl)carbamoyloxy,
N-((C₆-C₁₆)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-Alkoxy-(C₁-C₁₀)-alkyl)carbamoyloxy,
N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido und N-((C₁-C₁₀)-alkyl-(C₇-C₁₆)-Aralkylsulfonamido, tragen, wobei die Reste, die einen Arylrest enthalten ihrerseits am Aryl substituiert sein können durch 1 bis 5 gleiche oder verschiedene Reste aus der Reihe: Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkoy,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl`
N-(C₁-C₁₀)-Alkyl-N-((C₆-C₁₆)-Aryloxy-(C₁-C₁₀)-alkyl)carbamoyl`
N-(C₁-C₁₀)-Alkyl-N-((C₇-C₁₆)Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy,
N-((C₁-C₁₀)-Alkoxy-(C₁-C₁₀)-alkyl)carbamoyloxy,
N-((C₆-C₁₆)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-Alkoxy-(C₁-C₁₀)-alkyl)carbamoyloxy,
N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀]-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl und (C₇-C₁₆)-Aralkylsulfonyl,
- R⁵: Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1-3fach substituiert sein kann mit Hydroxy oder (C₁-C₄)-Alkoxy, oder ein Kation eines basischen Aminosäurederivates bedeutet
- R⁶: einen Rest der Formel II ausgenommen -SO₂H bedeutet

Y-[C-U]ᵣ-D-W (II)

in welchem
- Y: -SO₂- oder -CO- bedeutet,
- C: eine Bindung oder
einen verzweigten oder unverzweigten aliphatischen (C₁-C₁₆)-Alkandiyl- oder cycloaliphatischen (C₃-C₁₀)-Alkandiylrest oder einen verzweigten oder unverzweigten (C₂-C₁₆)-Alkendiyl- oder Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
- U: eine Bindung oder
Wasserstoff oder
einen Rest aus der Reihe folgender Heteroatomgruppierungen bedeutet
-CO-, -O(CO)-, -(CO)-O-, -(CO)NR-, -NR(CO)-, -O-, -SO-, - SO₂-, -NR, worin R (C₁-C₃)-Alkyl oder Wasserstoff bedeutet,
- r: 1, 2, 3 oder 4 ist,
- D: eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest bedeutet, der jeweils eine oder mehrere C-C-Mehrfachbindungen enthalten kann,
- W: eine Bindung oder
Wasserstoff oder
einen (C₃-C₁₀) cycloaliphatischen Alkyl-, Alkenyl-, Alkinyl-oder Alkeninyl-Rest oder
einen (C₆-C₁₆)-Arylrest oder einen 5- oder 6-gliedrigen Heteroarylrest bedeutet, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
- C, D und/oder W,: sofern diese keine Bindung oder Wasserstoff bedeuten, vorzugsweise ihrerseits substituiert sind durch eine Kombination von bis zu 5 gleichen oder verschiedenen Substituenten aus der Reihe Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy
N-((C₁-C₁₀)-Alkoxy-(C₁-C₁₀)-alkyl)carbamoyloxy,
N-((C₆-C₁₆)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-Alkoxy-(C₁-C₁₀)-alkyl)carbamoyloxy,
N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₁₀)-Alkyl-(C₇-C₁₀)-Aralkylamino, N-(C₁-C₁₀)-Alkyl-N(C₆-C₁₂)-Arylamino, (C₁-C₁₂)-Alkoxy-amino, (C₁-C₁₂)-Alkoxy-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂]-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido,
N-((C₁-C₁₀)-alkyl-(C₇-C₁₆)-Aralkylsulfonamido,
wobei die Reste, die einen Arylrest enthalten, ihrerseits am Aryl substituiert sein können mit 1, 2, 3, 4 oder 5 gleichen oder verschiedenen Substituenten aus der Reihe, Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋ ]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂]-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy
N-((C₁-C₁₀)-Alkoxy-(C₁-C₁₀)-alkyl)carbamoyloxy,
N-((C₆-C₁₆)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-Alkoxy-(C₁-C₁₀)-alkyl)carbamoyloxy,
N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₁₀)-Alkyl-N-(C₇-C₁₆)-Aralkylamino, N-(C₁-C₁₀)-Alkyl-N-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆]-Aroylamino-(C₁-C₈)-alkyl, (C₇-C₁₆)-Aralkanoylamino-(C₁-C₈)-alkyl, Amino-(C₁-C₁₀]-alkyl, N-(C₁-C₁₀)-alkylamino-(C₁-C₁₀]-alkyl, N,N-Di-(C₁-C₁₀)-alkylamino-(C₁-C₁₀)-alkyl, (C₃-C₈)-Cycloalkylamino-(C₁-C₁₀)alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl,
(C₃-C₈)-cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido, N-((C₁-C₁₀)-alkyl-(C₇-C₁₆)-Aralkylsulfonamido,
und
- n: 0 oder 1,
- f: 1 bis 8, vorzugsweise 1 bis 5,
- g: 0, 1 bis (2f+1) und
- x: 0 bis 8, vorzugsweise 0 oder 1 bedeuten, ausgenommen
5-[((Methylsulfonyl)amino)carbonyl]-pyridin-2-carbonsäuremethylester,
5-[((2-Propylsulfonyl)amino)carbonyl]-pyridin-2-carbonsäuremethylester,
5-[((Phenylsulfonyl)amino)carbonyl]-pyridin-2-carbonsäuremethylester,
5-[((Benzylsulfonyl)amino)carbonyl]-pyridin-2-carbonsäuremethylester,
5-[((4-Methoxyphenylsulfonyl)amino)carbonyl]-pyridin-2-carbonsäuremethylester,
5-[((1-Naphthylsulfonyl)amino)carbonyl]-pyridin-2-carbonsäuremethylester,
5-[((4,5-Dibrom-2-thienylsulfonyl)amino)carbonyl]-pyridin-2-carbonsäuremethylester,
5-[((5-Chlor-2-thienylsulfonyl)amino)carbonyl]-pyridin-2-carbonsäuremethylester,
5-[((8-Chinolylsulfonyl)amino)carbonyl]-pyridin-2-carbonsäuremethylester,
5-[((4-(2-(4,7-Dichlorchinolyl))phenyl-sulfonyl)amino)carbonyl]-pyridin-2-carbonsäuremethylester.

Unter Aryl-, Aryloxy-, Heteroaryl- bzw. Heteroaryloxyverbindungen versteht man insbesondere Phenyl-, Biphenyl- oder Naphtyl- bzw. unsubstituierte 5- und 6-gliedrige heteroaromatische Ringe mit 1, 2 oder 3 Stickstoff- und/oder Sauerstoff- und/oder Schwefelatomen, wie Pyridyl-, Pyridazyl-, Pyrimidyl-, Pyrazyl-, Imidazolyl-, Triazolyl-, Thienyl-, Oxazolyl- und Thiazolyl-Derivate, und deren benzoannellierte Derivate.

Bevorzugt sind Verbindungen der Formel I, in denen
- X: eine Einfachbindung oder -CO- ist,
- R¹, R² und R³: gleich oder verschieden sind und Wasserstoff, (C₁-C₆)-Alkyl, (C₁-C₆)-Alkoxy, Halogen, insbesondere Fluor oder Chlor, Hydroxy, Amino,
- R⁴: einen Rest eines Alkohols R⁴OH bedeutet, worin R⁴ (C₁-C₁₀]-Acyloxy-(C₁-C₆)-alkyl, vorzugsweise (C₁-C₁₀)-Alkanoyloxy-(C₁-C₆)-alkyl, Benzoyloxy-(C₁-C₆]-alkyl, Benzyloxycarbonyloxy-(C₁-C₆)-alkyl oder (C₁-C₆)-Alkoxycarbonyloxy-(C₁-C₆)-alkyl,
einen verzweigten oder unverzweigten aliphatischen oder cycloaliphatischen (C₁-C₁₂)-Alkyl-Rest,
einen verzweigten oder unverzweigten (C₁-C₁₂)-Alkenyl-Rest, einen (C₂-C₁₂)-Alkinyl-Rest, oder einen (C₂-C₁₂)-Alkeninyl-Rest bedeutet, der jeweils eine oder mehrere Mehrfachbindungen enthalten kann, oder
einen (C₆-C₁₆)-Aryl-Rest, einen (C₆-C₁₆)-Aralkyl-Rest, einen Heteroaryl-oder einen Heteroaralkyl-Rest bedeutet,
wobei die vorstehenden Reste einen oder zwei Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-Aryloxy, (C₇-C₁₆)-Aralkyloxy, (C₁-C₈)-Hydroxyalkyl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy,
N-((C₁-C₁₀)-Alkoxy-(C₁-C₁₀)-alkyl)carbamoyloxy,
N-((C₆-C₁₆)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-Alkoxy-(C₁-C₁₀)-alkyl)carbamoyloxy,
N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, N-(C₇-C₁₁)-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino und (C₇-C₁₁)-Aralkanoyl-N-(C₁-C₁₀)-alkylamino tragen können, und
wobei die Reste, die einen Arylrest enthalten, ihrerseits im Arylteil substituiert sein können durch 1 bis 5 gleiche oder verschiedene Reste der Reihe
Hydroxy, Halogen, Cyano, Trifluormethyl, (C₁-C₁₂)-Alkyl, (C₃-C₈)-Cycloalkyl, (C₁-C₁₂)-Alkoxy, (C₁-C₁₂)-Alkoxy-(C₁-C₁₂)-alkoxy,
(C₁-C₁₂)-Alkylcarbonyl, (C₃-C₈)-Cycloalkylcarbonyl,
(C₁-C₁₂)-Alkoxycarbonyl, (C₁-C₁₂)-Alkoxy-(C₁-C₁₂)-alkoxycarbonyl, (C₃-C₈)-Cycloalkoxycarbonyl,
(C₁-C₁₂)-Alkylcarbonyloxy, (C₃-C₈)-Cycloalkylcarbonyloxy,
(C₁-C₁₂)-Alkoxycarbonyloxy, (C₁-C₁₂)-Alkoxy-(C₁-C₁₂)-alkoxycarbonyloxy, (C₃-C₈)-Cycloalkoxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl,
N-((C₁-C₁₀)-Alkoxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy,
Amino, (C₁-C₁₂)-Alkylamino, Di-(C₁-C₁₂)-alkylamino, (C₃-C₈)-Cycloalkylamino,
(C₁-C₁₂)-Alkanoylamino, (C₃-C₈)-Cycloalkanoylamino,
(C₁-C₁₂-Alkanoylamino-(C₁-C₈)-alkyl, (C₃-C₈)-Cycloalkanoylamino-(C₁-C₈)-alkyl,
(C₁-C₁₂)Alkylmercapto, (C₁-C₁₂)-Alkylsulfinyl und (C₁-C₁₂)-Alkylsulfonyl,
- R⁵: Wasserstoff, (C₁-C₃)-Alkyl, (C₁-C₄)-Alkanoyl oder ein 1,2 oder 3-wertiges physiologisch verwendbares Kation, insbesondere Na⊕, K⊕, Mg²⊕, Ca²⊕ oder ein Ammoniumion, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1-3fach substituiert sein kann mit Hydroxy und/oder (C₁-C₄)-Alkoxy, oder ein Kation eines basischen Aminosäurederivates bedeutet,
- R⁶: einen Rest der Formel II ausgenommen -SO₂H bedeutet

-Y-[C-U]ᵣ-D-W (II)

in welchem
- Y: -SO₂- bedeutet,
- C: eine Bindung, oder einen verzweigten oder unverzweigten aliphatischen (C₁-C₁₂)-Alkandiyl-rest, oder
einen verzweigten oder unverzweigten (C₂-C₁₂)-Alkendiyl-Rest, einen (C₂-C₁₂)-Alkindiyl-Rest oder einen (C₂-C₁₂)-Alkenindiyl-Rest bedeutet, der eine oder mehrere C-C-Mehrfachbindungen enthalten kann,
- U: eine Bindung oder
Wasserstoff oder einen Rest aus der Reihe folgender Heteroatomgruppierungen bedeutet
-(CO)NR-, -NR(CO)-, -O-,-SO-, -SO₂-, worin R (C₂-C₃)-Alkyl oder Wasserstoff bedeutet,
- r: 1 oder 2 ist,
- D: eine Bindung oder
Wasserstoff oder
einen verzweigten oder unverzweigten aliphatischen (C₁-C₈)-Alkandiyl-Rest, oder
einen verzweigten oder unverzweigten (C₂-C₈)-Alkendiyl-Rest oder (C₂-C₈)-Alkindiyl-Rest bedeutet und
- W: eine Bindung oder
Wasserstoff oder
einen (C₃-C₁₀) cycloaliphatischen Alkyl-, Alkenyl-, Alkinyl-oder Alkeninyl-Rest oder
einen (C₆-C₁₆)-Arylrest oder einen 5- oder 6-gliedrigen Heteroarylrest bedeutet, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
- C, D und/oder W,: sofern diese keine Bindung oder Wasserstoff bedeuten, vorzugsweise ihrerseits substituiert sind durch eine Kombination von bis zu 5 gleichen oder verschiedenen Substituenten aus der Reihe
Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g})F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₅)-Alkyl-N-(C₇-C₁₀)-Aralkylamino, N-(C₁-C₅)-Alkyl-N-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
wobei die Reste, die einen Arylrest enthalten, ihrerseits am Aryl substituiert sein können mit 1, 2, 3, 4 oder 5 gleichen oder verschiedenen Substituenten aus der Reihe Wasserstoff, Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl, (C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₅)-Alkyl-(C₇-C₁₀)-Aralkylamino, N-(C₁-C₅)-Alkyl-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl ausgenommen die Verbindungen:
5-[((Methylsulfonyl)amino)carbonyl]-pyridin-2-carbonsäuremethylester,
5-[((2-Propylsulfonyl)amino)carbonyl]-pyridin-2-carbonsäuremethylester,
5-[((Phenylsulfonyl)amino)carbonyl]-pyridin-2-carbonsäuremethylester,
5-[((Benzylsulfonyl)amino)carbonyl]-pyridin-2-carbonsäuremethylester,
5-[((4-Methoxyphenylsulfonyl)amino]carbonyl]-pyridin-2-carbonsäuremethylester,
5-[((1-Naphthylsulfonyl)amino)carbonyl]-pyridin-2-carbonsäuremethylester,
5-[((4,5-Dibrom-2-thienylsulfonyl)amino)carbonyl]-pyridin-2-carbonsäuremethylester,
5-[((5-Chlor-2-thienylsulfonyl)amino)carbonyl]-pyridin-2-carbonsäuremethylester,
5-[((8-Chinolylsulfonyl)amino)carbonyl]-pyridin-2-carbonsäuremethylester,
5-[((4-(2-(4,7-Dichlorchinolyl))phenyl-sulfonyl)amino]carbonyl]-pyridin-2-carbonsäuremethylester.

Besonders bevorzugt sind Verbindungen der allgemeinen Formel I, in der
- X: eine Einfachbindung oder -CO- ist
- R¹, R² und R³: gleich oder verschieden sind und Wasserstoff oder (C₁-C₃)-Alkyl, (C₁-C₃)-Alkoxy, Hydroxy, Fluor oder Chlor,
- R⁴: den Rest eines Alkohols R⁴OH bedeutet, worin R⁴ (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl,
einen verzweigten oder unverzweigten oder cyclischen aliphatischen (C₁-C₁₀)-Alkyl-Rest, oder
einen verzweigten oder unverzweigten (C₂-C₁₀)-Alkenyl-Rest oder einen (C₂-C₁₂)-Alkinyl-Rest, der jeweils eine oder mehrere C-C-Mehrfachbindungen enthalten kann, oder
einen (C₆-C₁₆)-Aryl-Rest, einen (C₇-C₁₁)-Aralkyl-Rest oder einen Heteroaryl- oder Heteroalkyl-Rest bedeutet,
wobei die vorstehenden Reste einen oder zwei Substituenten aus der Reihe
Hydroxy, (C₁-C₆)-Alkoxy, (C₁-C₆)-Alkoxy-(C₁-C₆)-alkoxy, (C₆-C₁₂)-Aryloxy, (C₇-C₁₂)-Aralkyloxy,
(C₁-C₈)-Alkylcarbonyl, (C₃-C₈)-Cycloalkylcarbonyl, (C₆-C₁₂)-Arylcarbonyl, (C₇-C₁₂)-Aralkylcarbonyl,
(C₁-C₈)-Alkoxycarbonyl, (C₁-C₆)-Alkoxy-(C₁-C₆)-alkoxycarbonyl, (C₆-C₁₂)-Aryloxycarbonyl, (C₇-C₁₂)-Aralkoxycarbonyl, (C₃-C₈)-Cycloalkoxycarbonyl,
(C₁-C₈)-Alkylcarbonyloxy, (C₃-C₈)-Cycloalkylcarbonyloxy, (C₆-C₁₂)-Arylcarbonyloxy, (C₇-C₁₂)-Aralkylcarbonyloxy,
(C₁-C₈)-Alkoxycarbonyloxy, (C₁-C₆)-Alkoxy-(C₁-C₆)-alkoxycarbonyloxy, (C₆-C₁₂)-Aryloxycarbonyloxy, (C₇-C₁₂)-Aralkyloxycarbonyloxy, (C₃-C₈)-Cycloalkoxycarbonyloxy,
Carbamoyl, N-(C₁-C₈)-Alkylcarbamoyl, N,N-Di-(C₁-C₈)-alkylcarbamoyl, N-(C₃-C₈)-Cycloalkylcarbamoyl,
N-((C₁-C₆)-Alkoxy-(C₁-C₆)alkyl)carbamoyl,
Amino, (C₁-C₆)-Alkylamino, Di-(C₁-C₆)-alkylamino, (C₃-C₈)-Cycloalkylamino, N-(C₆-C₁₂)-Arylamino, N-(C₇-C₁₁)-Aralkylamino, N-(C₁-C₅)-Alkyl-(C₆-C₁₂)-Arylamino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₆)alkylamino, (C₇-C₁₁)-Aralkanoyl-N-(C₁-C₆)-alkylamino tragen können, und
wobei die Reste, die einen Arylrest enthalten, insbesondere substituiert sind mit bis zu 3 Substituenten aus der Reihe
Hydroxy, Fluor, Chlor, Cyano, Trifluormethyl, (C₁-C₆)-Alkyl, (C₃-C₈)-Cycloalkyl, (C₁-C₈)-Alkoxy,
(C₁-C₆)-Alkylcarbonyl, (C₃-C₈)-Cycloalkylcarbonyl,
(C₁-C₆)-Alkoxycarbonyl, (C₃-C₈)-Cycloalkoxycarbonyl,
(C₁-C₆)-Alkylcarbonyloxy, (C₃-C₈)-Cycloalkylcarbonyloxy,
(C₁-C₆)-Alkoxycarbonyloxy, (C₃-C₈)-Cycloalkoxycarbamoyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl,
N-((C₁-C₆)-Alkoxy-(C₁-C₆)-alkyl)carbamoyl,
N-(C₁-C₆)-Alkyl-N-((C₁-C₆)-Alkoxy-(C₁-C₆)-alkyl)carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyloxy,
Amino, (C₁-C₆)-Alkylamino, Di-(C₁-C₆)-alkylamino, (C₃-C₈)-Cycloalkylamino,
(C₁-C₆)-Alkanoylamino, (C₃-C₈)-Cycloalkanoylamino,
(C₁-C₆)Alkylmercapto, (C₁-C₆)-Alkylsulfinyl, (C₁-C₆)-Alkylsulfonyl, und
- R⁵: Wasserstoff, (C₁-C₃)-Alkyl, (C₁-C₄)-Alkanoyl oder ein 1,2 oder 3-wertiges physiologisch verwendbares Kation, insbesondere Na⊕, K⊕, Mg²⊕, Ca²⊕ oder ein Ammoniumion bedeutet, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1-3fach substituiert sein kann mit Hydroxy und/oder (C₁-C₄)-Alkoxy,
- R⁶: einen Rest der Formel II ausgenommen -SO₂H bedeutet,

-Y-[C-U]ᵣ-D-W (II)

in welchem
- Y: -SO₂- bedeutet,
- C: eine Bindung oder
einen (C₁-C₆)-Alkandiyl-Rest bedeutet,
- U: eine Bindung oder
Wasserstoff oder -O- bedeutet,
- r: 1 ist,
- D: eine Bindung oder
Wasserstoff oder
einen unverzweigten aliphatischen (C₁-C₈)-Alkandiyl-Rest bedeutet, und
- W: eine Bindung oder
Wasserstoff, einen (C₆-C₁₂)-Arylrest oder einen 5- oder 6-gliedrigen Heteroarylrest bedeutet, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
- C, D und/oder W,: sofern diese keine Bindung oder Wasserstoff bedeuten, vorzugsweise ihrerseits substituiert sind mit bis zu 3 gleichen oder verschiedenen Substituenten aus der Reihe Hydroxy, Halogen, Cyano, Trifluormethyl, Nitro, Carboxyl, (C₁-C₈)-Alkyl, (C₃-C₈)-Cycloalkyl, (C₆-C₁₂)-Aryl, (C₇-C₁₆)-Aralkyl, (C₃-C₈)-Alkenyl, (C₃-C₈)-Alkinyl, (C₁-C₈)-Alkoxy, (C₁-C₈)-Alkoxy-(C₁-C₈)-alkyl, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g},
(C₁-C₈)-Alkoxycarbonyl, (C₆-C₁₂)-Aryloxycarbonyl, (C₇-C₁₄)-Aralkoxycarbonyl, (C₃-C₈)-Cycloalkoxycarbonyl,
(C₁-C₈)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Amino, (C₁-C₈)-Alkylamino, Di-(C₁-C₈)-alkylamino, (C₃-C₈)-Cycloalkylamino, N-(C₆-C₁₂]-Arylamino, N-(C₇-C₁₁)-Aralkylamino, N-(C₁-C₃)-Alkyl-N-(C₇-C₁₁)-Aralkylamino,
(C₁-C₁₀)-Alkanoylamino, (C₃-C₈)-Cycloalkanoylamino, (C₆-C₁₂)-Aroylamino, (C₇-C₁₆)-Aralkanoylamino, (C₁-C₁₀)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₆)-alkyl, (C₃-C₈)-Cycloalkanoylamino-(C₁-C₈)-alkyl, (C₆-C₁₆)-Aroylamino-(C₁-C₆)-alkyl, (C₇-C₁₆)-Aralkanoylamino-(C₁-C₆)-alkyl,
(C₁-C₈)-Alkylmercapto, (C₁-C₈)-Alkylsulfinyl, (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₄)-Aralkylmercapto, (C₇-C₁₄)-Aralkylsulfinyl, (C₇-C₁₄)-Aralkylsulfonyl,
wobei die Reste, die einen Arylrest enthalten, ihrerseits am Aryl substituiert sein können mit 1, 2, 3, 4 oder 5 gleichen oder verschiedenen Substituenten aus der Reihe Wasserstoff, Hydroxy, Halogen, Cyano, Trifluormethyl, Nitro, Carboxyl, (C₁-C₈)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₈)-Alkoxy, (C₁-C₆)-Alkoxy-(C₁-C₆)-alkyl, (C₆-C₁₂)-Aryloxy, (C₇-C₁₆)-Aralkyloxy, -O-[CH₂₋]_{X}C_{f}H_{(2f+1-g)}F_{g},
(C₁-C₁₂)-Alkoxycarbonyl, (C₃-C₈)-Cycloalkoxycarbonyl,
(C₁-C₁₂)-Alkylcarbonyloxy, (C₃-C₈)-Cycloalkylcarbonyloxy,
Carbamoyl, N-(C₁-C₈)-Alkylcarbamoyl, N,N-Di-(C₁-C₈)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₈)-Alkyl-N-(C₆-C₁₆)-arylcarbamoyl, N-(C₁-C₈)-Alkyl-N-(C₇-C₆)-Aralkylcarbamoyl,
N-((C₁-C₆)-Alkoxy-(C₁-C₆)-alkyl)carbamoyl,
N-((C₆-C₆)-Aryloxy-(C₁-C₆)-alkyl)carbamoyl,
N-((C₇-C₁₆)-Aralkyloxy-(C₁-C₆)-alkyl)carbamoyl,
N-(C₁-C₈)-Alkyl-N-((C₁-C₆)-Alkoxy-(C₁-C₆)-alkyl)carbamoyl,
N-(C₁-C₈)-Alkyl-N-((C₆-C₁₆)-Aryloxy-(C₁-C₆)-alkyl)carbamoyl,
N-(C₁-C₈)-Alkyl-N-((C₇-C₁₆)-Aralkyloxy-(C₁-C₆)-alkyl)carbamoyl,
Amino, (C₁-C₆)-Alkylamino, Di-(C₁-C₆)-alkylamino, (C₃-C₈)-Cycloalkylamino, N-(C₆-C₁₂)-Arylamino, N-(C₇-C₁₁)-Aralkylamino, N-(C₁-C₃)-Alkyl-(C₇-C₁₁)-Aralkylamino, N-(C₁-C₃)-Alkyl-(C₆-C₁₂)-Arylamino, (C₁-C₈)-Alkoxy-amino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₁₀)-alkylamino, (C₇-C₁₁)-Aralkanoyl-N-(C₁-C₆)-alkylamino,
(C₁-C₈)-Alkanoylamino-(C₁-C₄)-alkyl, (C₃-C₈)-Cycloalkanoylamino-(C₁-C₄)alkyl, (C₆-C₁₂)-Aroylamino-(C₁-C₄)-alkyl und (C₇-C₁₂)-Aralkanoylamino-(C₁-C₄)-alkyl,
ausgenommen die Verbindungen:
5-[((Methylsulfonyl)amino)carbonyl]-pyridin-2-carbonsäuremethylester,
5-[((2-Propylsulfonyl)amino)carbonyl]-pyridin-2-carbonsäuremethylester,
5-[((Phenylsulfonyl)amino)carbonyl]-pyridin-2-carbonsäuremethylester,
5-[((Benzylsulfonyl)amino)carbonyl]-pyridin-2-carbonsäuremethylester,
5-[((4-Methoxyphenylsulfonyl)amino)carbonyl]-pyridin-2-carbonsäuremethylester,
5-[((1-Naphthylsulfonyl)amino)carbonyl]-pyridin-2-carbonsäuremethylester,
5-[((4,5-Dibrom-2-thienylsulfonyl)amino)carbonyl]-pyridin-2-carbonsäuremethylester,
5-[((5-Chlor-2-thienylsulfonyl)amino)carbonyl]-pyridin-2-carbonsäuremethylester,
5-[((8-Chinolylsulfonyl)amino)carbonyl]-pyridin-2-carbonsäuremethylester,
5-[((4-(2-(4,7-Dichlorchinolyl))phenyl-sulfonyl)amino)carbonyl]-pyridin-2-carbonsäuremethylester.

Ganz besonders bevorzugt sind Verbindungen der Formel I, worin
- X: eine Einfachbindung oder -CO- ist,
- R¹, R² und R³: Wasserstoff bedeuten,
- R⁴: den Rest eines Alkohols R⁴OH bedeutet und einen verzweigten oder unverzweigten oder cyclischen aliphatischen (C₁-C₉)-Alkyl-Rest,
einen verzweigten oder unverzweigten (C₁-C₈)-Alkenylrest oder (C₁-C₈)-Alkinylrest bedeutet, einen Phenyl-, Benzyl-, Phenethyl-, Phenylpropylrest,
wobei die vorstehenden Reste einen Substituenten aus der Reihe Wasserstoff, Hydroxy, (C₁-C₄)-Alkoxy,
(C₁-C₆)-Alkoxycarbonyl, (C₆-C₁₂)-Phenoxycarbonyl, (C₇-C₁₆)-Phenylalkylcarbonyl, (C₃-C₈)-Cycloalkoxycarbonyl,
(C₁-C₆)-Alkylcarbonyloxy, (C₃-C₈)-Cycloalkylcarbonyloxy, Benzoyloxy, (C₇-C₁₆)-Phenylalkylcarbonyloxy, (C₃-C₈)-Cycloalkoxycarbonyloxy enthalten,
(C₁-C₆)-Alkoxycarbonyloxy, Phenoxycarbonyloxy, (C₇-C₁₆)-Phenylalkylcarbonyloxy und (C₃-C₈)-Cycloalkoxycarbonyloxy,
- R⁵: Wasserstoff oder ein 1, 2 oder 3-wertiges physiologisch verwendbares Kation bedeutet, insbesondere Na⊕, K⊕, Mg²⊕, Ca²⊕ oder H₃N^{⊕}C(CH₂OH)₃ (Tris-Salz),
- R⁶: einen Rest der Formel II ausgenommen -SO₂H bedeutet

-Y-[C-U]ᵣ-D-W (II)

in welchem
- Y: -SO₂- bedeutet,
- C: eine Bindung oder
(C₁-C₄)-Alkandiyl bedeutet,
- U: eine Bindung, Wasserstoff oder
-O- bedeutet,
- r: 1 ist,
- D: eine Bindung, Wasserstoff oder
(C₁-C₄)-Alkandiyl,
- W: eine Bindung, Wasserstoff oder
einen Phenylrest bedeutet, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
- C, D und/oder W: substituiert sind durch Wasserstoff oder von 1 oder 2 Substituenten aus der folgenden Reihe stehen
Fluor, Chlor, (C₁-C₆)-Alkyl, Phenyl, (C₁-C₆)-Alkoxy, Phenoxy, -O-[CH₂₋]_{X}C_{f}H_{(2f+1-g)}F_{g},
Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl, N-(C₇-C₁₆)-Phenylalkylcarbamoyl, N-(C₁-C₈)-Alkyl-N-(C₆-C₁₆)-phenylcarbamoyl, N-(C₁-C₈)-Alkyl-N-(C₇-C₁₆)-Phenylalkylcarbamoyl,
N-((C₁-C₄)-Alkoxy-(C₁-C₈)-alkyl)carbamoyl,
N-Phenoxy-(C₁-C₈)-alkyl)carbamoyl,
N-((C₇-C₁₆)-Phenylalkyloxy-(C₁-C₈)-alkyl)carbamoyl,
N-(C₁-C₈)-Alkyl-N-((C₁-C₆)-Alkoxy-(C₁-C₈)-alkyl)carbamoyl,
N-(C₁-C₈)-Alkyl-N-((C₆-C₁₂)-Phenoxy-(C₁-C₈)-alkyl)carbamoyl,
N-(C₁-C₈)-Alkyl-N-((C₇-C₁₆)-Phenylalkyloxy-(C₁-C₈)-alkyl)carbamoyl,
(C₁-C₈)-Alkanoylamino, (C₃-C₈)-Cycloalkanoylamino, (C₆-C₁₂)-Phenylmino, (C₇-C₁₁)-Phenylalkanoylamino, (C₁-C₈)-Alkanoyl-N-(C₁-C₁₀)-alkylamino, (C₃-C₈)-Cycloalkanoyl-N-(C₁-C₆)-alkylamino, Benzoyl-N-(C₁-C₁₀)-alkylamino, (C₇-C₁₁)-Phenylalkanoyl-N-(C₁-C₆)-alkylamino,
(C₁-C₁₀)-Alkanoylamino-(C₁-C₂)-alkyl, (C₃-C₈)-Cycloalkanoylamino-(C₁-C₂)alkyl, Benzoylamino-(C₁-C₂)-alkyl, (C₇-C₁₄)-Phenylalkanoylamino-(C₁-C₂)-alkyl, wobei die Reste, die einen Arylrest enthalten, ihrerseits substituiert sind mit einem Substituenten aus der Reihe
Wasserstoff, Hydroxy, Fluor, Chlor, Trifluormethyl, (C₁-C₆)-Alkyl, (C₁-C₈)-Alkoxy,
- n: 0,
- f: 1 bis 5,
- g: 0, 1 bis (2f+1) und
- x: 0 oder 1
bedeuten, ausgenommen die Verbindungen
5-[((Methylsulfonyl)amino)carbonyl]-pyridin-2-carbonsäuremethylester,
5-[((2-Propylsulfonyl)amino)carbonyl]-pyridin-2-carbonsäuremethylester,
5-[((Phenylsulfonyl)amino)carbonyl]-pyridin-2-carbonsäuremethylester,
5-[((Benzylsulfonyl)amino)carbonyl]-pyridin-2-carbonsäuremethylester,
5-[((4-Methoxyphenylsulfonyl)amino)carbonyl]-pyridin-2-carbonsäuremethylester,
5-[((1-Naphthylsulfonyl)amino)carbonyl]-pyridin-2-carbonsäuremethylester,
5-[((4,5-Dibrom-2-thienylsulfonyl)amino)carbonyl]-pyridin-2-carbonsäuremethylester,
5-[((5-Chlor-2-thienylsulfonyl)amino)carbonyl]-pyridin-2-carbonsäuremethylester,
5-[((8-Chinolylsulfonyl)amino)carbonyl]-pyridin-2-carbonsäuremethylester,
5-[((4-(2-(4,7-Dichlorchinolyl))phenylsulfonyl)amino)carbonyl]-pyridin-2-carbonsäuremethylester.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen.

Schließlich betrifft die Erfindung die Verbindungen der allgemeinen Formel I zur Verwendung als Arzneimittel.

Insbesondere betrifft die Erfindung die Verbindungen der Formel I zur Anwendung als Fibrosuppressiva.

Weiterhin betrifft die Erfindung ein Verfahren zur Herstellung von Verbindungen der allgemeinen Formel I.

Weiterhin betrifft die Erfindung ein Verfahren zur Herstellung von Verbindungen der allgemeinen Formel I (im einzelnen Verbindungen der Formeln 3 und 8 bzw. ihren N-Oxiden 3' und 8'):
a) Verbindungen, in denen X eine Einfachbindung bedeutet, werden hergestellt wie folgt (Schema I): 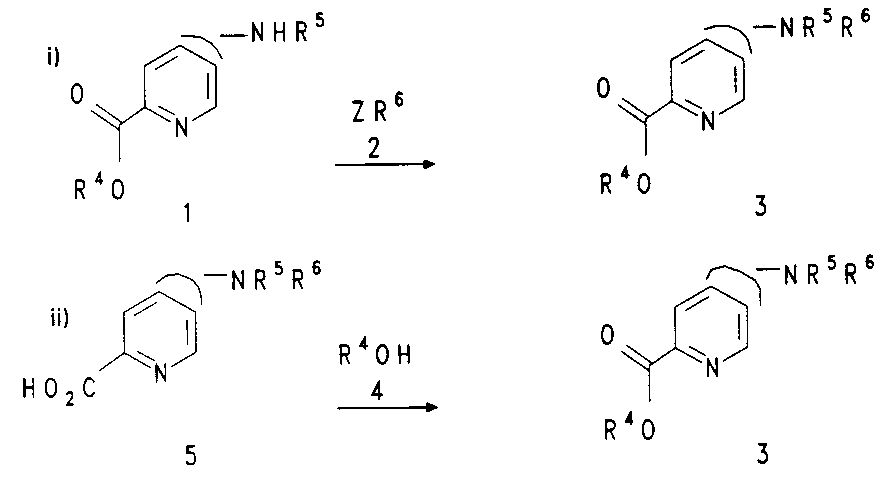 Das Schema ist sowohl gültig für 2,4 disubstituierte Pyridinderivate der Formel I, wie auch für die 2,5-Derivate mit allen Substituenten für R¹, R² und R³.
b) Verbindungen, in denen X -CO- bedeutet, werden wie folgt hergestellt (Schema II): 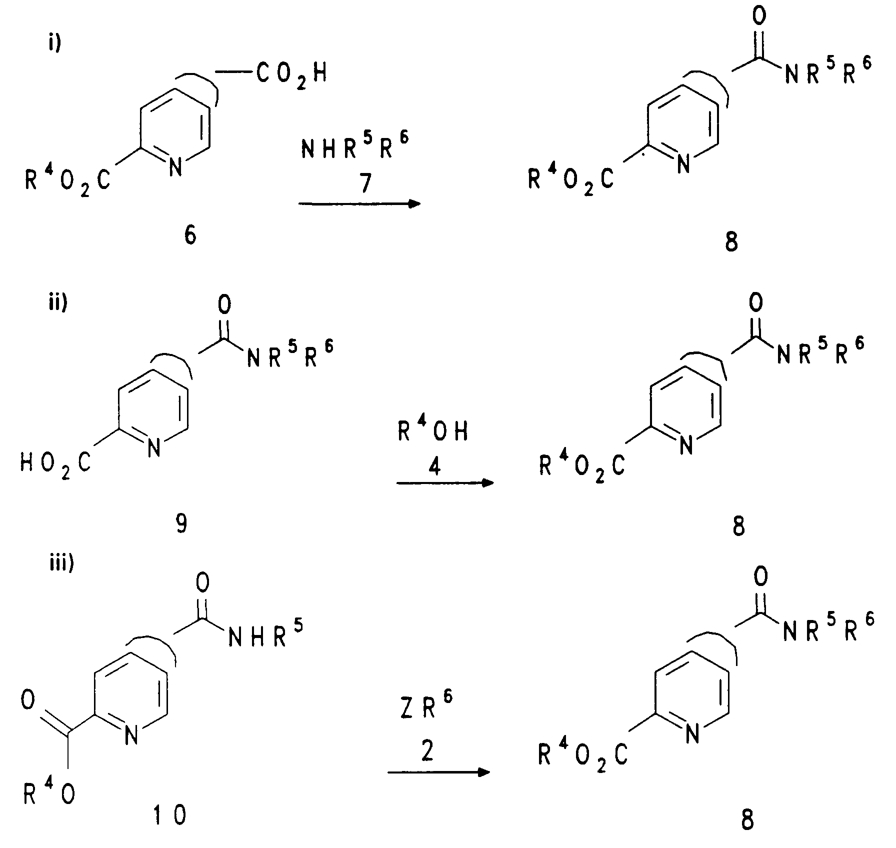 Das Schema ist sowohl gültig für 2,4-disubstituierte Pyridinderivate der Formel I, wie auch für die 2,5-Derivate mit allen Substituenten für R¹, R² und R³.

### Zu Schema I (Verfahren a)

i) 5-Aminopyridin-2-carboxylate der Formel 1 werden mit Verbindungen der Formel 2, in denen Z für Hydroxy oder für eine Abgangsgruppe steht, die nukleophil ablösbar ist und insbesondere F, Cl, Br, J oder Tosylat bedeutet, zu Verbindungen der Formel 3 umgesetzt. Diese Umsetzung erfolgt in einem dipolar aprotischen organischen Lösungsmittel oder einem Lösemittelgemisch. Insbesondere sind folgende Lösemittel genannt: Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin, gegebenenfalls unter Zusatz eines Säurebinders, wie Ammoniak, Triethylamin, Tributylamin, bei einer Reaktonstemperatur von 0° bis 180°C, vorzugsweise von 0° bis 80°C. Sofern Z für Hydroxy steht, handelt es sich um eine Kondensationsmethode, die aus der Peptidchemie bekannt ist (vgl. auch Methode B).
ii) Die Verbindungen der Formel 5 werden nach dem Fachmann bekannten und üblichen Methoden mit einem Alkohol der Formel 4 zu Verbindungen der Formel 3 verestert. Diese Methode wird insbesondere dann angewendet, wenn R⁴ in den Verbindungen der Formel 3, die aus dem Verfahren i) resultieren, Niederalkyl, insbesondere Methyl, bedeutet. In diesem Fall werden zuerst diese Verbindungen der Formel 3 zu den Verbindungen der Formel 5 verseift und anschließend mit Alkoholen der Formel 4, in der R⁴ nicht Niederalkyl, insbesondere Methyl, bedeutet, umgesetzt.
   Durch Verseifen der Verbindungen der Formel 1 lassen sich die Ausgangsverbindungen für Verfahren ii), Verbindungen der Formel 5, herstellen.
   Die Verbindungen der Formel 3 können weiterhin nach dem Fachmann bekannten Methoden in die Pyridin-N-oxide 3' übergeführt werden.
   Eine allgemeine Vorschrift dieser Oxidationsmethode ist in "E. Klingsberg, Pyridine and its Derivitives, Interscience Publishers, New York, 1961, Part 2, 93'' beschrieben. Die Oxidation mit Wasserstoffperoxid ist beispielsweise in "E. Ochiai, J. Org. Chem. 18, 534 (1953)" beschrieben. Die Verfahrensbedingungen können im Detail der deutschen Patentanmeldung P 38 26 471.4, 38 28 140.6, 39 24 093.2, 40 01 002.3 sowie den DE-A-37 03 959, 37 03 962 und 37 03 963 entnommen werden.
   Die Herstellung der literaturbekannten Verbindungen der Formel 1 wird von N. Finch et al., J. Med. Che. (1978), Vol. 21, Seite 1269 und Schneider and Harris, J. Org. Chem. (1984), Vol. 49, Seite 3683, beschrieben.

### Zu Schema II (Verfahren b)

i) Aus substituierten Pyridin-2,5-dicarbonsäuren (siehe CA: Vol. 68, 1968, 68840 h) können unter Veresterungsbedingungen die Pyridin-2-carbonsäure-5-carboxylate der Formel 6 hergestellt werden. Geeignete Bedingungen sind z. B. die Veresterung mit Methanol in Gegenwart von Schwefelsäure, wobei die Reaktionszeit so zu wählen ist, daß die vollständige Veresterung zum Diesterprodukt nur untergeordnet stattfindet, bzw. die Diesterprodukte als Nebenprodukte abgetrennt werden können.
   Die Herstellung der Verbindungen der Formel 8 erfolgt aus den Verbindungen der Formel 6 und den Amidderivaten der Formel 7, wobei es zweckmäßig sein kann, beide Reaktanden mit Hilfsreagenzien zu aktivieren (Houben-Weyl: Methoden der Organischen Chemie, Band IX, Kapitel 19, Seiten 636 bis 637).
   An Reagenzien zur Carbonsäurereaktivierung können die dem Fachmann bekannten Substanzen, wie Thionylchlorid, Oxalylchlorid, Pivaloylchlorid oder Chlorameisensäureester-Derivate Verwendung finden. Es ist nicht immer notwendig, diese aktivierten Derivate der Verbindungen der Formel 7 zu isolieren. Meist ist es zweckmäßig, sie nach Herstellung in situ oder als Rohprodukte mit den Sulfonamidderivaten der Formel 8 umzusetzen. Zweckmäßigerweise werden die Verbindungen der Formel 7 zunächst mit einer anorganischen oder organischen Base, wie z. B. Natrium- oder Kaliumhydroxid, -carbonat, -alkoxid, -hydrid, -amid, Ammoniak, Triethylamin, Tributylamin, Pyridin bei -20° bis + 150°C, vorzugsweise bei 0° bis -80°C zur Reaktion gebracht und dieses Reaktionsgemisch mit einer Verbindung der Formel 6 oder dessen aktivierter Form umgesetzt. Die Umsetzung erfolgt in einem inerten Lösungsmittel, wie z. B. Methylenchlorid, Methanol, Ethanol, Aceton, Essigsäureethylester, Toluol, Tetrahydrofuran, Acetonitril, N,N-Dimethylformamid, N,N-Dimethylacetamid, Nitromethan, Dimethylsulfoxid oder Gemischen dieser Lösungsmittel.
ii) Die Verbindungen der Formel 9 werden nach dem Fachmann bekannten und üblichen Methoden mit einem Alkohol der Formel 4 zu Verbindungen der Formel 8 verestert (siehe Methode a ii). Auch in diesem Fall wird diese Methode insbesondere dann angewendet, wenn R⁴ in den Verbindungen der Formel 8, die aus dem Verfahren i) resultieren, Niederalkyl, insbesondere Methyl, bedeutet. In diesem Fall werden zuerst diese Verbindungen der Formel 8 zu den Verbindungen der Formel 9 verseift und anschließend mit Alkoholen der Formel 4, in dem R⁴ nicht Niederalkyl, insbesondere Methyl bedeutet, umgesetzt.
iii) Diese Methode entspricht im wesentlichen der Methode a i). Verbindungen der Formel 10 (Herstellung beispielsweise aus 6) werden mit Verbindungen der Formel 2, in denen Z für Hydroxy oder für eine Abgangsgruppe steht, die nukleophil ablösbar ist und insbesondere F, Cl, Br, J oder Tosylat bedeutet, zu Verbindungen der Formel 8, umgesetzt. Diese Umsetzung erfolgt in einem dipolar aprotischen organischen Lösungsmittel oder einem Lösemittelgemisch. Insbesondere sind folgende Lösemittel genannt: Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin, gegebenenfalls unter Zusatz eines Säurebinders, wie Ammoniak, Triethylamin, Tributylamin, bei einer Reaktionstemperatur von 0° bis 180°C, vorzugsweise von 0° bis 80°C. Sofern Z für Hydroxy steht, handelt es sich um eine Kondensationsmethode, die aus der Peptidchemie bekannt ist (vgl. auch Methode B).
   Die Verbindungen der Formel 8 können weiterhin nach dem Fachmann bekannten Methoden in die Pyridin-N-oxide 8' übergeführt werden. Die hierzu notwendigen Angaben sind unter Methode a ii) zusammengestellt.

Zur Herstellung von Verbindungen gemäß der allgemeinen Formel I (3, 3', 8, 8') nach den Schemata I und II werden neutrale Verbindungen eingesetzt. Ggf. kann nach Abschluß der Synthese eine Salzbildung erfolgen. Als Salzbildner kommen bevorzugt N-Alkylamine, (Hydroxyalkyl)amine und (Alkoxyalkyl)amine, wie z. B. 2-Ethanolamin, 3-Propanolamin, 2-Methoxyethylamin, 2-Ethoxyethylamin und α,α,α-Tris-(hydroxymethyl)methylamin (= Trispuffer, Tromethan) oder auch basische Aminosäuren, wie z. B. Histidin, Arginin und Lysin in Frage.

Die erfindungsgemäßen Verbindungen der Formel I besitzen wertvolle pharmakologische Eigenschaften und zeigen insbesondere antifibrotische Wirksamkeit.

Die antifibrotische Wirkung kann im Modell der Tetrachlorkohlenstoff-induzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimjmung von Kollagenfragmenten und Prokollagenpeptiden im Serum bestimmt werden. Die erfindungsgemäßen Verbindungen sind in diesem Modell in Konzentration 1 bis 100 mg/kg wirksam.

Die Aktivität der Fibrogenese kann durch radioimmunologische Bestimmung des N-terminalen Propeptids des Kollagen Typ-III oder der N- bzw. C-terminalen Quervernetzungsdomäne des Kollagen-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurden
gemessen (diese Testmethode wird beschrieben von Rouiller, C., Experimental toxic injury of the liver; in The Liver, C. Rouiller, Vol. 2, S. 335 bis 476, New York, Academic Press, 1964).

Die Verbindungen der Formel I können als Medikamente in Form von pharmazeutischer Präparate Verwendung finden, welche sie gegebenenfalls mit verträglichen pharmazeutischen Trägern enthalten. Die Verbindungen können als Heilmittel, z. B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z. B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 bis 25 mg/kg/Tag, vorzugsweise 1 bis 5 mg/kg/Tag oder parenteral in Dosen von 0,01 bis 5 mg/kg/Tag, vorzugsweise 0,01 bis 2,5 mg/kg/Tag, insbesondere 0,5 bis 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

In den Beispielen 1 bis 592 bedeutet entsprechend Formel I X = CO. Die in den folgenden Beispielen beschriebenen Pyridin-2-carbonsäuremethylester-Derivate wurden
- aus Pyridin-2-carbonsäuremethylester-5-carbonsäurechlorid und den entsprechenden Amiden der Formel 7/Kalium-tert.Butylat, wie für die Beispiele 1b, 3, 4 und 68 explicit beschrieben (Methode A), oder
- aus Pyridin-2-carbonsäuremethylester-5-carbonsäure, den entsprechenden Amiden der Formel 7 und N, N'-Dicyclohexylcarbodiimid/ 4-N,N-Dimethylaminopyridin, wie für Beispiel 32 explicit beschrieben (Methode B)
hergestellt.

### Beispiel 1

5-[((4-Methoxyphenylsulfonyl)amino)carbonyl]-pyridin-2-carbonsäure-2-propylester
a) Pyridin-2-carbonsäuremethylester-5-carbonsäurechlorid
   14,5 g (80 mmol) Pyridin-2-carbonsäure-methylester-5-carbonsäure wurden in 200 ml wasserfreiem Toluol mit 6,48 ml Thionylchlorid und 2 ml wasserfreiem N,N-Dimethylacetamid versetzt. Unter Rühren wurde 3 h auf 70°C erhitzt. Danach engte man in Vakuum ein und löste den Rückstand in 150 ml Tetrahydrofuran.
b) 5-[(4-Methoxyphenylsulfonyl)amino)-carbonyl]pyridin-2-carbonsäuremethylester

### Methode A:

Zu 16,45 g (88 mmol) 4-Methoxybenzolsulfonsäureamid in 200 ml Tetrahydrofuran gab man bei 0°C 19,75 g (176 mmol) Kalium-tert. Butylat. Nachdem 3 h bei Raumtemperatur gerührt wurde, gab man die Lösung aus Beispiel 1a) bei 0 bis 5°C zu. Man rührte 3 h unter Erwärmung auf Raumtemperatur, gab 300 ml Ethylacetat zu, extrahierte zweimal mit wäßriger NaHCO₃-Lösung, säuerte die wäßrige Phase mit konzentrierter wäßriger Salzsäure an, extrahierte 3 mal mit Dichlormethan, trocknete, engte ein, kristallisierte den Rückstand aus Methanol und erhielt 9,9 g farbloses, kristallines Produkt, Fp. 197 bis 199°C.
c) 5-[(4-Methoxyphenylsulfonyl)amino-carbonyl]-pyridin-2-carbonsäure
   3,0 g (8,6 mmol) der Verbindung aus Beispiel 1b) wurden in 100 ml Methanol gelöst und bei 0 bis 5°C mit 17,2 ml (17,2 mmol) 1 N NaOH versetzt. Nachdem 4 h bei Raumtemperatur gerührt wurde, engte man in Vakuum ein, nahm den Rückstand in Wasser auf und gab bei 0 bis 5°C 17,2 ml (17,2 mmol) 1 N HCl zu, saugte den Feststoff ab, wusch mehrfach mit Wasser und erhielt 2,58 g der obigen Verbindung, Fp. 234 bis 236°C.
d) 0,58 g (1,73 mmol) des vorstehenden Pyridin-2-carbonsäure-Derivats wurden in 50 ml wasserfreiem 2-Propanol mit 4 Tropfen konz. Schwefelsäure versetzt und 56 h bei 80°C gerührt. Nach dem Abkühlen wurde i. Vak. eingeengt, der Rückstand mit gesättigter wäßriger NaHCO₃-Lösung behandelt, mit wäßriger Salzsäure angesäuert, das kristalline Rohprodukt abgesaugt, mit Wasser gewaschen und aus Methanol umkristallisiert. Man erhielt 0,47 g der Titelverbindung, Fp. 215 bis 216°C.

### Beispiel 2

5-[((Phenylsulfonyl)amino)carbonyl]-pyridin-2-carbonsäure-3-pentylester
a) 5-[((Phenylsulfonyl)amino)carbonyl]pyridin-2-carbonsäuremethylester
   Die Verbindung wurde analog Beispiel 1b) aus 3,46 g (22 mmol) Benzolsulfonsäureamid, 2,46 g (22 mmol) Kalium-tert. Butylat und 4,0 g (20 mmol) Pyridin-2-carbonsäuremethylester-5-carbonsäurechlorid erhalten. Man erhielt nach dem Umkristallisieren aus Methanol 1,6 g Produkt, Fp. 197 bis 198°C.
b) 5-[((Phenylsulfonyl)amino)carbonyl]-pyridin-2-carbonsäure
   3,5 g (10,94 mmol) der vorstehenden Verbindung wurden in 150 ml 1,5 N methanolischer Natronlauge 30 min unter Rückfluß gerührt. Dann wurde i. Vak. eingeengt, in wenig Wasser/Tetrahydrofuran gelöst, mit wäßriger Salzsäure auf pH 1 angesäuert, gekühlt, abgesaugt und getrocknet. Man erhielt 2,6 g; Fp. 247 bis 248°C.
c) 0,9 g (2,94 mmol) 5-[((Phenylsulfonyl)amino)carbonyl]-pyridin-2-carbonsäure wurden in 30 ml 3-Pentanol unter Rühren mit 5 Tropfen konz. Schwefelsäure versetzt und 6 h auf 90°C erhitzt. Das überschüssige 3-Pentanol wurde i. Vak. abdestilliert und der Rückstand mit Ethylacetat behandelt. Das kristalline Rohprodukt wurde mit dem aus der Mutterlauge durch säulenchromatographische Reinigung erhaltenen Produkt, (Fp. 186 bis 187°C) vereinigt, mit heißem Ethylacetat behandelt, abgekühlt, abgesaugt und mit Ethylacetat gewaschen. Man erhielt 0,55 g der farblosen Titelverbindung, Fp. 182 bis 183°C.

### Beispiel 3

5-[((4-n-Butoxyphenylsulfonyl)amino)carbonyl]-pyridin-2-carbonsäuremethylester
a) 4-n-Butoxybenzolsulfonsäureamid
   Zu 10 g 4-n-Butoxybenzolsulfonsäure-chlorid wurden unter Eiskühlung 100 ml methanolische Ammoniaklösung getropft. Nach 1/2 Stunde Rühren bei 20°C wurde eingeengt, mit Wasser versetzt, angesäuert auf pH 1 bis 2 und abgesaugt, Fp. 99 bis 101°C.
b) Analog Beispiel 1b) wurden 4,6 g (20 mmol) vorstehender Verbindung, 2,5 g (22 mmol) Kalium-tert.butylat und 5,0 g (25 mmol) Pyridin-2-carbonsäuremethylester-5-carbonsäure-chlorid umgesetzt. Ausgefallenes Kaliumsalz wurde im Dioxan-/Wasser-Gemisch mit 2 N HCl angesäuert. Das ausgefallene Produkt wurde abgesaugt und getrocknet; Ausbeute 1,5 g; Fp. 174 bis 176°C.

### Beispiel 4

5-[((4-Trifluormethoxyphenylsulfonyl)amino)carbonyl]-pyridin-2-carbonsäuremethylester
a) 4-Trifluormethoxybenzolsulfonsäureamid wurde aus dem entsprechenden Sulfonsäurechlorid durch Reaktion mit methanolischer Ammoniak-Lösung erhalten. Das Rohprodukt wurde mit Wasser versetzt, angesäuert, abgesaugt und getrocknet, Fp. 143 bis 145°C.
b) Analog Beispiel 1b) setzte man 4,8 g (20 mmol) der vorstehenden Verbindung, 2,5 g (22 mmol) Kalium-tert.butylat in Dioxan und 5 g (25 mmol) Pyridin-2-carbonsäuremethylester-5-carbonsäurechlorid um. Nach Einengen wurde der Rückstand in Wasser aufgenommen, angesäuert, die Fällung abgesaugt und getrocknet; 3,4 g Rohprodukt (Fp. 210 bis 214°C), das aus 75 ml Ethylacetat umkristallisiert wurde, 1,5 g farblos kristalline Substanz, Fp. 221 bis 223°C.

### Beispiel 5

5-[((-4-Fluorphenyl)sulfonyl)amino)carbonyl]-pyridin-2-carbonsäuremethylester
Die Titelverbindung wird analog Beispiel 1b) aus 4-Fluorbenzolsulfonsäureamid und Pyridin-2-carbonsäuremethylester-5-carbonsäurechlorid erhalten; Fp. 221 bis 223°C.

### Beispiel 6

5-[((4-Methoxyphenylsulfonyl)amino)carbonyl]-pyridin-2-carbonsäureethylester

### Beispiel 7

5-[((4-Methoxyphenylsulfonyl)amino)carbonyl]-pyridin-2-carbonsäure-n-propylester

### Beispiel 8

5-[((4-Methoxyphenylsulfonyl)amino)carbonyl]-pyridin-2-carbonsäure-1-(2-methylpropyl)ester

### Beispiel 9

5-[((4-Methoxyphenylsulfonyl)amino)carbonyl]-pyridin-2-carbonsäure-3-pentylester

### Beispiel 10

5-[((4-Methoxyphenylsulfonyl)amino)carbonyl]-pyridin-2-carbonsäurecyclohexylester

### Beispiel 11

5-[((4-Methoxyphenylsulfonyl)amino)carbonyl]-pyridin-2-carbonsäurecyclopentylester

### Beispiel 12

5-[((4-Phenoxy-phenylsulfonyl)amino)carbonyl]-pyridin-2-carbonsäuremethylester, Methode A
Fp. 194 bis 196°C (aus Methanol)

### Beispiel 13

5-[((4-Phenoxy-phenylsulfonyl)amino)carbonyl]-pyridin-2-carbonsäure-2-propylester

### Beispiel 14

5-[((4-Phenoxy-phenylsulfonyl)amino)carbonyl]-pyridin-2-carbonsäurecyclohexylester

### Beispiel 15

5-[((2-Phenyl-phenylsulfonyl)amino)carbonyl]-pyridin-2-carbonsäuremethylester

### Beispiel 16

5-[((2-Phenyl-phenylsulfonyl)amino)carbonyl]-pyridin-2-carbonsäure-2-propylester

### Beispiel 17

5-[((n-Butylsulfonyl)amino)carbonyl]-pyridin-2-carbonsäuremethylester, Methode A
Fp. 160 bis 162°C (aus Diethylether)

### Beispiel 18

5-[((n-Butylsulfonyl)amino)carbonyl]-pyridin-2-carbonsäure-2-propylester

### Beispiel 19

5-[((n-Butylsulfonyl)amino)carbonyl]-pyridin-2-carbonsäure-3-pentylester

### Beispiel 20

5-[((4-[3-(Trifluormethyl)phenoxy]phenylsulfonyl)amino)carbonyl]-pyridin-2-carbonsäure-methylester, Methode B
Fp. 190 bis 192°C (aus Diisopropylether)

### Beispiel 21

5-[((4-[3-(Trifluormethyl)phenyloxy]phenylsulfonyl)amino)carbonyl]-pyridin-2-carbonsäure-ethylester

### Beispiel 22

5-[((1-Naphthylsulfonyl)amino)carbonyl]-pyridin-2-carbonsäureethylester

### Beispiel 23

5-[((1-Naphthylsulfonyl)amino)carbonyl]-pyridin-2-carbonsäure-n-butylester

### Beispiel 24

5-[((1-Naphthylsulfonyl)amino)carbonyl]-pyridin-2-propylester

### Beispiel 25

5-[((1-Naphthylsulfonyl)amino)carbonyl]-pyridin-2-carbonsäure-3-pentylester

### Beispiel 26

5-[((2-Napthylsulfonyl)amino)carbonyl]-pyridin-2-carbonsäureethylester

### Beispiel 27

5-[((Phenylsulfonyl)amino)carbonyl]-pyridin-2-carbonsäureethylester

### Beispiel 28

5-[((Phenylsulfonyl)amino)carbonyl]-pyridin-2-carbonsäure-1-propylester

### Beispiel 29

5-[((Phenylsulfonyl)amino)carbonyl]-pyridin-2-carbonsäure-2-propylester

### Beispiel 30

5-[((Phenylsulfonyl)amino)carbonyl]-pyridin-2-carbonsäure-1-pentylester

### Beispiel 31

5-[((Phenylsulfonyl)amino)carbonyl]-pyridin-2-carbonsäure-1-(2-methylpropyl)ester

### Beispiel 32

5-[((4-((2-Phenylethyl)aminocarbonyl)phenylsulfonyl)amino)carbonyl]-pyridin-2-carbonsäure-methylester
a) 4-((2-Phenylethyl)aminocarbonyl)benzol-sulfonsäureamid
   20,1 g (0,1 mol) 4-Carboxy-benzolsulfonsäureamid wurden in 300 ml wasserfreiem Tetrahydrofuran suspendiert und bei 0°C unter Rühren tropfenweise mit 15,2 ml (0,11 mol) Triethylamin versetzt. Nach 30 min tropfte man bei 0°C 10,5 ml (0,11 mol) Chlorameisensäureethylester zu, rührte 1 h bei dieser Temperatur, kühlte ab auf -10°C und tropfte 12,1 g (0,1 mol, 12,5 ml) 2-Phenylethylamin in 30 ml wasserfreiem Tetrahydrofuran zu. Nach 1 h bei 0°C erwärmte man auf 20°C, engte im Vakuum ein, behandelte den festen Rückstand mit Wasser, saugte ab, kristallisierte aus Ethanol um und erhielt 20,4 g Produkt, Fp. 243 bis 245°C.
b) Methode B:
   1,8 g (10 mmol) Pyridin-2,5-dicarbonsäure-2-methylester wurden in 300 ml wasserfreiem Acetonitril suspendiert und bei 20°C unter Rühren mit 3,0 g (10 mmol) der vorstehenden Verbindung, 2,1 g (10 mmol) N,N'-Dicyclohexylcarbodiimid und 1,2 g (10 mmol) 4-N,N-Dimethylaminopyridin versetzt und 20 h bei 20°C gerührt. Dann wurde vom Ungelösten abfiltriert, das Filtrat im Vakuum eingeengt, der Rückstand in 200 ml Dichlormethan aufgenommen, zweimal mit gesättigter wäßriger NaHCO₃-Lösung, sodann mit 100 ml 2 N wäßriger HCl extrahiert. Die kristalline Fällung wurde anschließend mit warmem Methanol behandelt, abgesaugt und getrocknet. Man erhielt 2,2 g des Esters, Fp. 228 bis 230°C.

Die Beispiele 33 bis 67 wurden nach Methode B hergestellt:

### Beispiel 33

5-[((4-Benzylaminocarbonyl)phenylsulfonyl)amino)carbonyl]-pyridin-2-carbonsäure-methylester

### Beispiel 34

5-[((4-((3-Phenyl-n-propyl)aminocarbonyl)phenylsulfonyl)amino)carbonyl]-pyridin-2-carbonsäure-methylester
Fp. 240 bis 242°C (aus Methanol)

### Beispiel 35

5-[((4-((4-Phenyl-n-butyl)aminocarbonyl)phenylsulfonyl)amino)carbonyl]-pyridin-2-carbonsäure-methylester
Fp. 223 bis 225°C (aus Methanol)

### Beispiel 36

5-[((4-((2-(4-Methoxyphenyl)ethyl)aminocarbonyl)phenylsulfonyl)amino) carbonyl]-pyridin-2-carbonsäure-methylester
Fp. 240 bis 242°C (aus Methanol)

### Beispiel 37

5-[((4-((2-(3,4-Dimethoxyphenyl)ethyl)aminocarbonyl)phenylsulfonyl)amino)-carbonyl]-pyridin-2-carbonsäure-methylester
Fp. 213 bis 215°C (aus Methanol)

### Beispiel 38

5-[((4-((2-(2-Methoxyphenyl)ethyl)aminocarbonyl)phenylsulfonyl)amino) carbonyl]-pyridin-2-carbonsäure-methylester
Fp. 220 bis 222°C (aus Methanol)

### Beispiel 39

5-[((4-((2-(4-Fluorphenyl)ethyl)aminocarbonyl)phenylsulfonyl)amino)carbonyl]-pyridin-2-carbonsäure-methylester

### Beispiel 40

5-[((4-((2-(4-Chlorphenyl)ethyl)aminocarbonyl)phenylsulfonyl)amino)carbonyl]-pyridin-2-carbonsäure-methylester

### Beispiel 41

5-[((4-(Ethylaminocarbonyl)phenylsulfonyl)amino)carbonyl]-pyridin-2-carbonsäure-methylester

### Beispiel 42

5-[((4-(n-Butylaminocarbonyl)phenylsulfonyl)amino)carbonyl]-pyridin-2-carbonsäure-methylester

### Beispiel 43

5-[((4-(n-Hexylaminocarbonyl)phenylsulfonyl)amino)carbonyl]-pyridin-2-carbonsäure-methylester

### Beispiel 44

5-[((4-(N,N-Di-n-butylaminocarbonyl)phenylsulfonyl)amino)carbonyl]-pyridin-2-carbonsäure-methylester

### Beispiel 45

5-[((4-(Cyclohexylaminocarbonyl)phenylsulfonyl)amino)carbonyl]-pyridin-2-carbonsäure-methylester

### Beispiel 46

5-[((4-(2-Methoxyethyl)aminocarbonyl)phenylsulfonyl)amino)carbonyl]-pyridin-2-carbonsäure-methylester

### Beispiel 47

5-[((4-((2-Ethoxyethyl)aminocarbonyl)phenylsulfonyl)amino)carbonyl]-pyridin-2-carbonsäure-methylester

### Beispiel 48

5-[((4-((3-Ethoxypropyl)aminocarbonyl)phenylsulfonyl)amino)carbonyl]-pyridin-2-carbonsäure-methylester
Aus 5,4 g Pyridin-2-carbonsäuremethylester-5-carbonsaure, 6,3 g N,N'-Dicyclohexylcarbodiimid, 3,6 g 4-N,N-Dimethylamino-pyridin und 9 g 4-((3-Ethoxypropyl)aminocarbonyl)benzolsulfonsäureamid (je 30 mmol) wurden nach Methode B 9,9 g der Titelverbindung erhalten; Fp. 194 bis 196°C (aus Ethylacetat)

### Beispiel 49

5-[((4-((2-Phenoxyethyl)aminocarbonyl)phenylsulfonyl)amino)carbonyl]-pyridin-2-carbonsäure-methylester

### Beispiel 50

5-[((3-(Benzylaminocarbonyl)phenylsulfonyl)amino)carbonyl]-pyridin-2-carbonsäure-methylester

### Beispiel 51

5-[((3-((3-Phenylpropyl)aminocarbonyl)phenylsulfonyl)amino)carbonyl]-pyridin-2-carbonsäure-methylester

### Beispiel 52

5-[((3-((4-Phenyl-n-butyl)aminocarbonyl)phenylsulfonyl)amino)carbonyl]-pyridin-2-carbonsäure-methylester
Fp. 188 bis 191°C

### Beispiel 53

5-[((3-((2-(3,4-Dimethoxyphenyl)ethyl)aminocarbonyl)phenylsulfonyl)amino)carbonyl]-pyridin-2-carbonsäure-methylester

### Beispiel 54

5-[((3-((2-(3-Methoxyphenyl)ethyl)aminocarbonyl)phenylsulfonyl)amino) carbonyl]-pyridin-2-carbonsäure-methylester

### Beispiel 55

5-[((3-((2-(2-Methoxyphenyl)ethyl)aminocarbonyl)phenylsulfonyl)amino) carbonyl]-pyridin-2-carbonsäure-methylester

### Beispiel 56

5-[((3-((2-(4-Fluorphenyl)ethyl)aminocarbonyl)phenylsulfonyl)amino)carbonyl]-pyridin-2-carbonsäure-methylester

### Beispiel 57

5-[((3-((2-(4-Chlorphenyl)ethyl)aminocarbonyl)phenylsulfonyl)amino)carbonyl]-pyridin-2-carbonsäure-methylester

### Beispiel 58

5-[((3-(Ethylaminocarbonyl)phenylsulfonyl)amino)carbonyl]-pyridin-2-carbonsäure-methylester

### Beispiel 59

5-[((3-(n-Butylaminocarbonyl)phenylsulfonyl)amino)carbonyl]-pyridin-2-carbonsäure-methylester

### Beispiel 60

5-[((3-(n-Hexylaminocarbonyl)phenylsulfonyl)amino)carbonyl]-pyridin-2-carbonsäure-methylester

### Beispiel 61

5-[((3-(N,N-Di-n-butylaminocarbonyl)phenylsulfonyl)amino)carbonyl]-pyridin-2-carbonsäure-methylester

### Beispiel 62

5-[((3-(Cyclohexylaminocarbonyl)phenylsulfonyl)amino)carbonyl]-pyridin-2-carbonsäure-methylester

### Beispiel 63

5-[((3-((2-Methoxyethyl)aminocarbonyl)phenylsulfonyl)amino)carbonyl]-pyridin-2-carbonsäure-methylester

### Beispiel 64

5-[((3-((3-Ethoxypropyl)aminocarbonyl)phenylsulfonyl)amino)carbonyl]-pyridin-2-carbonsäure-methylester

### Beispiel 65

5-[((3-((3-Methoxypropyl)aminocarbonyl)phenylsulfonyl)amino)carbonyl]-pyridin-2-carbonsäure-methylester

### Beispiel 66

5-[((3-((2-Phenylethyl)aminocarbonyl)phenylsulfonyl)amino)carbonyl]-pyridin-2-carbonsäure-methylester
Fp. 214 bis 216°C

### Beispiel 67

5-[((3-((2-Phenoxyethyl)aminocarbonyl)phenylsulfonyl)amino)carbonyl]-pyridin-2-carbonsäure-methylester

### Beispiel 68

5-[((4-(2-((2-Chlor-5-methoxybenzoyl)amino)ethyl)phenylsulfonyl)amino) carbonyl]-pyridin-2-carbonsäure-methylester (Methode A)
Das aus 4,0 g (22 mmol) Pyridin-2,5-dicarbonsäure-2-methylester, wie in Beispiel 1 c) beschrieben, hergestellte Pyridin-2-carbonsäuremethylester-5-carbonsäurechlorid wurde in 50 ml wasserfreiem 1,4-Dioxan bei 40°C zu dem Reaktionsgemisch aus 7,4 g (20 mmol) 2-(((2-Chlor-5-methoxy-benzoyl)amino)ethyl) benzolsulfonsäureamid (hergestellt aus 4-(2-Aminoethyl)benzolsulfonsäureamid und 2-Chlor-5-methoxy-benzoesäure), 2,3 g (20 mmol) Kalium-tert.butylat in 150 ml wasserfreiem 1,4-Dioxan hinzugefügt (zur Bildung des Sulfonamid-Natriumsalzes war 15 min bei 50°C gerührt worden).
Die Reaktionsmischung wurde 90 min bei 60°C, dann 2 h unter Rückfluß gerührt, das Lösungsmittel im Vakuum abdestilliert, der Rückstand mit Wasser versetzt, mit wäßriger HCl auf pH 1 bis 2 gebracht und mit Dichlormethan extrahiert.
Der Rückstand wurde mit heißem Ethylacetat behandelt, abgesaugt und mit Ethylacetat gewaschen. Das so erhaltene Rohprodukt (2,8 g) wurde mit 100 ml kaltem Wasser, dann mit 100 ml heißem Wasser behandelt und das farblos kristalline Produkt abgesaugt. Man erhielt 2,6 g, Fp. 187 bis 190°C.

### Beispiel 69

5-[((4-(2-(n-Pentanoylamino)ethyl)phenylsulfonyl)amino)carbonyl]-pyridin-2-carbonsäuremethylester (Methode B)
Fp. 178 bis 180°C (aus wäßriger Salzsäure)

### Beispiel 70

5-[((4-(2-(n-Butanoylamino)ethyl)phenylsulfonyl)amino)carbonyl]-pyridin-2-carbonsäuremethylester (Methode B)
Fp. 195°C

### Beispiel 71

5-[((4-(2-(Benzoylamino)ethyl)phenylsulfonyl)amino)carbonyl]-pyridin-2-carbonsäuremethylester (Methode A)
Fp. 228 bis 230°C (aus Methanol)

### Beispiel 72

5-[((4-(2-((4-Chlorbenzoyl)amino)ethyl)phenylsulfonyl)amino)carbonyl]-pyridin-2-carbonsäuremethylester-Na-Salz (Methode A)
Fp. 260 bis 262°C (aus wäßriger NaHCO₃-Lösung)

### Beispiel 73

5-[((4-(2-((5-Chlor-2-methoxybenzoyl)amino)ethyl)phenylsulfonyl)amino) carbonyl]-pyridin-2-carbonsäuremethylester (Methode A)
Fp. 233°C (aus wäßriger Salzsäure)

### Beispiel 74

5-[((4-(2-((3-Phenyl-n-propionyl)amino)ethyl)phenylsulfonyl)amino)carbonyl]-pyridin-2-carbonsäuremethylester-Na-Salz (Methode A)
Fp. 236 bis 238°C (aus wäßriger NaHCO₃-Lösung)

### Beispiel 75

5-[((4-(2-((2-Phenylacetyl)amino)ethyl)phenylsulfonyl)amino)carbonyl]-pyridin-2-carbonsäuremethylester (Methode B)

### Beispiel 76

5-[((4-(2-((Phenoxyacetyl)amino)ethyl)phenylsulfonyl)amino)carbonyl]-pyridin-2-carbonsäuremethylester (Methode B)
Fp. 201 bis 203°C (aus Methanol)

### Beispiel 77

5-[((4-(2-((4-Fluorbenzoyl)amino)ethyl)phenylsulfonyl)amino)carbonyl]-pyridin-2-carbonsäuremethylester (Methode A)

### Beispiel 78

5-[((4-(2-((4-Ethoxybenzoyl)amino)ethyl)phenylsulfonyl)amino)carbonyl]-pyridin-2-carbonsäuremethylester (Methode A)

### Beispiel 79

5-[((4-(2-((Cyclohexanoyl)amino)ethyl)phenylsulfonyl)amino)carbonyl]-pyridin-2-carbonsäuremethylester (Methode B)

### Beispiel 80

5-[((4-(2-((Cyclohexylacetyl)amino)ethyl)phenylsulfonyl)amino)carbonyl]-pyridin-2-carbonsäuremethylester (Methode B)
Fp. 192 bis 194°C (aus Methanol/Diisopropylether)

### Beispiel 81

5-[((4-(2-((2-Methylpropionyl)amino)ethyl)phenylsulfonyl)amino)carbonyl]-pyridin-2-carbonsäuremethylester (Methode B)
Fp. 193 bis 197°C (aus wäßriger Salzsäure)

### Beispiel 82

5-[((4-(2-(Acetylamino]methyl)phenylsulfonyl)amino)carbonyl]-pyridin-2-carbonsäuremethylester (Methanol B)
Fp. 232 bis 235°C (aus wäßriger Salzsäure)

### Beispiel 83

5-[((4-(2-((3-Methylbutanoyl)amino)ethyl)phenylsulfonyl)amino)carbonyl]-pyridin-2-carbonsäuremethylester (Methode B)
Fp. 176 bis 178°C (aus Methanol/Diisopropylether)

### Beispiel 84

5-[((4-(2-((4-Methylpentanoyl)amino)ethyl)phenylsulfonyl)amino)carbonyl]-pyridin-2-carbonsäuremethylester (Methode B)
Fp. 204 bis 206°C (aus Methanol/Wasser)

### Beispiel 85

5-[((4-(2-((3-Cyclohexyl-n-propionyl)amino)ethyl)phenylsulfonyl)amino)carbonyl] pyridin-2-carbonsäuremethylester

### Beispiel 86

5-[((4-(2-(n-Pentanoylamino)ethyl)phenylsulfonyl)amino)carbonyl]-pyridin-2-carbonsäureethylester

### Beispiel 87

5-[((4-(2-((3-Methylbutanoyl)amino)methyl)phenylsulfonyl)amino)carbonyl]-pyridin-2-carbonsäuremethylester (Methode B)
Fp. 216 bis 218°C (aus Methanol)

### Beispiel 88

5-[((4-(2-(4-Methylpropionyl)amino)methyl)phenylsulfonyl)amino)carbonyl]-pyridin-2-carbonsäuremethylester (Methode B)
Fp. 225 bis 227°C (aus Diisopropylether)

### Beispiel 89

5-[((4-(2-((-Cyclohexyl-n-propionyl)amino)ethyl)phenylsulfonyl)amino)carbonyl]-pyridin-2-carbonsäureethylester

### Beispiel 90

5-[((4-(2-(n-Pentanoylamino)ethyl)phenylsulfonyl)amino)carbonyl]-pyridin-2-carbonsäure-2-propylester

### Beispiel 91

5-[((4-(2-((3-Methylbutanoyl)amino)ethyl)phenylsulfonyl)amino)carbonyl]-pyridin-2-carbonsäure-2-propylester

### Beispiel 92

5-[((4-(2-((4-Methylpentanoyl)amino)ethyl)phenylsulfonyl)amino)carbonyl]-pyridin-2-carbonsäure-2-propylester

### Beispiel 93

5-[((4-(2-((3-Cyclohexyl-n-propionyl)amino)ethyl)phenylsulfonyl)amino)carbonyl]-pyridin-2-carbonsäure-2-propylester

### Beispiel 94

5-[((4-(2-(n-Pentanoylamino)ethyl)phenylsulfonyl)amino)carbonyl]-pyridin-2-carbonsäure-1-pentylester

### Beispiel 95

5-[((4-(2-((3-Methylbutanoyl)amino)ethyl)phenylsulfonyl)amino)carbonyl]-pyridin-2-carbonsäure-1-pentylester

### Beispiel 96

5-[((4-(2-((4-Methylpentanoyl)amino)ethyl)phenylsulfonyl)amino)carbonyl]-pyridin-2-carbonsäure-1-pentylester

### Beispiel 97

5-[((4-(2-((3-Cyclohexyl-n-propionyl)amino)ethyl)phenylsulfonyl)amino)carbonyl]-pyridin-2-carbonsäure-1-pentylester

### Beispiel 98

5-[((4-(2-(n-Pentanoylamino)ethyl)phenylsulfonyl)amino)carbonyl]-pyridin-2-carbonsäure-3-pentylester

### Beispiel 99

5-[((4-(2-((3-Methylbutanoyl)amino)ethyl)phenylsulfonyl)amino)carbonyl]-pyridin-2-carbonsäure-3-pentylester

### Beispiel 100

5-[((4-(2-((4-Methylpentanoyl)amino)ethyl)phenylsulfonyl)amino)carbonyl]-pyridin-2-carbonsäure-3-pentylester)

### Beispiel 101

5-[((4-(2-((3-Cyclohexyl-n-propionyl)amino)ethyl)phenylsulfonyl)aminolcarbonyl]-pyridin-2-carbonsäure-3-pentylester

### Beispiel 102

5-[((4-(2-(n-Pentanoylamino)ethyl)phenylsulfonyl)amino)carbonyl]-pyridin-2-carbonsäure-(2,5-dimethyl-3-pentyl)ester

### Beispiel 103

5-[((4-(2-((3-Methylbutanoyl)amino)ethyl)phenylsulfonyl)amino)carbonyl]-pyridin-2-carbonsäure-(2,5-dimethyl-3-pentyl)ester

### Beispiel 104

5-[((4-(2-((4-Methylpentanoyl)amino)ethyl)phenylsulfonyl)amino)carbonyl]-pyridin-2-carbonsäure-(2,5-dimethyl-3-pentyl)ester

### Beispiel 105

5-[((4-(2-((3-Cyclohexyl-n-propionyl)amino)ethyl)phenylsulfonyl)amino)carbonyl]-pyridin-2-carbonsäure-(2,5-dimethyl-3-pentyl)ester

### Beispiel 106

5-[((4-(2-(n-Pentanoylamino)ethyl)phenylsulfonyl)amino)carbonyl]-pyridin-2-carbonsäure-cyclohexylester

### Beispiel 107

5-[((4-(2-((3-Methylbutanoyl)amino)ethyl)phenylsulfonyl)amino)carbonyl]-pyridin-2-carbonsäure-cyclohexylester

### Beispiel 108

5-[((4-(2-((4-Methylpentanoyl)amino)ethyl)phenylsulfonyl)amino)carbonyl]-pyridin-2-carbonsäure-cyclohexylester

### Beispiel 109

5-[((4-(2-((3-Cyclohexyl-n-propionyl)amino)ethyl)phenylsulfonyl)amino)carbonyl]-pyridin-2-carbonsäure-cyclohexylester

### Beispiel 110

5-[((4-(2-((3-Methylbutanoyl)amino)ethyl)phenylsulfonyl)amino)carbonyl]-pyridin-2-carbonsäure-benzylester

### Beispiel 111

5-[((4-(2-((4-Methylpentanoyl)amino)ethyl)phenylsulfonyl)amino)carbonyl]-pyridin-2-carbonsäure-benzylester

### Beispiel 112

5-[((4-((2-Phenylethyl)aminocarbonyl)phenylsulfonyl)amino)carbonyl]-pyridin-2-carbonsäure-3-pentylester
a) 5-[((4-((2-Phenylethyl)aminocarbonyl)phenylsulfonyl)amino)carbonyl]-pyridin-2-carbonsäure
   0,7 g (1,5 mmol) des in Beispiel 32b) beschriebenen Methylesters wurden bei 20°C unter Rühren in 100 ml 1,5 n methanolische NaOH eingetragen. Nachdem eine Lösung entstanden war, fiel ein kristallines Produkt aus. Dann wurde noch 30 min gerührt. Man engte im Vakuum ein, löste den Rückstand in einem Gemisch aus Wasser und Tetrahydrofuran, säuerte mit wäßriger HCl auf pH = 1 an, engte im Vakuum ein und saugte das farblos kristalline Produkt ab. Man erhielt 0,6 g, Fp. 263°C (u. Zers.).
b) 0,5 g (1,1 mmol) der vorstehenden Carbonsäure wurden in 30 ml 3-Pentanol und 20 ml 1,4-Dioxan mit 5 Tropfen konz. H₂SO₄ versetzt und 8 h unter Rühren auf 90 bis 100°C erhitzt. Vom Ungelöstem wurde abgetrennt, die Lösung eingeengt und der Rückstand mit Wasser zur Kristallisation gebracht. Dieses Rohprodukt wurde in Ethylacetat aufgenommen, getrocknet, eingeengt und der Rückstand mit Aceton/n-Heptan (3:1) an Kieselgel chromatographiert. Aus entsprechenden Fraktionen wurde die Titelverbindung nach Einengen mit Diethylether zur Kristallisation gebracht. Man erhielt 0,045 g der Titelverbindung; Fp. 234 bis 235°C.

### Beispiel 113

5-[((4-Benzylaminocarbonyl)phenylsulfonyl)amino)carbonyl]-pyridin-2-carbonsäure-3-pentylester

### Beispiel 114

5-[((4-((3-Phenyl-n-propyl)aminocarbonyl)phenylsulfonyl)amino)carbonyl]-pyridin-2-carbonsäure-3-pentylester

### Beispiel 115

5-[((4-((4-Phenyl-n-butyl)aminocarbonyl)phenylsulfonyl)amino)carbonyl]-pyridin-2-carbonsäure-3-pentylester

### Beispiel 116

5-[((4-((2-(4-Methoxyphenyl)ethyl)aminocarbonyl)phenylsulfonyl)amino) carbonyl]-pyridin-2-carbonsäure-3-pentylester

### Beispiel 117

5-[((4-((2-(3,4-Dimethoxyphenyl)ethyl)aminocarbonyl)phenylsulfonyl)amino)-carbonyl]-pyridin-2-carbonsäure-3-pentylester

### Beispiel 118

5-[((4-((2-(2-Methoxyphenyl)ethyl)aminocarbonyl)phenylsulfonyl)amino) carbonyl]-pyridin-2-carbonsäure-3-pentylester

### Beispiel 119

5-[((4-((2-(4-Fluorphenyl)ethyl)aminocarbonyl)phenylsulfonyl)amino)carbonyl]-pyridin-2-carbonsäure-3-pentylester

### Beispiel 120

5-[((4-((2-(4-Chlorphenyl)ethyl)aminocarbonyl)phenylsulfonyl)amino)carbonyl]-pyridin-2-carbonsäure-3-pentylester

### Beispiel 121

5-[((4-(Ethylaminocarbonyl)phenylsulfonyl)amino)carbonyl]-pyridin-2-carbonsäure-3-pentylester

### Beispiel 122

5-[((4-(n-Butylaminocarbonyl)phenylsulfonyl)amino)carbonyl]-pyridin-2-carbonsäure-3-pentylester

### Beispiel 123

5-[((4-(n-Hexylaminocarbonyl)phenylsulfonyl)amino)carbonyl]-pyridin-2-carbonsäure-3-pentylester

### Beispiel 124

5-[((4-(N,N-Di-n-butylaminocarbonyl)phenylsulfonyl)amino)carbonyl]-pyridin-2-carbonsäure-3-pentylester

### Beispiel 125

5-[((4-(Cyclohexylaminocarbonyl)phenylsulfonyl)amino)carbonyl]-pyridin-2-carbonsäure-3-pentylester

### Beispiel 126

5-[((4-(2-Methoxyethyl)aminocarbonyl)phenylsulfonyl)amino)carbonyl]-pyridin-2 carbonsäure-3-pentylester

### Beispiel 127

5-[((4-((2-Ethoxyethyl)aminocarbonyl)phenylsulfonyl)amino)carbonyl]-pyridin-2-carbonsäure-3-pentylester

### Beispiel 128

5-[((4-((3-Ethoxypropyl)aminocarbonyl)phenylsulfonyl)amino)carbonyl]-pyridin-2-carbonsäure-3-pentylester

### Beispiel 129

5-[((4-((2-Phenoxyethyl)aminocarbonyl)phenylsulfonyl)amino)carbonyl]-pyridin-2-carbonsäure-3-pentylester

### Beispiel 130

5-[((3-(Benzylaminocarbonyl)phenylsulfonyl)amino)carbonyl]-pyridin-2-carbonsäure-3-pentylester

### Beispiel 131

5-[((3-((3-Phenylpropyl)aminocarbonyl)phenylsulfonyl)amino)carbonyl]-pyridin-2-carbonsäure-3-pentylester

### Beispiel 132

5-[((3-((4-Phenyl-n-butyl)aminocarbonyl)phenylsulfonyl)amino)carbonyl]-pyridin-2-carbonsäure-3-pentylester

### Beispiel 133

5-[((3-((2-(4-Methoxyphenyl)ethyl)aminocarbonyl)phenylsulfonyl)amino) carbonyl]-pyridin-2-carbonsäure-3-pentylester

### Beispiel 134

5-[((3-((2-(3-Methoxyphenyl)ethyl)aminocarbonyl)phenylsulfonyl)amino) carbonyl]-pyridin-2-carbonsäure-3-pentylester

### Beispiel 135

5-[((3-((2-(2-Methoxyphenyl)ethyl)aminocarbonyl)phenylsulfonyl)amino) carbonyl]-pyridin-2-carbonsäure-3-pentylester

### Beispiel 136

5-[((3-((2-(4-Fluorphenyl)ethyl)aminocarbonyl)phenylsulfonyl)amino)carbonyl]-pyridin-2-carbonsäure-3-pentylester

### Beispiel 137

5-[((3-((2-(4-Chlorphenyl)ethyl)aminocarbonyl)phenylsulfonyl)amino)carbonyl]-pyridin-2-carbonsäure-3-pentylester

### Beispiel 138

5-[((3-(Ethylaminocarbonyl)phenylsulfonyl)amino)carbonyl]-pyridin-2-carbonsäure-3-pentylester

### Beispiel 139

5-[((3-(n-Butylaminocarbonyl)phenylsulfonyl)amino)carbonyl]-pyridin-2-carbonsäure-3-pentylester

### Beispiel 140

5-[((3-(n-Hexylaminocarbonyl)phenylsulfonyl)amino)carbonyl]-pyridin-2-carbonsäure-3-pentylester

### Beispiel 141

5-[((3-(N,N-Di-n-butylaminocarbonyl)phenylsulfonyl)amino)carbonyl]-pyridin-2-carbonsäure-3-pentylester

### Beispiel 142

5-[((3-(Cycohexylaminocarbonyl)phenylsulfonyl)amino)carbonyl]-pyridin-2-carbonsäure-3-pentylester

### Beispiel 143

5-[((3-((2-Methoxyethyl)aminocarbonyl)phenylsulfonyl)amino)carbonyl]-pyridin-2-carbonsäure-3-pentylester

### Beispiel 144

5-[((3-((3-Ethoxypropyl)aminocarbonyl)phenylsulfonyl)amino)carbonyl]-pyridin-2-carbonsäure-3-pentylester

### Beispiel 145

5-[((3-((3-Methoxypropyl)aminocarbonyl)phenylsulfonyl)amino)carbonyl]-pyridin-2-carbonsäure-3-pentylester

### Beispiel 146

5-[((3-((2-Phenylethyl)aminocarbonyl)phenylsulfonyl)amino)carbonyl]-pyridin-2-carbonsäure-3-pentylester
F. 189 bis 191°C (aus Wasser)

### Beispiel 147

5-[((3-((2-Phenoxyethyl)aminocarbonyl)phenylsulfonyl)amino)carbonyl]-pyridin-2-carbonsäure-3-pentylester

### Beispiel 148

5-[((4-(Benzylaminocarbonyl)phenylsulfonyl)amino)carbonyl]-pyridin-2-carbonsäure-ethylester

### Beispiel 149

5-[((4-((3-Phenyl-n-propyl)aminocarbonyl)phenylsulfonyl)amino)carbonyl]-pyridin-2-carbonsäure-ethylester

### Beispiel 150

5-[((4-((4-Phenyl-n-butyl)aminocarbonyl)phenylsulfonyl]amino)carbonyl]-pyridin-2-carbonsäure-ethylester

### Beispiel 151

5-[((4-((2-(4-Methoxyphenyl)ethyl)aminocarbonyl)phenylsulfonyl)amino) carbonyl]-pyridin-2-carbonsäure-ethylester

### Beispiel 152

5-[((4-((2-(3-Methoxyphenyl)ethyl)aminocarbonyl)phenylsulfonyl)amino) carbonyl]-pyridin-2-carbonsäure-ethylester

### Beispiel 153

5-[((4-((2-(2-Methoxyphenyl)ethyl)aminocarbonyl)phenylsulfonyl)amino) carbonyl]-pyridin-2-carbonsäure-ethylester

### Beispiel 154

5-[((4-((2-(4-Fluorphenyl)ethyl)aminocarbonyl)phenylsulfonyl)amino)carbonyl]-pyridin-2-carbonsäure-ethylester

### Beispiel 155

5-[((4-((2-(4-Chlorphenyl)ethyl)aminocarbonyl)phenylsulfonyl)amino)carbonyl]-pyridin-2-carbonsäure-ethylester

### Beispiel 156

5-[((4-(Ethylaminocarbonyl)phenylsulfonyl)amino)carbonyl]-pyridin-2-carbonsäure-ethylester

### Beispiel 157

5-[((4-(n-Butylaminocarbonyl)phenylsulfonyl)amino)carbonyl]-pyridin-2-carbonsäure-ethylester

### Beispiel 158

5-[((4-(n-Hexylaminocarbonyl)phenylsulfonyl)amino)carbonyl]-pyridin-2-carbonsäure-ethylester

### Beispiel 159

5-[((4-(N,N-Di-n-butylaminocarbonyl)phenylsulfonyl)amino)carbonyl]-pyridin-2-carbonsäure-ethylester

### Beispiel 160

5-[((4-(Cyclohexylaminocarbonyl)phenylsulfonyl)amino)carbonyl]-pyridin-2-carbonsäure-ethylester

### Beispiel 161

5-[((4-((2-Methoxyethyl)aminocarbonyl)phenylsulfonyl)amino)carbonyl]-pyridin-2-carbonsäure-ethylester

### Beispiel 162

5-[((4-((2-Ethoxyethyl)aminocarbonyl)phenylsulfonyl)amino)carbonyl]-pyridin-2-carbonsäure-ethylester

### Beispiel 163

5-[((4-((3-Ethoxypropyl)aminocarbonyl)phenylsulfonyl)amino)carbonyl]-pyridin-2-carbonsäure-ethylester

### Beispiel 164

5-[((4-((2-Phenylethyl)aminocarbonyl)phenylsulfonyl)amino)carbonyl]-pyridin-2-carbonsäure-ethylester

### Beispiel 165

5-[((4-((2-Phenoxyethyl)aminocarbonyl)phenylsulfonyl)amino)carbonyl]-pyridin-2-carbonsäure-ethylester

### Beispiel 166

5-[((3-(Benzylaminocarbonyl)phenylsulfonyl)amino)carbonyl]-pyridin-2-carbonsäure-1-propylester

### Beispiel 167

5-[((3-((Phenylpropyl)aminocarbonyl)phenylsulfonyl)amino)carbonyl]-pyridin-2-carbonsäure-1-propylester

### Beispiel 168

5-[((3-((4-Phenyl-n-butanoyl)aminocarbonyl)phenylsulfonyl)amino)carbonyl]-pyridin-2-carbonsäure-1-propylester

### Beispiel 169

5-[((3-((2-(4-Methoxyphenyl)ethyl)aminocarbonyl)phenylsulfonyl)aminocarbonyl]-pyridin-2-carbonsäure-1-propylester

### Beispiel 170

5-[((3-((2-(3-Methoxyphenyl)ethyl)aminocarbonyl)phenylsulfonyl)amino) carbonyl]-pyridin-2-carbonsäure-1-propylester

### Beispiel 171

5-[((3-((2-(2-Methoxyphenyl)ethyl)aminocarbonyl)phenylsulfonyl)amino) carbonyl]-pyridin-2-carbonsäure-1-propylester

### Beispiel 172

5-[((3-((2-(4-Fluorphenyl)ethyl)aminocarbonyl)phenylsulfonyl)amino)carbonyl]-pyridin-2-carbonsäure-1-propylester

### Beispiel 173

5[((3-((2-(4-Chlorphenyl)ethyl)aminocarbonyl)phenylsulfonyl)amino)carbonyl]-pyridin-2-carbonsäure-1-propylester

### Beispiel 174

5-[((3-(Ethylaminocarbonyl)phenylsulfonyl)amino)carbonyl]-pyridin-2-carbonsäure-1-propylester

### Beispiel 175

5-[((3-(n-Butylaminocarbonyl)phenylsulfonyl)amino)carbonyl]-pyridin-2-carbonsäure-1-propylester

### Beispiel 176

5-[((3-(n-Hexylaminocarbonyl)phenylsulfonyl)amino)carbonyl]-pyridin-2-carbonsäure-1-propylester

### Beispiel 177

5-[((3-(N,N-Di-n-butylaminocarbonyl)phenylsulfonyl)amino)carbonyl]-pyridin-2-carbonsäure-1-propylester

### Beispiel 178

5-[((3-(Cyclohexylaminocarbonyl)phenylsulfonyl)amino)carbonyl]-pyridin-2-carbonsäure-1-propylester

### Beispiel 179

5-[((3-((2-Methoxyethyl)aminocarbonyl)phenylsulfonyl)amino)carbonyl]-pyridin-2-carbonsäure-1-propylester

### Beispiel 180

5-[((3-((2-Ethoxyethyl)aminocarbonyl)phenylsulfonyl)amino)carbonyl]-pyridin-2-carbonsäure-1-propylester

### Beispiel 181

5-[((3-((3-Methoxypropyl)aminocarbonyl)phenylsulfonyl)amino)carbonyl]-pyridin-2-carbonsäure-1-propylester

### Beispiel 182

5-[((3-((2-Phenylethyl)aminocarbonyl)phenylsulfonyl)amino)carbonyl]-pyridin-2-carbonsäure-1-propylester

### Beispiel 183

5-[((3-((2-Phenoxyethyl)aminocarbonyl)phenylsulfonyl)amino)carbonyl]-pyridin-2-carbonsäure-1-propylester

### Beispiel 184

5-[((4-(Benzylaminocarbonyl)phenylsulfonyl)amino)carbonyl]-pyridin-2-carbonsäure-2-propylester

### Beispiel 185

5-[((4-((3-Phenylpropyl)aminocarbonyl)phenylsulfonyl)amino)carbonyl]-pyridin-2-carbonsäure-2-propylester

### Beispiel 186

5-[((4-((4-Phenyl-n-butyl)aminocarbonyl)phenylsulfonyl)amino)carbonyl]-pyridin-2-carbonsäure-2-propylester

### Beispiel 187

5-[((4-((2-(4-Methoxyphenyl)ethyl)aminocarbonyl)phenylsulfonyl)amino) carbonyl]-pyridin-2-carbonsäure-2-propylester

### Beispiel 188

5-[((4-((2-(3-Methoxyphenyl)ethyl)aminocarbonyl)phenylsulfonyl)amino) carbonyl]-pyridin-2-carbonsäure-2-propylester

### Beispiel 189

5-[((4-((2-(2-Methoxyphenyl)ethyl)aminocarbonyl)phenylsulfonyl)amino) carbonyl]-pyridin-2-carbonsäure-2-propylester

### Beispiel 190

5-[((4-((2-(4-Fluorphenyl)ethyl)aminocarbonyl)phenylsulfonyl)amino)carbonyl]-pyridin-2-carbonsäure-2-propylester

### Beispiel 191

5-[((4-((2-(4-Chlorphenyl)ethyl)aminocarbonyl)phenylsulfonyl)amino)carbonyl]-pyridin-2-carbonsäure-2-propylester

### Beispiel 192

5-[((4-(Ethylaminocarbonyl)phenylsulfonyl)amino)carbonyl]-pyridin-2-carbonsäure-2-propylester

### Beispiel 193

5-[((4-(n-Butylaminocarbonyl)phenylsulfonyl)amino)carbonyl]-pyridin-2-carbonsäure-2-propylester

### Beispiel 194

5-[((4-(n-Hexylaminocarbonyl)phenylsulfonyl)amino)carbonyl]-pyridin-2-carbonsäure-2-propylester

### Beispiel 195

5-[((4-(N,N-Di-n-butylaminocarbonyl)phenylsulfonyl)amino)carbonyl]-pyridin-2-carbonsäure-2-propylester

### Beispiel 196

5-[((4-(Cyclohexylaminocarbonyl)phenylsulfonyl)amino)carbonyl]-pyridin-2-carbonsäure-2-propylester

### Beispiel 197

5-[((4-((2-Methoxyethyl)aminocarbonyl)phenylsulfonyl)amino)carbonyl]-pyridin-2-carbonsäure-2-propylester

### Beispiel 198

5-[((4-((3-Ethoxypropyl)aminocarbonyl)phenylsulfonyl)amino)carbonyl]-pyridin-2-carbonsäure-2-propylester-Natrium-Salz
a) 5-[((4-((3-Ethoxypropyl)amino)carbonyl)phenylsulfonyl)amino)carbonyl]-pyridin-2-carbonsäure
   3,8 g (8,45 mmol) des in Beispiel 48 beschriebenen Methylesters wurden in 300 ml 1,5 N methanolische Natronlauge eingetragen. Es entstand kurzzeitig einer klare Lösung. Dann fiel kristallines Material aus. Man rührte 30 min weiter, engte i. Vak., erwärmte den Rückstand in 250 ml Wasser auf dem Dampfbad, gab zur Lösung in der Wärme 100 ml 1,4-Dioxan zu, filtrierte heiß vom Ungelösten ab, säuerte mit konz. wäßriger HCl auf pH 1 an, engte i. Vak. ein, saugte das ausgefallene Produkt ab, wusch mit Wasser und trocknet: 3,3 g Produkt; Fp. 192 bis 194°C.
b) 1 g (2,30 mmol) der vorstehenden Pyridin-2-carbonsäure wurden in 50 ml 2-Propanol mt 4 Tropfen konz. H₂SO₄ versetzte und unter Rühren 8 h auf 80°C erwärmt. Man engte i. Vak. ein, behandelte den öligen Rückstand mit 80 ml gesättigter wäßriger NaHCO₃-Lösung, trennte einen nicht identifizierten Feststoff ab, säuerte die NaHCO₃-Phase mit wäßriger HCl auf pH 1 an, saugte die Fällung ab und wusch mit Wasser. Man löste die Substanz in Dichlormethan und isolierte nach Behandeln mit wäßriger NaHCO₃-Lösung die Titelverbindung in Form farbloser Kristalle. Man erhielt 0,15 g; Fp. 145°C.

### Beispiel 199

5-[((4-((3-Methoxypropyl)aminocarbonyl)phenylsulfonyl)amino)carbonyl]-pyridin-2-carbonsäure-2-propylester

### Beispiel 200

5-[((4-((2-Phenylethyl)aminocarbonyl)phenylsulfonyl)amino)carbonyl]-pyridin-2-carbonsäure-2-propylester

### Beispiel 201

5-[((4-((2-Phenoxyethyl)aminocarbonyl)phenylsulfonyl)amino)carbonyl]-pyridin-2-carbonsäure-2-propylester

### Beispiel 202

5-[((4-((2-Phenylethyl)aminocarbonyl)phenylsulfonyl)amino)carbonyl]-pyridin-2-carbonsäure-cyclohexylester

### Beispiel 203

5-[((4-(Benzylaminocarbonyl)phenylsulfonyl)amino)carbonyl]-pyridin-2-carbonsäure-cyclohexylester

### Beispiel 204

5-[((4-((3-Phenylpropyl)aminocarbonyl)phenylsulfonyl)amino)carbonyl]-pyridin-2-carbonsäure-cyclohexylester

### Beispiel 205

5-[((4-((4-Phenyl-n-butyl)aminocarbonyl)phenylsulfonyl)amino)carbonyl]-pyridin-2-carbonsäure-cyclohexylester

### Beispiel 206

5-[((4-((2-(4-Methoxyphenyl)ethyl)aminocarbonyl)phenylsulfonyl)amino) carbonyl]-pyridin-2-carbonsäure-cyclohexylester

### Beispiel 207

5-[((4-((2-(3-Methoxyphenyl)ethyl)aminocarbonyl)phenylsulfonyl)amino) carbonyl]-pyridin-2-carbonsäure-cyclohexylester

### Beispiel 208

5-[((4-((2-(2-Methoxyphenyl)ethyl)aminocarbonyl)phenylsulfonyl)amino)carbonyl]-pyridin-2-carbonsäure-cyclohexylester

### Beispiel 209

5-[((4-((2-(4-Fluorphenyl)ethyl)aminocarbonyl)phenylsulfonyl)amino)carbonyl]-pyridin-2-carbonsäure-2-cyclohexylester

### Beispiel 210

5-[((4-((2-(4-Chlorphenyl)ethyl)aminocarbonyl)phenylsulfonyl)amino)carbonyl]-pyridin-2-carbonsäure-cyclohexylester

### Beispiel 211

5-[((4-(Ethylaminocarbonyl)phenylsulfonyl)amino)carbonyl]-pyridin-2-carbonsäure-cyclohexylester

### Beispiel 212

5-[((4-(n-Butylaminocarbonyl)phenylsulfonyl)amino)carbonyl]-pyridin-2-carbonsäure-cyclohexylester

### Beispiel 213

5-[((4-(n-Hexylaminocarbonyl)phenylsulfonyl)amino)carbonyl]-pyridin-2-carbonsäure-cyclohexylester

### Beispiel 214

5-[((4-(N,N-Di-n-butylaminocarbonyl)phenylsulfonyl)amino)carbonyl]-pyridin-2-carbonsäure-cyclohexylester

### Beispiel 215

5-[((4-(Cyclohexylaminocarbonyl)phenylsulfonyl)amino)carbonyl]-pyridin-2-carbonsäure-cyclohexylester

### Beispiel 216

5-[((4-((2-Methoxyethyl)aminocarbonyl)phenylsulfonyl)amino)carbonyl]-pyridin-2-carbonsäure-cyclohexylester

### Beispiel 217

5-[((4-((2-Ethoxyethyl)aminocarbonyl)phenylsulfonyl)amino)carbonyl]-pyridin-2-carbonsäure-cyclohexylester

### Beispiel 218

5-(((4-((3-Ethoxypropyl)aminocarbonyl)phenylsulfonyl)amino)carbonyl]-pyridin-2-carbonsäure-cyclohexylester

### Beispiel 219

5-[((4-((2-Phenoxyethyl)aminocarbonyl)phenylsulfonyl)amino)carbonyl]-pyridin-2-carbonsäure-cyclohexylester

### Beispiel 220

5-[((3-(Benzylaminocarbonyl)phenylsulfonyl)amino)carbonyl]-pyridin-2-carbonsäure-2-propylester

### Beispiel 221

5-[((3-((3-Phenylpropyl)aminocarbonyl)phenylsulfonyl)amino)carbonyl]-pyridin-2-carbonsäure-2-propylester

### Beispiel 222

5-[((3-((4-Phenyl-n-butyl)aminocarbonyl)phenylsulfonyl)amino)carbonyl]-pyridin-2-carbonsäure-2-propylester

### Beispiel 223

5-[((3-((2-(4-Methoxyphenyl)ethyl)aminocarbonyl)phenylsulfonyl)amino) carbonyl]-pyridin-2-carbonsäure-2-propylester

### Beispiel 224

5-[((3-((2-(3-Methoxyphenyl)ethyl)aminocarbonyl)phenylsulfonyl)amino) carbonyl]-pyridin-2-carbonsäure-2-propylester

### Beispiel 225

5-[((3-((2-(2-Methoxyphenyl)ethyl)aminocarbonyl)phenylsulfonyl)amino) carbonyl]-pyridin-2-carbonsäure-2-propylester

### Beispiel 226

5-[((3-((2-(4-Fluorphenyl)ethyl)aminocarbonyl)phenylsulfonyl)amino)carbonyl]-pyridin-2-carbonsäure-2-propylester

### Beispiel 227

5-[((3-((2-(4-Chlorphenyl)ethyl)aminocarbonyl)phenylsulfonyl)amino)carbonyl]-pyridin-2-carbonsäure-2-propylester

### Beispiel 228

5-[((3-(Ethylaminocarbonyl)phenylsulfonyl)amino)carbonyl]-pyridin-2-carbonsäure-2-propylester

### Beispiel 229

5-[((3-(n-Butylaminocarbonyl)phenylsulfonyl)amino)carbonyl]-pyridin-2-carbonsäure-2-propylester

### Beispiel 230

5-[((3-(n-Hexylaminocarbonyl)phenylsulfonyl)amino)carbonyl]-pyridin-2-carbonsäure-2-propylester

### Beispiel 231

5-[((3-(N,N-Di-n-butylaminocarbonyl)phenylsulfonyl)amino)carbonyl]-pyridin-2-carbonsäure-2-propylester

### Beispiel 232

5-[((3-(Cyclohexylaminocarbonyl)phenylsulfonyl)amino)carbonyl]-pyridin-2-carbonsäure-2-propylester

### Beispiel 233

5-[((3-((2-Methoxyethyl)aminocarbonyl)phenylsulfonyl)amino)carbonyl]-pyridin-2-carbonsäure-2-propylester

### Beispiel 234

5-[((3-((2-Ethoxyethyl)aminocarbonyl)phenylsulfonyl)amino)carbonyl]-pyridin-2-carbonsäure-2-propylester

### Beispiel 235

5-[((3-((3-Ethoxypropyl)aminocarbonyl)phenylsulfonyl)amino)carbonyl]-pyridin-2-carbonsäure-2-propylester

### Beispiel 236

5-[((3-((2-Phenylethyl)aminocarbonyl)phenylsulfonyl)amino)carbonyl]-pyridin-2-carbonsäure-2-propylester

### Beispiel 237

5-[((3-((2-Phenoxyethyl)aminocarbonyl)phenylsulfonyl)amino)carbonyl]-pyridin-2-carbonsäure-2-propylester

### Beispiel 238

5-[((4-(Benzylaminocarbonyl)phenylsulfonyl)amino)carbonyl]-pyridin-2-carbonsäure-4-heptylester

### Beispiel 239

5-[((4-((3-Phenylpropyl)aminocarbonyl)phenylsulfonyl)amino)carbonyl]-pyridin-2-carbonsäure-4-heptylester

### Beispiel 240

5-[((4-((4-Phenyl-n-butyl)aminocarbonyl)phenylsulfonyl)amino)carbonyl]-pyridin-2-carbonsäure-4-heptylester

### Beispiel 241

5-[((4-((2-(4-Methoxyphenyl)ethyl)aminocarbonyl)phenylsulfonyl)amino) carbonyl]-pyridin-2-carbonsäure-4-heptylester

### Beispiel 242

5-[((4-((2-(3-Methoxyphenyl)ethyl)aminocarbonyl)phenylsulfonyl)amino) carbonyl]-pyridin-2-carbonsäure-4-heptylester

### Beispiel 243

5-[((4-((2-(2-Methoxyphenyl)ethyl)aminocarbonyl)phenylsulfonyl)amino) carbonyl]-pyridin-2-carbonsäure-4-heptylester

### Beispiel 244

5-[((4-((2-(4-Fluorphenyl)ethyl)aminocarbonyl)phenylsulfonyl)amino)carbonyl]-pyridin-2-carbonsäure-4-heptylester

### Beispiel 245

5-[((4-((2-(4-Chlorphenyl)ethyl)aminocarbonyl)phenylsulfonyl)amino)carbonyl]-pyridin-2-carbonsäure-4-heptylester

### Beispiel 246

5-[((4-(Ethylaminocarbonyl)phenylsulfonyl)amino)carbonyl]-pyridin-2-carbonsäure-4-heptylester

### Beispiel 247

5-[((4-(n-Butylaminocarbonyl)phenylsulfonyl)amino)carbonyl]-pyridin-2-carbonsäure-4-heptylester

### Beispiel 248

5-[((4-(n-Hexylaminocarbonyl)phenylsulfonyl)amino)carbonyl]-pyridin-2-carbonsäure-4-heptylester

### Beispiel 249

5-[((4-(N,N-Di-n-butylaminocarbonyl)phenylsulfonyl)amino)carbonyl]-pyridin-2-carbonsäure-4-heptylester

### Beispiel 250

5-[((4-(Cyclohexylaminocarbonyl)phenylsulfonyl)amino)carbonyl]-pyridin-2-carbonsäure-4-heptylester

### Beispiel 251

5-[((4-((2-Methoxyethyl)aminocarbonyl)phenylsulfonyl)amino)carbonyl]-pyridin-2-carbonsäure-4-heptylester

### Beispiel 252

5-[((4-((2-Ethoxyethyl)aminocarbonyl)phenylsulfonyl)amino)carbonyl]-pyridin-2-carbonsäure-4-heptylester

### Beispiel 253

5-[((4-((3-Ethoxypropyl)aminocarbonyl)phenylsulfonyl)amino)carbonyl]-pyridin-2-carbonsäure-4-heptylester

### Beispiel 254

5-[((4-((2-Phenylethyl)aminocarbonyl)phenylsulfonyl)amino)carbonyl]-pyridin-2-carbonsäure-4-heptylester

### Beispiel 255

5-[((4-((2-Phenoxyethyl)aminocarbonyl)phenylsulfonyl)amino)carbonyl]-pyridin-2-carbonsäure-4-heptylester

### Beispiel 256

5-[((4-(Benzylaminocarbonyl)phenylsulfonyl)amino)carbonyl]-pyridin-2-carbonsäure-2-(2-methylpropyl)ester

### Beispiel 257

5-[((4-((3-Phenylpropyl)aminocarbonyl)phenylsulfonyl)amino)carbonyl]-pyridin-2-carbonsäure-2-(2-methylpropyl)ester

### Beispiel 258

5-[((4-((4-Phenyl-n-butyl)aminocarbonyl)phenylsulfonyl)amino)carbonyl]-pyridin-2-carbonsäure-2-(2-methylpropyl)ester

### Beispiel 259

5-[((4-((2-(4-Methoxyphenyl)ethyl)aminocarbonyl)phenylsulfonyl)amino) carbonyl]-pyridin-2-carbonsäure-2-(2-methylpropyl)ester

### Beispiel 260

5-[((4-((2-(3-Methoxyphenyl)ethyl)aminocarbonyl)phenylsulfonyl)amino) carbonyl]-pyridin-2-carbonsäure-2-(2-methylpropyl)ester

### Beispiel 261

5-[((4-((2-(2-Methoxyphenyl)ethyl)aminocarbonyl)phenylsulfonyl)amino) carbonyl]-pyridin-2-carbonsäure-2-(2-methylpropyl)ester

### Beispiel 262

5-[((4-((2-(4-Fluorphenyl)ethyl)aminocarbonyl)phenylsulfonyl)amino)carbonyl]-pyridin-2-carbonsäure-2-(2-methylpropyl)ester

### Beispiel 263

5-[((4-((2-(4-Chlorphenyl)ethyl)aminocarbonyl)phenylsulfonyl)amino)carbonyl]-pyridin-2-carbonsäure-2-(2-methylpropyl)ester

### Beispiel 264

5-[((4-(Ethylaminocarbonyl)phenylsulfonyl)amino)carbonyl]-pyridin-2-carbonsäure-2-(2-methylpropyl)ester

### Beispiel 265

5-[((4-(n-Butylaminocarbonyl)phenylsulfonyl)amino)carbonyl]-pyridin-2-carbonsäure-1-(2-methylpropyl)ester

### Beispiel 266

5-[((4-(n-Hexylaminocarbonyl)phenylsulfonyl)amino)carbonyl]-pyridin-2-carbonsäure-1-(2-methylpropyl)ester

### Beispiel 267

5-[((4-(N,N-Di-n-butylaminocarbonyl)phenylsulfonyl)amino)carbonyl]-pyridin-2-carbonsäure-1-(2-methylpropyl)ester

### Beispiel 268

5-[((4-(Cyclohexylaminocarbonyl)phenylsulfonyl)amino)carbonyl]-pyridin-2-carbonsäure-1-(2-methylpropyl)ester

### Beispiel 269

5-[((4-((2-Methoxyethyl)aminocarbonyl)phenylsulfonyl)amino)carbonyl]-pyridin-2-carbonsäure-1-(2-methylpropyl)ester

### Beispiel 270

5-[((4-((2-Ethoxyethyl)aminocarbonyl)phenylsulfonyl)amino)carbonyl]-pyridin-2-carbonsäure-1-(2-methylpropyl)ester

### Beispiel 271

5-[((4-((3-Ethoxypropyl)aminocarbonyl)phenylsulfonyl)amino)carbonyl]-pyridin-2-carbonsäure-1-(2-methylpropyl)ester

### Beispiel 272

5-[((4-((2-Phenylethyl)aminocarbonyl)phenylsulfonyl)amino)carbonyl]-pyridin-2-carbonsäure-1-(2-methylpropyl)ester

### Beispiel 273

5-[((4-((2-Phenoxyethyl)aminocarbonyl)phenylsulfonyl)amino)carbonyl]-pyridin-2-carbonsäure-1-(2-methylpropyl)ester

### Beispiel 274

5-[((4-(Benzylaminocarbonyl)phenylsulfonyl)amino)carbonyl]-pyridin-2-carbonsäure-3-(2,5-dimethylpentyl)ester

### Beispiel 275

5-[((4-((3-Phenylpropyl)aminocarbonyl)phenylsulfonyl)amino)carbonyl]-pyridin-2-carbonsäure-3-(2,5-dimethylpentyl)ester

### Beispiel 276

5-[((4-((4-Phenyl-n-butyl)aminocarbonyl)phenylsulfonyl)amino)carbonyl]-pyridin-2-carbonsäure-3-(2,5-dimethylpentyl)ester

### Beispiel 277

5-[((4-((2-(4-Methoxyphenyl)ethyl)aminocarbonyl)phenylsulfonyl)amino)carbonyl]-pyridin-2-carbonsäure-3-(2,5-dimethylpentyl)ester

### Beispiel 278

5-[((4-((2-(3-Methoxyphenyl)ethyl)aminocarbonyl)phenylsulfonyl)amino) carbonyl]-pyridin-2-carbonsäure-3-(2,5-dimethylpentyl)ester

### Beispiel 279

5-[((4-((2-(2-Methoxyphenyl)ethyl)aminocarbonyl)phenylsulfonyl)amino) carbonyl]-pyridin-2-carbonsäure-3-(2,5-dimethylpentyl)ester

### Beispiel 280

5-[((4-((2-(4-Fluorphenyl)ethyl)aminocarbonyl)phenylsulfonyl)amino)carbonyl]-pyridin-2-carbonsäure-3-(2,5-dimethylpentyl)ester

### Beispiel 281

5-[((4-((2-(4-Chlorphenyl)ethyl)aminocarbonyl)phenylsulfonyl)amino)carbonyl]-pyridin-2-carbonsäure-3-(2,5-dimethylpentyl)ester

### Beispiel 282

5-[((4-(Ethylaminocarbonyl)phenylsulfonyl)amino)carbonyl]-pyridin-2-carbonsäure-3-(2,5-dimethylpentyl)ester

### Beispiel 283

5-[((4-(n-Butylaminocarbonyl)phenylsulfonyl)amino)carbonyl]-pyridin-2-carbonsäure-3-(2,5-dimethylpentyl)ester

### Beispiel 284

5-[((4-(n-Hexylaminocarbonyl)phenylsulfonyl)amino)carbonyl]-pyridin-2-carbonsäure-3-(2,5-dimethylpentyl)ester

### Beispiel 285

5-[((4-(N,N-Di-n-butylaminocarbonyl)phenylsulfonyl)amino)carbonyl]-pyridin-2-carbonsäure-3-(2,5-dimethylpentyl)ester

### Beispiel 286

5-[((4-(Cyclohexylaminocarbonyl)phenylsulfonyl)amino)carbonyl]-pyridin-2-carbonsäure-3-(2,5-dimethylpentyl)ester

### Beispiel 287

5-[((4-((2-Methoxyethyl)aminocarbonyl)phenylsulfonyl)amino)carbonyl]-pyridin-2-carbonsäure-3-(2,5-dimethylpentyl)ester

### Beispiel 288

5-[((4-((2-Ethoxyethyl)aminocarbonyl)phenylsulfonyl)amino)carbonyl]-pyridin-2-carbonsäure-3-(2,5-dimethylpentyl)ester

### Beispiel 289

5-[((4-((3-Ethoxypropyl)aminocarbonyl)phenylsulfonyl)amino)carbonyl]-pyridin-2-carbonsäure-3-(2,5-dimethylpentyl)ester

### Beispiel 290

5-[((4-((2-Phenylethyl)aminocarbonyl)phenylsulfonyl)amino)carbonyl]-pyridin-2-carbonsäure-3-(2,5-dimethylpentyl)ester

### Beispiel 291

5-[((4-((2-Phenoxyethyl)aminocarbonyl)phenylsulfonyl)amino)carbonyl]-pyridin-2-carbonsäure-benzylester

### Beispiel 292

5-[((4-(Benzylaminocarbonyl)phenylsulfonyl)amino)carbonyl]-pyridin-2-carbonsäure-benzylester

### Beispiel 293

5-[((4-((3-Phenylpropyl)aminocarbonyl)phenylsulfonyl)amino)carbonyl]-pyridin-2-carbonsäure-benzylester

### Beispiel 294

5-[((4-((4-Phenyl-n-butyl)aminocarbonyl)phenylsulfonyl)amino)carbonyl]-pyridin-2-carbonsäure-benzylester

### Beispiel 295

5-[((4-((2-(4-Methoxyphenyl)ethyl)aminocarbonyl)phenylsulfonyl)amino) carbonyl]-pyridin-2-carbonsäure-benzylester

### Beispiel 296

5-[((4-((2-(3-Methoxyphenyl)ethyl)aminocarbonyl)phenylsulfonyl)amino) carbonyl]-pyridin-2-carbonsäure-benzylester

### Beispiel 297

5-[((4-((2-(2-Methoxyphenyl)ethyl)aminocarbonyl)phenylsulfonyl)amino) carbonyl]-pyridin-2-carbonsäure-benzylester

### Beispiel 298

5-[((4-((2-(4-Fluorphenyl)ethyl)aminocarbonyl)phenylsulfonyl)amino)carbonyl]-pyridin-2-carbonsäure-2-benzylester

### Beispiel 299

5-[((4-((2-(4-Chlorphenyl)ethyl)aminocarbonyl)phenylsulfonyl)amino)carbonyl]-pyridin-2-carbonsäure-benzylester

### Beispiel 300

5-[((4-(Ethylaminocarbonyl)phenylsulfonyl)amino)carbonyl]-pyridin-2-carbonsäure-benzylester

### Beispiel 301

5-[((4-(n-Butylaminocarbonyl)phenylsulfonyl)amino)carbonyl]-pyridin-2-carbonsäure-cyclopentylester

### Beispiel 302

5-[((4-(n-Hexylaminocarbonyl)phenylsulfonyl)amino)carbonyl]-pyridin-2-carbonsäure-cyclopentylester

### Beispiel 303

5-[((4-(N,N-Di-n-butylaminocarbonyl)phenylsulfonyl)amino)carbonyl]-pyridin-2-carbonsäure-cyclopentylester

### Beispiel 304

5-[((4-(Cyclohexylaminocarbonyl)phenylsulfonyl)amino)carbonyl]-pyridin-2-carbonsäure-cyclopentylester

### Beispiel 305

5-[((4-((2-Methoxyethyl)aminocarbonyl)phenylsulfonyl)amino)carbonyl]-pyridin-2-carbonsäure-cyclopentylester

### Beispiel 306

5-[((4-((2-Ethoxyethyl)aminocarbonyl)phenylsulfonyl)amino)carbonyl]-pyridin-2-carbonsäure-cyclopentylester

### Beispiel 307

5-[((4-((3-Ethoxypropyl)aminocarbonyl)phenylsulfonyl)amino)carbonyl]-pyridin-2-carbonsäure-cyclopentylester

### Beispiel 308

5-[((4-((2-Phenylethyl)aminocarbonyl)phenylsulfonyl)amino)carbonyl]-pyridin-2-carbonsäure-cyclopentylester

### Beispiel 309

5-[((4-((2-Phenoxyethyl)aminocarbonyl)phenylsulfonyl)amino)carbonyl]-pyridin-2-carbonsäure-cyclopentylester

### Beispiel 310

5-[((2-Chlor-4-((2-phenylethyl)aminocarbonyl)phenylsulfonyl)amino)carbonyl]-pyridin-2-carbonsäure-methylester-Na-Salz
Fp. 258 bis 260°C (aus Ethylacetat)

### Beispiel 311

5-[((2-Chlor-4-((3-ethoxypropyl)aminocarbonyl)phenylsulfonyl)amino)carbonyl]-pyridin-2-carbonsäure-methylester
Fp. 164 bis 166°C (aus Diethylether)

### Beispiel 312

5-[((4-Chlor-3-((3-ethoxypropyl)aminocarbonyl)phenylsulfonyl)amino)carbonyl]-pyridin-2-carbonsäure-methylester

### Beispiel 313

5-[((4-Chlor-3-((2-phenoxyethyl)aminocarbonyl)phenylsulfonyl)amino)carbonyl]-pyridin-2-carbonsäure-methylester

### Beispiel 314

5-[((2-Chlor-4-((2-phenylethyl)aminocarbonyl)phenylsulfonyl)amino)carbonyl]-pyridin-2-carbonsäure-2-propylester

### Beispiel 315

5-[((2-Chlor-4-((3-ethoxypropyl)aminocarbonyl)phenylsulfonyl)amino)carbonyl]-pyridin-2-carbonsäure-2-propylester

### Beispiel 316

5-[((4-Chlor-3-((3-ethoxypropyl)aminocarbonyl)phenylsulfonyl)amino)carbonyl]-pyridin-2-carbonsäure-2-propylester

### Beispiel 317

5-[((4-Chlor-3-((2-phenoxyethyl)aminocarbonyl)phenylsulfonyl)amino)carbonyl]-pyridin-2-carbonsäure-2-propylester

### Beispiel 318

5-[((2-Chlor-4-((2-phenylethyl)aminocarbonyl)phenylsulfonyl)amino)carbonyl]-pyridin-2-carbonsäure-2-(methylbutyl)ester

### Beispiel 319

5-[((2-Chlor-4-((3-ethoxypropyl)aminocarbonyl)phenylsulfonyl)amino)carbonyl]-pyridin-2-carbonsäure-2-(2-methylbutyl)ester

### Beispiel 320

5-[((4-Chlor-3-((3-ethoxypropyl)aminocarbonyl)phenylsulfonyl)amino)carbonyl]-pyridin-2-carbonsäure-2-(2-methylbutyl)ester

### Beispiel 321

5-[((4-Chlor-3-((2-phenylethyl)aminocarbonyl)phenylsulfonyl)amino)carbonyl]-pyridin-2-carbonsäure-2-(2-methylbutyl)ester

### Beispiel 322

5-[((2-Chlor-4-((2-phenoxyethyl)aminocarbonyl)phenylsulfonyl)amino)carbonyl]-pyridin-2-carbonsäure-2-(2-methylhexyl)ester

### Beispiel 323

5-[((2-Chlor-4-((3-ethoxypropyl)aminocarbonyl)phenylsulfonyl)amino)carbonyl]-pyridin-2-carbonsäure-2-(2-methylhexyl)ester

### Beispiel 324

5-[((2-Chlor-4-((2-phenoxyethyl)aminocarbonyl)phenylsulfonyl)amino)carbonyl]-pyridin-2-carbonsäure-2-(2-methylhexyl)ester

### Beispiel 325

5-[((2-Chlor-4-((3-ethoxypropyl)aminocarbonyl)phenylsulfonyl)amino)carbonyl]-pyridin-2-carbonsäure-2-(2-methylhexyl)ester

### Beispiel 326

5-[((4-Chlor-3-((3-ethoxypropyl)aminocarbonyl)phenylsulfonyl)amino)carbonyl]-pyridin-2-carbonsäure-cyclohexylester

### Beispiel 327

5-[((4-Chlor-3-((2-phenoxyethyl)aminocarbonyl)phenylsulfonyl)amino)carbonyl]-pyridin-2-carbonsäure-cyclohexylester

### Beispiel 328

5-[((2-Chlor-4-((2-phenylethyl)aminocarbonyl)phenylsulfonyl)amino)carbonyl]-pyridin-2-carbonsäure-cyclohexylester

### Beispiel 329

5-[((2-Chlor-4-((3-ethoxypropyl)aminocarbonyl)phenylsulfonyl)amino)carbonyl]-pyridin-2-carbonsäure-cyclohexylester

### Beispiel 330

5-[((2-Chlor-4-((2-phenylethyl)aminocarbonyl)phenylsulfonyl)amino)carbonyl]-pyridin-2-carbonsäure-(methylcyclohexyl)ester

### Beispiel 331

5-[((2-Chlor-4-((3-ethoxypropyl)aminocarbonyl)phenylsulfonyl)amino)carbonyl]-pyridin-2-carbonsäure-(methylcyclohexyl)ester

### Beispiel 332

5-[((4-(2-((2-Chlor-5-methoxybenzoyl)amino)ethyl)phenylsulfonyl)amino) carbonyl]-pyridin-2-carbonsäure-ethylester

### Beispiel 333

5-[((4-(2-(Acetylamino)ethyl)phenylsulfonyl)amino)carbonyl]-pyridin-2-carbonsäure-ethylester (Methode B)
Fp. 245°C (aus wäßriger Salzsäure)

### Beispiel 334

5-[((4-(2-(n-Butanoylamino)ethyl)phenylsulfonyl)amino)carbonyl]-pyridin-2-carbonsäure-ethylester

### Beispiel 335

5-[((4-(2-(Benzoylamino)ethyl)phenylsulfonyl)amino)carbonyl]-pyridin-2-carbonsäure-ethylester

### Beispiel 336

5-[((4-(2-((4-Chlorbenzoyl)amino)ethyl)phenylsulfonyl)amino)carbonyl]-pyridin-2-carbonsäure-ethylester

### Beispiel 337

5-[((4-(2-((5-Chlor-2-methoxybenzoyl)amino)ethyl)phenylsulfonyl)amino) carbonyl]-pyridin-2-carbonsäure-ethylester

### Beispiel 338

5-[((4-(2-((3-Phenyl-n-propionyl)amino)ethyl)phenylsulfonyl)amino)carbonyl]-pyridin-2-carbonsäure-ethylester

### Beispiel 339

5-[((4-(2-((2-Phenylacetyl)amino)ethyl)phenylsulfonyl)amino)carbonyl]-pyridin-2-carbonsäure-ethylester

### Beispiel 340

5-[((4-(2-((Phenoxyacetyl)amino)ethyl)phenylsulfonyl)amino)carbonyl]-pyridin-2-carbonsäure-ethylester

### Beispiel 341

5-[((4-(2-((4-Fluorbenzoyl)amino)ethyl)phenylsulfonyl)amino)carbonyl]-pyridin-2-carbonsäure-ethylester

### Beispiel 342

5-[((4-(2-((4-Ethoxybenzoyl)amino)ethyl)phenylsulfonyl)amino]carbonyl]-pyridin-2-carbonsäure-ethylester

### Beispiel 343

5-[((4-(2-((Cyclohexanoyl)amino)ethyl)phenylsulfonyl)amino)carbonyl]-pyridin-2-carbonsäure-ethylester

### Beispiel 344

5-[((4-(2-((Cyclohexylacetyl)amino)ethyl)phenylsulfonyl)amino)carbhonyl]-pyridin-2-carbonsäure-ethylester

### Beispiel 345

5-[((4-(2-((2-Methylpropionyl)amino)ethyl)phenylsulfonyl)amino)carbonyl]-pyridin-2-carbonsäure-ethylester

### Beispiel 346

5-[((3-(2-((2-Chlor-5-methoxybenzoyl)amino)ethyl)phenylsulfonyl)amino) carbonyl]-pyridin-2-carbonsäure-methylester

### Beispiel 347

5-[((3-(2-(Acetylamino)ethyl)phenylsulfonyl)amino)carbonyl]-pyridin-2-carbonsäure-methylester

### Beispiel 348

5-[((3-(2-(n-Butanoylamino)ethyl)phenylsulfonyl)amino)carbonyl]-pyridin-2-carbonsäure-methylester

### Beispiel 349

5-[((3-(2-(Benzoylamino)ethyl)phenylsulfonyl)amino)carbonyl]-pyridin-2-carbonsäure-methylester

### Beispiel 350

5-[((3-(2-((4-Chlorbenzoyl)amino)ethyl)phenylsulfonyl)amino)carbonyl]-pyridin-2-carbonsäure-methylester

### Beispiel 351

5-[((3-(2-((5-Chlor-2-methoxybenzoyl)amino)ethyl)phenylsulfonyl)amino) carbonyl]-pyridin-2-carbonsäure-methylester

### Beispiel 352

5-[((3-(2-((3-Phenyl-n-propionyl)amino)ethyl)phenylsulfonyl)amino)carbonyl]-pyridin-2-carbonsäure-methylester

### Beispiel 353

5-[((3-(2-((2-Phenylacetyl)amino)ethyl)phenylsulfonyl)amino)carbonyl]-pyridin-2-carbonsäure-methylester

### Beispiel 354

5-[((3-(2-((Phenoxyacetyl)amino)ethyl)phenylsulfonyl)amino)carbonyl]-pyridin-2-carbonsäure-methylester

### Beispiel 355

5-[((3-(2-((4-Fluorbenzoyl)amino)ethyl)phenylsulfonyl)amino)carbonyl]-pyridin-2-carbonsäure-methylester

### Beispiel 356

5-[((3-(2-((4-Ethoxybenzoyl)amino)ethyl)phenylsulfonyl)amino)carbonyl]-pyridin-2-carbonsäure-methylester

### Beispiel 357

5-[((3-(2-((Cyclohexanoyl)amino)ethyl)phenylsulfonyl)amino)carbonyl]-pyridin-2-carbonsäure-methylester

### Beispiel 358

5-[((3-(2-((Cyclohexylacetyl)amino)ethyl)phenylsulfonyl)amino)carbhonyl]-pyridin-2-carbonsäure-methylester

### Beispiel 359

5-[((3-(2-((2-Methylpropionyl)amino)ethyl)phenylsulfonyl)amino)carbonyl]-pyridin-2-carbonsäure-methylester

### Beispiel 360

5-[((4-(2-((2-Chlor-5-methoxybenzoyl)amino)ethyl)phenylsulfonyl)amino) carbonyl]-pyridin-2-carbonsäure-2-propylester

### Beispiel 361

5-[((4-(2-(Acetylamino)ethyl)phenylsulfonyl)amino)carbonyl]pyridin-2-carbonsäure-2-propylester

### Beispiel 362

5-[((4-(2-(n-Butanoylamino)ethyl)phenylsulfonyl)amino)carbonyl]-pyridin-2-carbonsäure-2-propylester

### Beispiel 363

5-[((4-(2-(Benzoylamino)ethyl)phenylsulfonyl)amino)carbonyl]-pyridin-2-carbonsäure-2-propylester

### Beispiel 364

5-[((4-(2-((4-Chlorbenzoyl)amino)ethyl)phenylsulfonyl)amino)carbonyl]-pyridin-2-carbonsäure-2-propylester

### Beipsiel 365

5-[((4-(2-((5-Chlor-2-methoxybenzoyl)amino)ethyl)phenylsulfonyl)amino) carbonyl]-pyridin-2-carbonsäure-2-propylester

### Beispiel 366

5-[((4-(2-((3-Phenyl-n-propionyl)amino)ethyl)phenylsulfonyl)amino)carbonyl]-pyridin-2-carbonsäure-2-propylester

### Beispiel 367

5-[((4-(2-((2-Phenylacetyl)amino)ethyl)phenylsulfonyl)amino)carbonyl]-pyridin-2 carbonsäure-2-propylester

### Beispiel 368

5-[((4-(2-((Phenoxyacetyl)amino)ethyl)phenylsulfonyl)amino)carbonyl]-pyridin-2-carbonsäure-2-propylester

### Beispiel 369

5-[((4-(2-((4-Fluorbenzoyl)amino)ethyl)phenylsulfonyl)amino)carbonyl]-pyridin-2-carbonsäure-2-propylester

### Beispiel 370

5-[((4-(2-((4-Ethoxybenzoyl)amino)ethyl)phenylsulfonyl)amino)carbonyl]-pyridin-2-carbonsäure-2-propylester

### Beispiel 371

5-[((4-(2-((Cyclohexanoyl)amino)ethyl)phenylsulfonyl)amino)carbonyl]-pyridin-2-carbonsäure-2-propylester

### Beispiel 372

5-[((4-(2-((Cyclohexylacetyl)amino)ethyl)phenylsulfonyl)amino)carbonyl]-pyridin-2-carbonsäure-2-propylester

### Beispiel 373

5-[((4-(2-((2-Methylpropionyl)amino)ethyl)phenylsulfonyl)amino)carbonyl]-pyridin-2-carbonsäure-2-propylester

### Beispiel 374

5-[((3-(2-((2-Chlor-5-methoxybenzoyl)amino)ethyl)phenylsulfonyl)amino) carbonyl]-pyridin-2-carbonsäure-3-pentylester
a) 5-[((4-(2-((2-Chlor-5-methoxybenzoyl)amino)ethyl)phenylsulfonyl)amino) carbonyl]-pyridin-2-carbonsäure
   0,8 g (1,36 mmol) des Methylesters aus Beispiel 68 wurden analog Beispiel 112 a) mit 30 ml 1n methanolischer NaOH verseift. Nach dem Einengen i. Vak. wurde der Rückstand in Tetrahydrofuran gelöst, mit 2n wäßriger HCl angesäuert, eingeengt, der Rückstand mit Wasser behandelt und abgesaugt. Man isolierte 0,75 g Produkt, Fp. 149°C (Zers.)
b) Die Titelverbindung wurde analog Beispiel 2c) erhalten. 1,04 g (2 mmol) der vorstehenden Pyridin-2-carbonsäure wurden in 40 ml 3-Pentanol mit 4 Tropfen konz. H₂SO₄ versetzt und 24 h unter Rühren auf 80°C erhitzt. Man engte i. Vak. ein, brachte den harzigen Rückstand mit Aceton zur Kristallisation und erhielt 0,32 g Rohprodukt; Fp. 174 bis 176°C. Die Mutterlauge wurde eingeengt, der Rückstand in Dichlormethan gelöst und mit gesättigter wäßriger NaHCO₃-Lösung geschüttelt. Die organische Phase wurde getrocknet und das Na-Salz des Produkts mit Aceton zur Kristallisation gebracht. Dieses wurde in Tetrahydrofuran suspendiert und mit wäßriger HCl angesäuert, die entstandene klare Lösung wurde mit dem krstallinenen Rohprodukt vereinigt, eingeengt und der Rückstand mit Ethylacetat zur Kristallisation gebracht. Man erhielt 0,54 g farbloses Produkt; Fp. 164 bis 166°C.

### Beispiel 375

5-[((3-(2-(Acetylamino)ethyl)phenylsulfonyl)amino)carbonyl]-pyridin-2-carbonsäure-3-pentylester

### Beispiel 376

5-[((3-(2-(n-Butanoylamino)ethyl)phenylsulfonyl)amino)carbonyl]-pyridin-2-carbonsäure-3-pentylester

### Beispiel 377

5-[((3-(2-(Benzoylamino)ethyl)phenylsulfonyl)amino)carbonyl]-pyridin-2-carbonsäure-3-pentylester

### Beispiel 378

5-[((3-(2-((4-Chlorbenzoyl)amino)ethyl)phenylsulfonyl)amino)carbonyl]-pyridin-2-carbonsäure-3-pentylester

### Beipsiel 379

5-[((3-(2-((5-Chlor-2-methoxybenzoyl)amino)ethyl)phenylsulfonyl)amino) carbonyl]-pyridin-2-carbonsäure-3-pentylester

### Beispiel 380

5-[((3-(2-((3-Phenyl-n-propionyl)amino)ethyl)phenylsulfonyl)amino)carbonyl]-pyridin-2-carbonsäure-3-pentylester

### Beispiel 381

5-[((3-(2-((2-Phenylacetyl)amino)ethyl)phenylsulfonyl)amino)carbonyl]-pyridin-2-carbonsäure-3-pentylester

### Beispiel 382

5-[((3-(2-((Phenoxyacetyl)amino)ethyl)phenylsulfonyl)amino)carbonyl]-pyridin-2-carbonsäure-3-pentylester

### Beispiel 383

5-[((3-(2-((4-Fluorbenzoyl)amino)ethyl)phenylsulfonyl)amino)carbonyl]-pyridin-2-carbonsäure-3-pentylester

### Beispiel 384

5-[((3-(2-((4-Ethoxybenzoyl)amino)ethyl)phenylsulfonyl)amino)carbonyl]-pyridin-2-carbonsäure-3-pentylester

### Beispiel 385

5-[((3-(2-((Cyclohexanoyl)amino)ethyl)phenylsulfonyl)amino)carbonyl]-pyridin-2-carbonsäure-3-pentylester

### Beispiel 386

5-[((3-(2-((Cyclohexylacetyl)amino)ethyl)phenylsulfonyl)amino)carbonyl]-pyridin-2-carbonsäure-3-pentylester

### Beispiel 387

5-[((3-(2-((2-Methylpropionyl)amino)ethyl)phenylsulfonyl]amino)carbonyl]-pyridin-2-carbonsäure-3-pentylester

### Beispiel 388

5-[((4-(2-((2-Chlor-5-methoxybenzoyl)amino)ethyl)phenylsulfonyl)amino) carbonyl]-pyridin-2-carbonsäure-3-pentylester

### Beispiel 389

5-[((4-(2-(Acetylamino)ethyl)phenylsulfonyl)amino)carbonyl]-pyridin-2-carbonsäure-3-pentylester

### Beispiel 390

5-[((4-(2-(n-Butanoylamino)ethyl)phenylsulfonyl)amino)carbonyl]-pyridin-2-carbonsäure-3-pentylester

### Beispiel 391

5-[((4-(2-(Benzoylamino)ethyl)phenylsulfonyl)amino)carbonyl]-pyridin-2-carbonsäure-3-pentylester

### Beispiel 392

5-[((4-(2-((4-Chlorbenzoyl)amino)ethyl)phenylsulfonyl)amino)carbonyl]-pyridin-2-carbonsäure-3-pentylester

### Beispiel 393

5-[((4-(2-((5-Chlor-2-methoxybenzoyl)amino)ethyl)phenylsulfonyl)amino) carbonyl]-pyridin-2-carbonsäure-3-pentylester

### Beispiel 394

5-[((4-(2-((3-Phenyl-n-propionyl)amino)ethyl)phenylsulfonyl)amino)carbonyl]-pyridin-2-carbonsäure-3-pentylester

### Beispiel 395

5-[((4-(2-((2-Phenylacetyl)amino)ethyl)phenylsulfonyl)amino)carbonyl]-pyridin-2-carbonsäure-3-pentylester

### Beispiel 396

5-[((4-(2-((Phenoxyacetyl)amino)ethyl)phenylsulfonyl)amino)carbonyl]-pyridin-2-carbonsäure-3-pentylester

### Beispiel 397

5-[((4-(2-((4-Fluorbenzoyl)amino)ethyl)phenylsulfonyl)amino)carbonyl]-pyridin-2-carbonsäure-3-pentylester

### Beispiel 398

5-[((4-(2-((4-Ethoxybenzoyl)amino)ethyl)phenylsulfonyl)amino)carbonyl]-pyridin-2-carbonsäure-3-pentylester

### Beispiel 399

5-[((4-(2-((Cyclohexanoyl)amino)ethyl)phenylsulfonyl)amino)carbonyl]-pyridin-2-carbonsäure-3-pentylester

### Beispiel 400

5-[((4-(2-((Cyclohexylacetyl)amino)ethyl)phenylsulfonyl)amino)carbonyl]-pyridin-2-carbonsäure-3-pentylester

### Beispiel 401

5-[((4-(2-((2-Methylpropionyl)amino)ethyl]phenylsulfonyl)amino)carbonyl]-pyridin-2-carbonsäure-3-pentylester

### Beispiel 402

5-[((4-(2-((2-Chlor-5-methoxybenzoyl)amino)ethyl)phenylsulfonyl)amino) carbonyl]-pyridin-2-carbonsäure-2-(2-methylhexyl)ester

### Beispiel 403

5-[((4-(2-(Acetylamino)ethyl)phenylsulfonyl)amino)carbonyl]-pyridin-2-carbonsäure-2-(2-methylhexyl)ester

### Beispiel 404

5-[((4-(2-(n-Butanoylamino)ethyl)phenylsulfonyl)amino)carbonyl]-pyridin-2-carbonsäure-2-(2-methylhexyl)ester

### Beispiel 405

5-[((4-(2-(Benzoylamino)ethyl)phenylsulfonyl)amino)carbonyl]-pyridin-2-carbonsäure-2-(2-methylhexyl)ester

### Beispiel 406

5-[((4-(2-((4-Chlorbenzoyl)amino)ethyl)phenylsulfonyl)amino)carbonyl]-pyridin-2-carbonsäure-2-(2-methylhexyl)ester

### Beispiel 407

5-[((4-(2-((5-Chlor-2-methoxybenzoyl)amino)ethyl)phenylsulfonyl)amino) carbonyl]-pyridin-2-carbonsäure-2-(2-methylhexyl)ester

### Beispiel 408

5-[((4-(2-((3-Phenyl-n-propionyl)amino)ethyl)phenylsulfonyl)amino)carbonyl]-pyridin-2-carbonsäure-2-(2-methylhexyl)ester

### Beispiel 409

5-[((4-(2-((2-Phenylacetyl)amino)ethyl)phenylsulfonyl)amino)carbonyl]-pyridin-2-carbonsäure-2-(2-methylhexyl)ester

### Beispiel 410

5-[((4-(2-((Phenoxyacetyl)amino)ethyl)phenylsulfonyl)amino)carbonyl]-pyridin-2-carbonsäure-2-(2-methylhexyl)ester

### Beispiel 411

5-[((4-(2-((4-Fluorbenzoyl)amino)ethyl)phenylsulfonyl)amino)carbonyl]-pyridin-2-carbonsäure-2-(2-methylhexyl)ester

### Beispiel 412

5-[((4-(2-((4-Ethoxybenzoyl)amino)ethyl)phenylsulfonyl)amino)carbonyl]-pyridin-2-carbonsäure-2-(2-methylhexyl)ester

### Beispiel 413

5-[((4-(2-((Cyclohexanoyl)amino)ethyl)phenylsulfonyl)amino)carbonyl]-pyridin-2-carbonsäure-2-(2-methylhexyl)ester

### Beispiel 414

5-[((4-(2-((Cyclohexylacetyl)amino)ethyl)phenylsulfonyl)amino)carbonyl]-pyridin-2-carbonsäure-2-(2-methylhexyl)ester

### Beispiel 415

5-[((4-(2-((2-Methylpropionyl)amino)ethyl)phenylsulfonyl)amino)carbonyl]-pyridin-2-carbonsäure-2-(2-methylhexyl)ester

### Beispiel 416

5-[((4-(2-((2-Chlor-5-methoxybenzoyl)amino)ethyl)phenylsulfonyl)amino) carbonyl]-pyridin-2-carbonsäure-2-(2-methylbutyl)ester

### Beispiel 417

5-[((4-(2-(Acetylamino)ethyl)phenylsulfonyl)amino)carbonyl]-pyridin-2-carbonsäure-2-(2-methylbutyl)ester

### Beispiel 418

5-[((4-(2-(n-Butanoylamino)ethyl)phenylsulfonyl)amino)carbonyl]-pyridin-2-carbonsäure-2-(2-methylbutyl)ester

### Beispiel 419

5-[((4-(2-(Benzoylamino)ethyl)phenylsulfonyl)amino)carbonyl]-pyridin-2-carbonsäure-2-(2-methylbutyl)ester

### Beispiel 420

5-[((4-(2-((4-Chlorbenzoyl)amino)ethyl)phenylsulfonyl)amino)carbonyl]-pyridin-2-carbonsäure-2-(2-methylbutyl)ester

### Beispiel 421

5-[((4-(2-((5-Chlor-2-methoxybenzoyl)amino)ethyl)phenylsulfonyl)amino) carbonyl]-pyridin-2-carbonsäure-2-(2-methylbutyl)ester

### Beispiel 422

5-[((4-(2-((3-Phenyl-n-propionyl)amino)ethyl)phenylsulfonyl)amino)carbonyl]-pyridin-2-carbonsäure-2-(2-methylbutyl)ester

### Beispiel 423

5-[((4-(2-((2-Phenylacetyl)amino)ethyl)phenylsulfonyl)amino)carbonyl]pyridin-2-carbonsäure-3-(2,5-dimethylpentyl)ester

### Beispiel 424

5-[((4-(2-((Phenoxyacetyl)amino)ethyl)phenylsulfonyl)amino)carbonyl]-pyridin-2-carbonsäure-3-(2,5-dimethylpentyl)ester

### Beispiel 425

5-[((4-(2-((4-Fluorbenzoyl)amino)ethyl)phenylsulfonyl)amino)carbonyl]-pyridin-2-carbonsäure-3-(2,5-dimethylpentyl)ester

### Beispiel 426

5-[((4-(2-((4-Ethoxybenzoyl)amino)ethyl)phenylsulfonyl)amino)carbonyl]-pyridin-2-carbonsäure-3-(2,5-dimethylpentyl)ester

### Beispiel 427

5-[((4-(2-((Cyclohexanoyl)amino)ethyl)phenylsulfonyl)amino)carbonyl]-pyridin-2-carbonsäure-3-(2,5-dimethylpentyl)ester

### Beispiel 428

5-[((4-(2-((Cyclohexylacetyl)amino)ethyl)phenylsulfonyl)amino)carbonyl]-pyridin-2-carbonsäure-3-(2,5-dimethylpentyl)ester

### Beispiel 429

5-[((4-(2-((2-Methylpropionyl)amino)ethyl)phenylsulfonyl)amino)carbonyl]-pyridin-2-carbonsäure-3-(2,5-dimethylpentyl)ester

### Beispiel 430

5-[((4-(2-((2-Ethylbutanoyl)amino)ethyl)phenylsulfonyl)amino)carbonyl]-pyridin-2-carbonsäure-methylester
Fp. 186°C (aus Ethylacetat)

### Beispiel 431

5-[((4-(2-((2-Ethylbutanoyl)amino)ethyl)phenylsulfonyl)amino)carbonyl]-pyridin-2-carbonsäure-3-pentylester

### Beispiel 432

5-[((4-(2-((4-n-Butoxybenzoyl)amino)ethyl)phenylsulfonyl)amino)carbonyl]-pyridin-2-carbonsäure-2-(2-methylpentyl)ester

### Beispiel 433

5-[((4-(2-((2-Chlor-5-methoxybenzoyl)amino)ethyl)phenylsulfonyl)amino) carbonyl]-pyridin-2-carbonsäure-benzylester

### Beispiel 434

5-[((4-(2-(Acetylamino)ethyl)phenylsulfonyl)amino)carbonyl]-pyridin-2-carbonsäure-benzylester

### Beispiel 435

5-[((4-(2-(n-Butanoylamino)ethyl)phenylsulfonyl)amino)carbonyl]-pyridin-2-carbonsäure-benzylester

### Beispiel 436

5-[((4-(2-(Benzoylamino)ethyl)phenylsulfonyl)amino)carbonyl]-pyridin-2-carbonsäure-benzylester

### Beispiel 437

5-[((4-(2-((4-Chlorbenzoyl)amino)ethyl)phenylsulfonyl)amino)carbonyl]-pyridin-2-carbonsäure-phenylester

### Beispiel 438

5-[((4-(2-((5-Chlor-2-methoxybenzoyl)amino)ethyl)phenylsulfonyl)amino) carbonyl]-pyridin-2-carbonsäure-phenylester

### Beispiel 439

5-[((4-(2-((3-Phenyl-n-propionyl)amino)ethyl)phenylsulfonyl)amino)carbonyl]-pyridin-2-carbonsäure-phenylester

### Beispiel 440

5-[((4-(2-((2-Phenylacetyl)amino)ethyl)phenylsulfonyl)amino)carbonyl]-pyridin-2-carbonsäure-(4-methoxybenzyl)ester

### Beispiel 441

5-[((4-(2-((Phenoxyacetyl)amino)ethyl)phenylsulfonyl)amino)carbonyl]-pyridin-2-carbonsäure-(4-methoxybenzyl)ester

### Beispiel 442

5-[((4-(2-((4-Fluorbenzoyl)amino)ethyl)phenylsulfonyl)amino)carbonyl]-pyridin-2-carbonsäure-(2-methoxyethyl)ester

### Beispiel 443

5-[((4-(2-((4-Ethoxybenzoyl)amino)ethyl)phenylsulfonyl)amino)carbonyl]-pyridin-2-carbonsäure(2-methoxyethyl)ester

### Beispiel 444

5-[((4-(2-((Cyclohexanoyl)amino)ethyl)phenylsulfonyl)amino)carbonyl]-pyridin-2-carbonsäure-(2-ethoxyethyl)ester

### Beispiel 445

5-[((4-(2-((Cyclohexylacetyl)amino)ethyl)phenylsulfonyl)amino)carbony]-pyridin-2-carbonsäure(2-ethoxyethyl)ester

### Beispiel 446

5-[(( -(2-((2-Methylpropionyl)amino)ethyl)phenylsulfonyl)amino)carbonyl]-pyridin-2-carbonsäure-(3-methoxypropyl)ester

### Beispiel 447

5-[((4-(2-(2-Ethylbutanonyl)amino)ethyl)phenylsulfonyl)amino)carbonyl]-pyridin-2-carbonsäure-(3-hydroxypropyl)ester

### Beispiel 448

5-[((4-(2-((2-Chlor-5-methoxybenzoyl)amino)ethyl)phenylsulfonyl)amino) carbonyl]-pyridin-2-carbonsäure((R)-2-butyl)ester

### Beispiel 449

5-[((4-(2-(Acetylamino)ethyl)phenylsulfonyl)amino)carbonyl]-pyridin-2-carbonsäure-((R)-2-butyl)ester

### Beispiel 450

5-[((4-(2-(n-Butanoylamino)ethyl)phenylsulfonyl)amino)carbonyl]-pyridin-2-carbonsäure-((R)-2-butyl)ester

### Beispiel 451

5-[((4-(2-(Benzoylamino)ethyl)phenylsulfonyl)amino)carbonyl]-pyridin-2-carbonsäure-((R)-2-butyl)ester

### Beispiel 452

5-[((4-(2-((4-Chlorbenzoyl)amino)ethyl)phenylsulfonyl)amino)carbonyl]-pyridin-2-carbonsäure-((R)-2-butyl)ester

### Beispiel 453

5-[((4-(2-((5-Chlor-2-methoxybenzoyl)amino)ethyl)phenylsulfonyl)amino) carbonyl]-pyridin-2-carbonsäure-((R)-2-butyl)ester

### Beispiel 454

5-[((4-(2-((3-Phenyl-n-propionyl)amino)ethyl)phenylsulfonyl)amino)carbonyl]-pyridin-2-carbonsäure-((R)-2-butyl)ester

### Beispiel 455

5-[((4-(2-((2-Phenylacetyl)amino)ethyl)phenylsulfonyl)amino)carbonyl]-pyridin-2-carbonsäure-((R)-2-butyl)ester

### Beispiel 456

5-[((4-(2-((Phenoxyacetyl)amino)ethyl)phenylsulfonyl)amino)carbonyl]-pyridin-2-carbonsäure-((R)-2-butyl)ester

### Beispiel 457

5-[((4-(2-((4-Fluorbenzoyl)amino)ethyl)phenylsulfonyl)amino)carbonyl]-pyridin-2-carbonsäure-((R)-2-butyl)ester

### Beispiel 458

5-[((4-(2-((4-Ethoxybenzoyl)amino)ethyl)phenylsulfonyl)amino)carbonyl]-pyridin-2-carbonsäure-((R)-2-butyl)ester

### Beispiel 459

5-[((4-(2-((Cyclohexanoyl)amino)ethyl)phenylsulfonyl)amino)carbonyl]-pyridin-2-carbonsäure-((R)-2-butyl)ester

### Beispiel 460

5-[((4-(2-((Cyclohexylacetyl)amino)ethyl)phenylsulfonyl)amino)carbonyl]-pyridin-2-carbonsäure-((R)-2-butyl)ester

### Beispiel 461

5-[((4-(2-(2-Methylpropionyl)amino)ethyl)phenylsulfonyl)amino)carbonyl]-pyridin-2-carbonsäure-((R)-2-butyl)ester

### Beispiel 462

5-[((4-(2-(2-Ethylbutanonyl)amino)ethyl)phenylsulfonyl)amino)carbonyl]-pyridin-2-carbonsäure-((R)-2-butyl)ester

### Beispiel 463

5-[((4-(2-((2-Chlor-5-methoxybenzoyl)amino)ethyl)phenylsulfonyl)amino) carbonyl]-pyridin-2-carbonsäure-((S)-2-butyl)ester

### Beispiel 464

5-[((4-(2-(Acetylamino)ethyl)phenylsulfonyl)amino)carbonyl]-pyridin-2 carbonsäure-((S)-2-butyl)ester

### Beispiel 465

5-[((4-(2-(n-Butanoylamino)ethyl)phenylsulfonyl)amino)carbonyl]-pyridin-((S)-2-butyl)ester

### Beispiel 466

5-[((4-(2-(Benzoylamino)ethyl)phenylsulfonyl)amino)carbonyl]-pyridin-2-carbonsäure-((S)-2-butyl)ester

### Beispiel 467

5-[((4-(2-((4-Chlorbenzoyl)amino)ethyl)phenylsulfonyl)amino)carbonyl]-pyridin-2-carbonsäure-((S)-2-butyl)ester

### Beispiel 468

5-[((4-(2-((5-Chlor-2-methoxybenzoyl)amino)ethyl)phenylsulfonyl)amino) carbonyl]-pyridin-2-carbonsäure-((S)-2-butyl)ester

### Beispiel 469

5-[((4-(2-((3-Phenyl-n-propionyl)amino)ethyl)phenylsulfonyl)amino)carbonyl]-pyridin-2-carbonsäure-((S)-2-butyl)ester

### Beispiel 470

5-[((4-(2-((2-Phenylacetyl)amino)ethyl)phenylsulfonyl)amino)carbonyl]-pyridin-2-carbonsäure-((S)-2-butyl)ester

### Beispiel 471

5-[((4-(2-((Phenoxyacetyl)amino)ethyl)phenylsulfonyl)amino)carbonyl]-pyridin-2-carbonsäure-((S)-2-butyl)ester

### Beispiel 472

5-[((4-(2-((4-Fluorbenzoyl)amino)ethyl)phenylsulfonyl)amino)carbonyl]-pyridin-2-carbonsäure-((S)-2-butyl)ester

### Beispiel 473

5-[((4-(2-((4-Ethoxybenzoyl)amino)ethyl)phenylsulfonyl)amino)carbonyl]-pyridin-2-carbonsäure-((S)-2-butyl)ester

### Beispiel 474

5-[((4-(2-((Cyclohexanoyl)amino)ethyl)phenylsulfonyl)amino)carbonyl]-pyridin-2-carbonsäure-((S)-2-butyl)ester

### Beispiel 475

5-[((4-(2-((Cyclohexylacetyl)amino)ethyl)phenylsulfonyl)amino)carbonyl]-pyridin-2-carbonsäure-((S)-2-butyl)ester

### Beispiel 476

5-[((4-(2-(2-Methylpropionyl)amino)ethyl)phenylsulfonyl)amino)carbonyl]-pyridin-2-carbonsäure-((S)-2-butyl)ester

### Beispiel 477

5-[((4-(2-(2-Ethylbutanonyl)amino)ethyl)phenylsulfonyl)amino)carbonyl]-pyridin-2-carbonsäure-((S)-2-butyl)ester

### Beispiel 478

5-[((4-(2-((2-Chlor-5-methoxybenzoyl)amino)ethyl)phenylsulfonyl)amino) carbonyl]-pyridin-2-carbonsäure-4-heptylester

### Beispiel 479

5-[((4-(2-(Acetylamino)ethyl)phenylsulfonyl)amino)carbonyl]-pyridin-2-carbonsäure-4-heptylester

### Beispiel 480

5-[((4-(2-(n-Butanoylamino)ethyl)phenylsulfonyl)amino)carbonyl]-pyridin-2-carbonsäure-4-heptylester

### Beispiel 481

5-[((4-(2-(Benzoylamino)ethyl)phenylsulfonyl)amino)carbonyl]-pyridin-2-carbonsäure-4-heptylester

### Beispiel 482

5-[((4-(2-((4-Chlorbenzoyl)amino)ethyl)phenylsulfonyl)amino)carbonyl]-pyridin-2-carbonsäure-4-heptylester

### Beispiel 483

5-[((4-(2-((5-Chlor-2-methoxybenzoyl)amino)ethyl)phenylsulfonyl)amino) carbonyl]-pyridin-2-carbonsäure-cyclopentylester

### Beispiel 484

5-[((4-(2-((3-Phenyl-n-propionyl)amino)ethyl)phenylsulfonyl)amino)carbonyl]-pyridin-2-carbonsäure-cyclopentylester

### Beispiel 485

5-[((4-(2-((2-Phenylacetyl)amino)ethyl)phenylsulfonyl)amino)carbonyl]-pyridin-2-carbonsäure-cyclohexylester

### Beispiel 486

5-[((4-(2-((Phenoxyacetyl)amino)ethyl)phenylsulfonyl)amino)carbonyl]-pyridin-2-carbonsäure-cyclohexylester

### Beispiel 487

5-[((4-(2-((4-Fluorbenzoyl)amino)ethyl)phenylsulfonyl)amino)carbonyl]-pyridin-2-carbonsäure-cyclohexylester

### Beispiel 488

5-[((4-(2-((4-Ethoxybenzoyl)amino)ethyl)phenylsulfonyl)amino)carbonyl]-pyridin-2-carbonsäure-cyclobutylester

### Beispiel 489

5-[((4-(2-((Cyclohexanoyl)amino)ethyl)phenylsulfonyl)amino)carbonyl]-pyridin-2-carbonsäure-2-(2-methyl-3-butinyl)ester

### Beispiel 490

5-[((4-(2-((Cyclohexylacetyl)amino)ethyl)phenylsulfonyl)amino)carbonyl]-pyridin-2-carbonsäure-2-(2-methyl-3-butinyl)ester

### Beispiel 491

5-[(( -(2-(2-Methylpropionyl)amino)ethyl)phenylsulfonyl)amino)carbonyl]-pyridin-2-carbonsäure-2-(2-methyl-3-butinyl)ester

### Beispiel 492

5-[((4-(2-(2-Ethylbutanonyl)amino)ethyl)phenylsulfonyl)amino)carbonyl]-pyridin-2-carbonsäure-2-(2-methyl-3-butinyl)ester

### Beispiel 493

5-[((3-(2-((2-Chlor-5-methoxybenzoyl)amino)ethyl)phenylsulfonyl)amino) carbonyl]-pyridin-2-carbonsäure-1-(2-butinyl)ester

### Beispiel 494

5-[((3-(2-(Acetylamino)ethyl)phenylsulfonyl)amino)carbonyl]-pyridin-2-carbonsäure-1-(3-butinyl)ester

### Beispiel 495

5-[((3-(2-(n-Butanoylamino)ethyl)phenylsulfonyl)amino)carbonyl]-pyridin-2-carbonsäure-1-(3-butenyl)ester

### Beispiel 496

5-[((3-(2-(Benzoylamino)ethyl)phenylsulfonyl)amino)carbonyl]-pyridin-2-carbonsäure-1-(3-butenyl)ester

### Beispiel 497

5-[((3-(2-((4-Chlorbenzoyl)amino)ethyl)phenylsulfonyl)amino)carbonyl]-pyridin-2-carbonsäure-((1R,2S,5R)-(-)menthyl)ester

### Beispiel 498

5-[((3-(2-((5-Chlor-2-methoxybenzoyl)amino)ethyl)phenylsulfonyl)amino) carbonyl]-pyridin-2-carbonsäure-2-(2-methyl-3-butinyl)ester

### Beispiel 499

5-[((3-(2-((3-Phenyl-n-propionyl)amino)ethyl)phenylsulfonyl)amino)carbonyl]-pyridin-2-carbonsäure-2-(2-methyl-3-butinyl)ester

### Beispiel 500

5-[((3-(2-((3-Phenylacetyl)amino)ethyl)phenylsulfonyl)amino)carbonyl]-pyridin-2-carbonsäure-(2-methoxycarbonyl-2,2-dimethylethyl)ester

### Beispiel 501

5-[((4-(3-((Phenoxyacetyl)amino)ethyl)phenylsulfonyl)amino)carbonyl]-pyridin-2-carbonsäure-(2-methoxycarbonyl-2,2-dimethylethyl)ester

### Beispiel 502

5-[((3-(2-((4-Fluorbenzoyl)amino)ethyl)phenylsulfonyl)amino)carbonyl]-pyridin-2-carbonsäure-((1R)-endo-(+)-fenchyl)ester

### Beispiel 503

5-[((3-(2-((4-Ethoxybenzoyl)amino)ethyl)phenylsulfonyl)amino)carbonyl]-pyridin-2-carbonsäure-((1R)-endo-(+)-fenchyl)ester

### Beispiel 504

5-[((3-(2-((Cyclohexanoyl)amino)ethyl)phenylsulfonyl)amino)carbonyl]-pyridin-2-carbonsäure-(1-isopropoxycarbonylethyl)ester

### Beispiel 505

5-[((3-(2-((Cyclohexylacetyl)amino)ethyl)phenylsulfonyl)amino)carbonyl]-pyridin-2-carbonsäure-(1-ethoxycarbonylethyl)ester

### Beispiel 506

5-[((3-(2-(2-Methylpropionyl)amino)ethyl)phenylsulfonyl)amino)carbonyl]-pyridin-2-carbonsäure-((1R,2S,5R)-(-)menthyl)ester

### Beispiel 507

5-[((3-(2-(2-Ethylbutanonyl)amino)ethyl)phenylsulfonyl)amino)carbonyl]-pyridin-2-carbonsäure-((1S,2R,5S)-(+)menthyl)ester

### Beispiel 508

5-[((4-((4-Phenyl-n-butanoyl)amino)phenylsulfonyl)amino)carbonyl]-pyridin-2-carbonsäure-methylester
a) 4-((Phenyl-n-butanoyl)amino)benzolsulfonsäureamid
   16,5 g (0,1 mol) 4-Phenylbuttersäure wurden in 300 ml wasserfreiem Tetrahydrofuran bei 0°C mit 11,1 g (0,11 mol, 15,2ml) Triethylamin versetzt. Nach 30 min. tropfte man bei 0°C 12g (0,11 mol, 10,5 ml) Chlorameisensäureethylester zu. Zu dieser dicken Suspension wurde bei - 10°C eine Lösung von 18,1 g (0,105 mol) 4-Aminobenzolsulfonamid in 150 ml wasserfreiem Tetrahydrofuran hinzugetropft. Es wurde 1 h bei 0°C, 1 h bei 25°C gerührt, i. Vak eingeengt, der Rückstand mit wäßriger Salzsäure behandelt. Das kristalline Rohprodukt wurde mit Wasser gewaschen und aus 250 ml Methanol umkristallisiert; Ausbeute 18 g; Fp. 166 bis 168°C.
b) Analog Beispiel 32b) wurden 1,8 g (10 mmol) Pyridin-2,5-dicarbonsäure-2-methylester in 300 ml Acetonitril mit 3,2 g (10 mmol) des vorstehenden Benzolsulfonamids, 2,1 g (10 mmol) N,N'-Dicyclohexylcarbodiimid und 1,2 g (10 mmol) 4-N,N-Dimethylaminopyridin umgesetzt.
   Man filtrierte vom Ungelösten ab, engte ein, versetzte mit wäßriger Salzsäure (pH1) und saugte das feinkristalline Produkt ab. Dieses wurde in N,N-Dimethylformamid gelöst und bis zur beginnenden Trübung mit Wasser versetzt. Das kristalline Rohprodukt wurde mit Wasser gewaschen und getrocknet; 3,3 g; Fp. 258 bis 264°C.
   Nach Chromatographie mit Ethylacetat/Methanol (3:1) an Kieselgel wurden entsprechende Fraktionen eingedampft und aus Methanol umkristallisiert. Man isolierte 1,4 g farblos kristallines Produkt; Fp. 258°C (u. Zers.)

### Beispiel 509

5-[((4-(3-Phenyl-n-propionylamino)phenylsulfonyl)amino)carbonyl]-pyridin-2-carbonsäure-methylester

### Beispiel 510

5-[((4-(2-Phenylacetylamino)phenylsulfonyl)amino)carbonyl]-pyridin-2-carbonsäure-methylester

### Beispiel 511

5[((4-(Benzoylamino)phenylsulfonyl)amino)carbonyl]-pyridin-2-carbonsäuremethylester

### Beispiel 512

5[((4-(Acetylamino)phenylsulfonyl)amino)carbonyl]-pyridin-2-carbonsäuremethylester

### Beispiel 513

5-[((4-(n-Propionylamino)phenylsulfonyl)amino)carbonyl]-pyridin-2-carbonsäuremethylester

### Beispiel 514

5-[((4-(n-Hexanoylamino)phenylsulfonyl)amino)carbonyl]-pyridin-2-carbonsäuremethylester

### Beispiel 515

5-[((4-((2-Phenoxyacetyl)amino)phenylsulfonyl)amino)carbonyl]-pyridin-2-carbonsäure-methylester
Fp. 222 bis 224°C

### Beispiel 516

5-[((4-(n-Butanoylamino)phenylsulfonyl)amino)carbonyl]-pyridin-2-carbonsäuremethylester

### Beispiel 517

5-[((4-(Cyclohexanoylamino)phenylsulfonyl)amino)carbonyl]-pyridin-2-carbonsäure-methylester

### Beispiel 518

5-[((4-(Cyclohexylacetyl)amino)phenylsulfonyl)amino)carbonyl]-pyridin-2-carbonsäure-methylester

### Beispiel 519

5-[((4-(4-Phenyl-n-butanoylamino)phenylsulfonyl)amino)carbonyl]-pyridin-2-carbonsäure-1-propylester

### Beispiel 520

5-[((4-(3-Phenyl-n-propionylamino)phenylsulfonyl)amino)carbonyl]-pyridin-2-carbonsäure-1-propylester

### Beispiel 521

5-[((4-(2-Phenylacetylamino)phenylsulfonyl)amino)carbonyl]-pyridin-2-carbonsäure-2-propylester

### Beispiel 522

5[((4-(Benzoylamino)phenylsulfonyl)amino)carbonyl]-pyridin-2-carbonsäure-2-propylester

### Beispiel 523

5[((4-(2-Methylpentanoylamino)phenylsulfonyl)amino)carbonyl]-pyridin-2-carbonsäure-3-pentylester

### Beispiel 524

5-[((4-(n-Propionylamino)phenylsulfonyl)amino)carbonyl]-pyridin-2-carbonsäure-3-pentylester

### Beispiel 525

5-[((4-(n-Hexanoylamino)phenylsulfonyl)amino)carbonyl]-pyridin-2-carbonsäure-3-pentylester

### Beispiel 526

5-[((4-((2-Phenoxyacetyl)amino)phenylsulfonyl)amino)carbonyl]-pyridin-2-carbonsäure-2-(2-methylhexyl)ester

### Beispiel 527

5-[((4-(n-Butanoylamino)phenylsulfonyl)amino)carbonyl]-pyridin-2-carbonsäure-2-(2-methylhexyl)ester

### Beispiel 528

5-[((4-(Cyclohexanoylamino)phenylsulfonyl)amino)carbonyl]-pyridin-2-carbonsäure-4-heptylester

### Beispiel 529

5-[((4-(Cyclohexylacetyl)amino)phenylsulfonyl]amino)carbonyl]-pyridin-2-carbonsäure-2-propylester

### Beispiel 530

5-[(((4-Phenyl-n-butyl)sulfonyl)amino)carbonyl]-pyridin-2-carbonsäuremethylester

### Beispiel 531

5-[(((2-Phenoxyethyl)sulfonyl)amino)carbonyl]-pyridin-2-carbonsäuremethylester

### Beispiel 532

5-[((2-(4-Fluorphenoxy)ethylsulfonyl)amino)carbonyl]-pyridin-2-carbonsäuremethylester

### Beispiel 533

5-[((Phenylmethylsulfonyl)amino)carbonyl]-pyridin-2-carbonsäuremethylester

### Beispiel 534

5-[(((2-Phenylethyl)sulfonyl)amino)carbonyl]-pyridin-2-carbonsäuremethylester

### Beispiel 535

5-[(((2-(4-Fluorphenyl)ethyl)sulfonyl)amino)carbonyl]-pyridin-2-carbonsäuremethylester

### Beispiel 536

5-[(((2-(4-Methoxyphenyl)ethyl)sulfonyl)amino)carbonyl]-pyridin-2-carbonsäuremethylester

### Beispiel 537

5-[(((3-Phenyl-n-propyl)sulfonyl)amino)carbonyl]-pyridin-2-carbonsäuremethylester

### Beispiel 538

5-[(((4-Phenyl-n-butyl)sulfonyl)amino)carbonyl]-pyridin-2-carbonsäureethylester

### Beispiel 539

5-[(((2-Phenoxyethyl)sulfonyl)amino)carbonyl]-pyridin-2-carbonsäureethylester

### Beispiel 540

5-[(((2-(4-Fluorphenoxy)ethyl)sulfonyl)amino)carbonyl]-pyridin-2-carbonsäureethylester

### Beispiel 541

5-[((Phenylmethylsulfonyl)amino)carbonyl]-pyridin-2-carbonsäureethylester

### Beispiel 542

5-[(((2-Phenylethyl)sulfonyl)amino)carbonyl]-pyridin-2-carbonsäureethylester

### Beispiel 543

5-[(((2-(4-Fluorphenyl)ethyl)sulfonyl)amino)carbonyl]-pyridin-2-carbonsäureethylester

### Beispiel 544

5-[(((2-(4-Methoxyphenyl)ethyl)sulfonyl)amino)carbonyl]-pyridin-2-carbonsäureethylester

### Beispiel 545

5-[(((3-Phenyl-n-propyl)sulfonyl)amino)carbonyl]-pyridin-2-carbonsäureethylester

### Beispiel 546

5-[(((4-Phenyl-n-butyl)sulfonyl)amino)carbonyl]-pyridin-2-carbonsäure-2-propylester

### Beispiel 547

5-[(((2-Phenoxyethyl)sulfonyl)amino)carbonyl]-pyridin-2-carbonsäure-2-propylester

### Beispiel 548

5-[(((2-(4-Fluorphenoxy)ethyl)sulfonyl)amino)carbonyl]-pyridin-2-carbonsäure-2-propylester

### Beispiel 549

5-[((Phenylmethylsulfonyl)amino)carbonyl]-pyridin-2-carbonsäure-2-propylester

### Beispiel 550

5-[(((2-Phenylethyl)sulfonyl)amino)carbonyl]-pyridin-2-carbonsäure-2-propylester

### Beispiel 551

5-[(((2-(4-Fluorphenyl)ethyl)sulfonyl)amino)carbonyl]-pyridin-2-carbonsäure-2-propylester

### Beispiel 552

5-[(((2-(4-Methoxyphenyl)ethyl)sulfonyl)amino)carbonyl]-pyridin-2-carbonsäure-2-propylester

### Beispiel 553

5-[(((3-Phenyl-n-propyl)sulfonyl)amino)carbonyl]-pyridin-2-carbonsäure-2-propylester

### Beispiel 554

5-[((4-(2-(Acetylamino)methyl)phenylsulfonyl)amino)carbonyl]-pyridin-2-carbonsäuremethylester

### Beispiel 555

5-[((4-(2-(n-Butanoylamino)methyl)phenylsulfonyl)amino)carbonyl]-pyridin-2-carbonsäuremethylester

### Beispiel 556

5-[((4-(2-(Benzoylamino)methyl)phenylsulfonyl)amino)carbonyl]-pyridin-2-carbonsäuremethylester

### Beispiel 557

5-[((4-(2-((4-Chlorbenzoyl)amino)methyl)phenylsulfonyl)amino)carbonyl]-pyridin-2-carbonsäuremethylester

### Beispiel 558

5-[((4-(2-((5-Chlor-2-methoxybenzoyl)amino)methyl)phenylsulfonyl)amino) carbonyl]-pyridin-2-carbonsäuremethylester

### Beispiel 559

5-[((4-(2-((3-Phenyl-n-propionyl)amino)methyl)phenylsulfonyl)amino)carbonyl]-pyridin-2-carbonsäuremethylester

### Beispiel 560

5-[((4-(2-((2-Phenylacetyl)amino)methyl)phenylsulfonyl)amino)carbonyl]-pyridin-2-carbonsäuremethylester

### Beispiel 561

5-[((4-(2-((Phenoxyacetyl)amino)methyl)phenylsulfonyl)amino)carbonyl]-pyridin-2-carbonsäuremethylester

### Beispiel 562

5-[((4-(2-((4-Fluorbenzoyl)amino)methyl)phenylsulfonyl)amino)carbonyl]-pyridin-2-carbonsäuremethylester

### Beispiel 563

5-[((4-(2-((4-Ethoxybenzoyl)amino)methyl)phenylsulfonyl)amino)carbonyl]-pyridin-2-carbonsäuremethylester

### Beispiel 564

5-[((4-(2-((Cyclohexanoyl)amino)methyl)phenylsulfonyl)amino)carbonyl]-pyridin-2-carbonsäuremethylester

### Beispiel 565

5-[((4-(2-((Cyclohexylacetyl)amino)methyl)phenylsulfonyl)amino)carbonyl]-pyridin-2-carbonsäuremethylester

### Beispiel 566

5-[((4-(2-((2-Methylpropionyl)amino)methyl)phenylsulfonyl)amino)carbonyl]-pyridin-2-carbonsäuremethylester

### Beispiel 567

5-[((3-(2-((2-Chlor-5-methoxybenzoyl)amino)methyl)phenylsulfonyl)amino) carbonyl]- pyridin-2-carbonsäuremethylester

### Beispiel 568

5-[((4-(2-(n-Pentanoylamino)methyl)phenylsulfonyl)amino)carbonyl]-pyridin-2-carbonsäuremethylester

### Beispiel 569

5-[((4-(2-((3-Methylbutanoyl)amino)methyl)phenylsulfonyl)amino)carbonyl]-pyridin-2-carbonsäuremethylester

### Beispiel 570

5-[((4-(2-((4-Methylpentanoyl)amino)methyl)phenylsulfonyl)amino)carbonyl]-pyridin-2-carbonsäuremethylester

### Bespiel 571

5-[((4-(2-((3-Cyclohexyl-n-propionyl)amino)methyl)phenylsulfonyl)amino) carbonyl]-pyridin-2-carbonsäuremethylester

### Beispiel 572

5-[((4-(2-(n-Pentanoylamino)methyl)phenylsulfonyl)amino)carbonyl]-pyridin-2-carbonsäure-2-propylester

### Beispiel 573

5-[((4-(2-((-Methylbutanoyl)amino)methyl)phenylsulfonyl)amino)carbononyl]-pyridin-2-carbonsäure-2-propylester

### Beispiel 574

5-[((4-(2-((4-Methylpentanoyl)amino)methyl)phenylsulfonyl)amino)carbonyl]-pyridin-2-carbonsäure-2-propylester

### Beispiel 575

5-[((4-(2-(Acetylamino)methyl)phenylsulfonyl)amino)carbonyl]-pyridin-2-carbonsäure-3-pentylester

### Beispiel 576

5-[((4-(2-((5-Chor-2-methoxybenzoyl)amino)methyl)phenylsulfonyl)amino) carbonyl]-pyridin-2-carbonsäure-2-propylester

### Beispiel 577

5-[((4-(2-((3-Phenyl-n-propionyl)amino)methyl)phenylsulfonyl)amino)carbonyl]-pyridin-2-carbonsäure-2-propylester

### Beispiel 578

5-[((4-(2-((2-(3,4-Dimethoxyphenyl)acetyl)amino)ethyl)phenylsulfonyl)amino)-carbonyl]-pyridin-2-carbonsäure-2-methylester (Methode B)
Fp. 164 bis 169°C (aus Methanol/Diisopropylether)

### Beispiel 579

5-[((4-(2-((3,4-Dimethoxybenzoyl)amino)ethyl)phenylsulfonyl)amino)carbonyl]-pyridin-2-carbonsäure-2-propylester

### Beispiel 580

5-[((4-(2-((2,5-Dimethoxybenzoyl)amino)ethyl)phenylsulfonyl)amino)carbonyl]-pyridin-2-carbonsäure-2-propylester

### Beispiel 581

5-[((4-(2-((3,4-Dimethoxybenzoyl)amino)ethyl)phenylsulfonyl)amino)carbonyl]-pyridin-2-carbonsäure-2-propylester

### Beispiel 582

5-[((4-(2-((3,4-Dimethoxybenzoyl)amino)ethyl)phenylsulfonyl)amino)carbonyl]-pyridin-2-carbonsäure-2-propylester

### Beispiel 583

5-[((4-(2-((2,5-Dimethoxybenzoyl)amino)ethyl)phenylsulfonyl)amino)carbonyl]-pyridin-2-carbonsäure-2-propylester

### Beispiel 584

5-[((4-(2-((3,4-Dimethoxybenzoyl)amino)ethyl)phenylsulfonyl)amino)carbonyl]-pyridin-2-carbonsäure-2-propylester

### Beispiel 585

5-[((4-(2-((3,4-Dimethoxybenzoyl)amino)ethyl)phenylsulfonyl)amino)carbonyl]-pyridin-2-carbonsäure-2-propylester

### Beispiel 586

5-[((4-(2-((2,5-Dimethoxybenzoyl)amino)ethyl)phenylsulfonyl)amino)carbonyl]-pyridin-2-carbonsäure-2-propylester

### Beispiel 587

5-[((4-(2-((3,4-Dimethoxybenzoyl)amino)ethyl)phenylsulfonyl)amino)carbonyl]-pyridin-2-carbonsäure-2-propylester

### Beispiel 588

5-[((4-(2-((3,4-Dimethoxybenzoyl)amino)ethyl)phenylsulfonyl)amino)carbonyl]-pyridin-2-carbonsäure-2-propylester

### Beispiel 589

5-[((4-(2-((2,5-Dimethoxybenzoyl)amino)ethyl)phenylsulfonyl)amino)carbonyl]-pyridin-2-carbonsäure-2-propylester

### Beispiel 590

5-[((4-(2-((3,4-Dimethoxybenzoyl)amino)ethyl)phenylsulfonyl)amino)carbonyl]-pyridin-2-carbonsäure-2-propylester

### Beispiel 591

5-[((4-(2-((3,4-Dimethoxybenzoyl)amino)ethyl)phenylsulfonyl)amino)carbonyl]-pyridin-2-carbonsäure-2-propylester

### Beispiel 592

5-[((4-(2-((2,5-Dimethoxybenzoyl)amino)ethyl)phenylsulfonyl)amino)carbonyl]-pyridin-2-carbonsäure-2-propylester

### Beispiel 593

5-[((4-((2-(4-Methoxyphenyl)ethyl)aminocarbonyl)phenylsulfonyl)amino) carbonyl]-pyridin-2-carbonsäuremethylester-α,α,α-tris(hydroxymethyl) methylamin-Salz
0,5 g (1,0 mmol) der Titelverbindung aus Beispiel 36) wurden in 50 ml Tetrahydrofuran/Dichlormethan-Gemisch suspendiert und bei 20°C mit 0,12 g (1 mmol) α,α,α-Tris(hydroxymethyl)methylamin versetzt.

Unter leichtem Erwärmen wurde 2 h gerührt, wobei vollständige Lösung eintrat. Nach dem Abkühlen wurde i. Vak. eingeengt und der amorphe Rückstand an der Ölpumpe getrocknet. Man erhielt 0,5 g der Titelverbindung; Fp. ca. 50°C. Das Produkt löste sich in Wasser, wobei der pH-Wert der wäßrigen Lösung 7 betrug.

In den Beispielen 594 bis 598 steht entsprechend Formel I X für eine Bindung.

### Beispiel 594

a) 5-[(4-Fluorphenylsulfonyl)amino]-pyridin-2-carbonsäuremethylester 3,8 g (25 mmol) 5-Amino-pyridin-2-carbonsäuremethylester wurden in 75 ml wasserfreiem Pyridin gelöst und portionsweise mit 5,8 g (30 mmol) 4-Fluorbenzolsulfonsäurechlorid versetzt, wobei sich die Temperatur der Reaktionslösung auf 35°C erhöhte. Nach 1 h wurde im Vakuum eingeengt, der Rückstand mit Wasser verrieben, abgesaugt, mit Wasser gewaschen und getrocknet. Man erhielt 7,3 g Produkt, Fp. 183 bis 185°C.

### Beispiel 595

5-[(4-Fluorphenylsulfonyl)amino]-pyridin-2-carbonsäure-2-propylester

### Beispiel 596

5-[(3,5-Bis[trifluorethyloxy]phenylsulfonyl)amino]-pyridin-2-carbonsäuremethylester
Fp. 158 bis 160°C (aus Wasser)

### Beispiel 597

5-[(1-Naphthylsulfonyl)amino]-pyridin-2-carbonsäuremethylester
Fp. 176 bis 179°C (aus Wasser)

### Beispiel 598

5-[(4-(3-Chlor-2-cyano-phenoxy)phenylsulfonyl)amino]-pyridin-2-carbonsäuremethylester
Fp. 153 bis 155°C (aus Ethanol)
In den Beispielen 599 bis 660 steht entsprechend Formel I X für -CO-:

### Beispiel 599

5-[((4-(2-(n-Hexanoylamino)ethyl)phenylsulfonyl)amino)carbonyl]-pyridin-2-carbonsäuremethylester

### Beispiel 600

5-[((4-(2-(n-Heptanoylamino)ethyl)phenylsulfonyl)amino)carbonyl]-pyridin-2-carbonsäuremethylester

### Beispiel 601

5-[((4-(2-(n-Octanoylamino)ethyl)phenylsulfonyl)amino)carbonyl]-pyridin-2-carbonsäuremethylester

### Beispiel 602

5-[((4-(2-(Acetylamino)ethyl)phenylsulfonyl)amino)carbonyl]-pyridin-2-carbonsäure-1-(2,2-dimethyl-3-hydroxypropyl)ester

### Beispiel 603

5-[((4-(2-(n-Butanoylamino)ethyl)phenylsulfonyl)amino)carbonyl]-pyridin-2-carbonsäure-1-(2,2-dimethyl-3-hydroxypropyl)ester

### Beispiel 604

5-[((4-(2-(n-Pentanoylamino)ethyl)phenylsulfonyl)amino)carbonyl]-pyridin-2-carbonsäure-1-(2,2-dimethyl-3-hydroxypropyl)ester

### Beispiel 605

5-[((4-(2-(n-Hexanoylamino)ethyl)phenylsulfonyl)amino)carbonyl]-pyridin-2-carbonsäure-1-(2,2-dimethyl-3-hydroxypropyl)ester

### Beispiel 606

5-[((4-(2-(4-Methylpentanoylamino)ethyl)phenylsulfonyl)amino)carbonyl]-pyridin-2-carbonsäure-1-(2,2-dimethyl-3-hydroxypropyl)ester

### Beispiel 607

5-[((4-(2-(n-Heptanoylamino)ethyl)phenylsulfonyl)amino)carbonyl]-pyridin-2-carbonsäure-1-(2,2-dimethyl-3-hydroxypropyl)ester

### Beispiel 608

5-[((4-(2-(n-Octanoylamino)ethyl)phenylsulfonyl)amino)carbonyl]-pyridin-2-carbonsäure-1-(2,2-dimethyl-3-hydroxypropyl)ester

### Beispiel 609

5-[((4-(2-((2-Methylpropionyl)amino)ethyl)phenylsulfonyl)amino)carbonyl]-pyridin-2-carbonsäure-1-(2,2-dimethyl-3-hydroxypropyl)ester

### Beispiel 610

5-[((4-(2-(Benzoylamino)ethyl)phenylsulfonyl)amino)carbonyl]-pyridin-2-carbonsäure-1-(2,2-dimethyl-3-hydroxypropyl)ester

### Beispiel 611

5-[((4-(2-((4-n-Butoxybenzoyl)amino)ethyl)phenylsulfonyl)amino)carbonyl]-pyridin-2-carbonsäure-1-(2,2-dimethyl-3-hydroxypropyl)ester

### Beispiel 612

5-[((4-(2-((5-Chlor-2-methoxybenzoyl)amino)ethyl)phenylsulfonyl)amino) carbonyl]-pyridin-2-carbonsäure-1-(2,2-dimethyl-3-hydroxypropyl)ester

### Beispiel 613

5-[((4-(2-((3-Phenyl-n-propionyl)amino)ethyl)phenylsulfonyl)amino)carbonyl]-pyridin-2-carbonsäure-1-(2,2-dimethyl-3-hydroxypropyl)ester

### Beispiel 614

5-[((4-(2-((2-Phenylacetyl)amino)ethyl)phenylsulfonyl)amino)carbonyl]-pyridin-2-carbonsäure-1-(2,2-dimethyl-3-hydroxypropyl)ester

### Beispiel 615

5-[((4-(2-((Phenoxyacetyl)amino)ethyl)phenylsulfonyl)amino)carbonyl]-pyridin-2-carbonsäure-1-(2,2-dimethyl-3-hydroxypropyl)ester

### Beispiel 616

5-[((4-(2-((4-Fluorbenzoyl)amino)ethyl)phenylsulfonyl)amino)carbonyl]-pyridin-2-carbonsäure-1-(2,2-dimethyl-3-hydroxypropyl)ester

### Beispiel 617

5-[((4-(2-((4-Ethoxybenzoyl)amino)ethyl)phenylsulfonyl)amino)carbonyl]-pyridin-2-carbonsäure-1-(2,2-dimethyl-3-hydroxypropyl)ester

### Beispiel 618

5-[((4-(2-((Cyclohexanoyl)amino)ethyl)phenylsulfonyl)amino)carbonyl]-pyridin-2-carbonsäure-1-(2,2-dimethyl-3-hydroxypropyl)ester

### Beispiel 619

5-[((4-(2-((Cyclohexylacetyl)amino)ethyl)phenylsulfonyl)amino)carbonyl]-pyridin-2-carbonsäure-1-(2,2-dimethyl-3-hydroxypropyl)ester

### Beispiel 620

5-[((3-(2-((2-Chlor-5-methoxybenzoyl)amino)ethyl)phenylsulfonyl)amino) carbonyl]-pyridin-2-carbonsäure-1-(2,2-dimethyl-3-hydroxypropyl)ester

### Beispiel 621

5-[((4-(2-((2-(3,4-Dimethoxyphenyl)acetyl)amino)ethyl)phenylsulfonyl)amino)carbonyl]-pyridin-2-carbonsäure-1-(2,2-dimethyl-3-hydroxypropyl)ester

### Beispiel 622

5-[((4-(2-(Acetylamino)ethyl)phenylsulfonyl)amino)carbonyl]-pyridin-2-carbonsäure-1-(2-ethylbutyl)ester

### Beispiel 623

5-[((4-(2-(n-Butanoyamino)ethyl)phenylsulfonyl)amino)carbonyl]-pyridin-2-carbonsäure-1-(2-ethylbutyl)ester

### Beispiel 624

5-[((4-(2-(n-Pentanoylamino)ethyl)phenylsulfonyl)amino)carbonyl]-pyridin-2-carbonsäure-1-(2-ethylbutyl)ester

### Beispiel 625

5-[((4-(2-(n-Hexanoylamino)ethyl)phenylsulfonyl)amino)carbonyl]-pyridin-2-carbonsäure-1-(2-ethylbutyl)ester

### Beispiel 626

5-[((4-(2-(4-Methylpentanoylamino)ethyl)phenylsulfonyl)amino)carbonyl]-pyridin-2-carbonsäure-1-(2-ethylbutyl)ester

### Beispiel 627

5-[((4-(2-(n-Heptanoylamino)ethyl)phenylsulfonyl)amino)carbonyl]-pyridin-2-carbonsäure-1-(2-ethylbutyl)ester

### Beispiel 628

5-[((4-(2-(n-Octanoylamino)ethyl)phenylsulfonyl)amino)carbonyl]-pyridin-2-carbonsäure-1-(2-ethylbutyl)ester

### Beispiel 629

5-[((4-(2-((2-Methylpropionyl)amino)ethyl)phenylsulfonyl)amino)carbonyl]-pyridin-2-carbonsäure-1-(2-ethylbutyl)ester

### Beispiel 630

5-[((4-(2-(Benzoylamino)ethyl)phenylsulfonyl)amino)carbonyl]-pyridin-2-carbonsäure-1-(2-ethylbutyl)ester

### Beispiel 631

5-[((4-(2-((n-Butoxybenzoyl)amino)ethyl)phenylsulfonyl)amino)carbonyl]-pyridin-2-carbonsäure-1-(2-ethylbutyl)ester

### Beispiel 632

5-[((4-(2-((5-Chlor-2-methoxybenzoyl)amino)ethyl)phenylsulfonyl)amino) carbonyl]-pyridin-2-carbonsäure-1-(2-ethylbutyl)ester

### Beispiel 633

5-[((4-(2-((3-Phenyl-n-propionyl)amino)ethyl)phenylsulfonyl)amino)carbonyl]-pyridin-2-carbonsäure-1-(2-ethylbutyl)ester

### Beispiel 634

5-[((4-(2-((2-Phenylacetyl)amino)ethyl)phenylsulfonyl)amino)carbonyl]-pyridin-2-carbonsäure-1-(2-ethylbutyl)ester

### Beispiel 635

5-[((4-(2-((Phenoxyacetyl)amino)ethyl)phenylsulfonyl)amino)carbonyl]-pyridin-2-carbonsäure-1-(2-ethylbutyl)ester

### Beispiel 636

5-[((4-(2-((4-Fluorbenzoyl)amino)ethyl)phenylsulfonyl)amino)carbonyl]-pyridin-2-carbonsäure-1-(2-ethylbutyl)ester

### Beispiel 637

5-[((4-(2-((4-Ethoxybenzoyl)amino)ethyl)phenylsulfonyl)amino)carbonyl]-pyridin-2-carbonsäure-1-(2-ethylbutyl)ester

### Beispiel 638

5-[((4-(2-((Cyclohexanoyl)amino)ethyl)phenylsulfonyl)amino)carbonyl]-pyridin-2-carbonsäure-1-(2-ethylbutyl)ester

### Beispiel 639

5-[((4-(2-((Cyclohexylacetyl)amino)ethyl)phenylsulfonyl)amino)carbonyl]-pyridin-2-carbonsäure-1-(2-ethylbutyl)ester

### Beispiel 640

5-[((3-(2-((2-Chlor-5-methoxybenzoyl)amino)ethyl)phenylsulfonyl)amino) carbonyl]-pyridin-2-carbonsäure-1-(2-ethylbutyl)ester

### Beispiel 641

5-[((4-(2-((2-(3,4-Dimethoxyphenyl)acetyl)amino)ethyl)phenylsulfonyl)amino)-carbonyl]-pyridin-2-carbonsäure-1-(2-ethylbutyl)ester

### Beispiel 642

5-[((4-(2-(Acetylamino)ethyl)phenylsulfonyl)amino)carbonyl]-pyridin-2-carbonsäure-1-(2,2-dimethylpropyl)ester

### Beispiel 643

5-[((4-(2-(n-Butanoylamino)ethyl)phenylsulfonyl)amino)carbonyl]-pyridin-2-carbonsäure-1-(2,2-dimethylpropyl)ester

### Beispiel 644

5-[((4-(2-(n-Pentanoylamino)ethyl)phenylsulfonyl)amino)carbonyl]-pyridin-2-carbonsäure-1-(2,2-dimethylpropyl)ester

### Beispiel 645

5-[((4-(2-(n-Hexanoylamino)ethyl)phenylsulfonyl)amino)carbonyl]-pyridin-2-carbonsäure-1-(2,2-dimethylpropyl)ester

### Beispiel 646

5-[((4-(2-(4-Methylpentanoylamino)ethyl)phenylsulfonyl)amino)carbonyl]-pyridin-2-carbonsäure-1-(2,2-dimethylpropyl)ester

### Beispiel 647

5-[((4-(2-(n-Heptanoylamino)ethyl)phenylsulfonyl)amino)carbonyl]-pyridin-2-carbonsäure-1-(2,2-dimethylpropyl)ester

### Beispiel 648

5-[((4-(2-(n-Octanoylamino)ethyl)phenylsulfonyl)amino)carbonyl]-pyridin-2-carbonsäure-1-(2,2-dimethylpropyl)ester

### Beispiel 649

5-[((4-(2-((2-Methypropionyl)amino)ethyl)phenylsulfonyl)amino)carbonyl]-pyridin-2-carbonsäure-1-(2,2-dimethylpropyl)ester

### Beispiel 650

5-[((4-(2-(Benzoylamino)ethyl)phenylsulfonyl)amino)carbonyl]-pyridin-2-carbonsäure-1-(2,2-dimethylpropyl)ester

### Beispiel 651

5-[((4-(2-((n-Butoxybenzoyl)amino)ethyl)phenylsulfonyl)amino)carbonyl]-pyridin-2-carbonsäure-1-(2,2-dimethylpropyl)ester

### Beispiel 652

5-[((4-(2-((5-Chlor-2-methoxybenzoyl)amino)ethyl)phenylsulfonyl)amino) carbonyl]-pyridin-2-carbonsäure-1-(2,2-dimethylpropyl)ester

### Beispiel 653

5-[((4-(2-((3-Phenyl-n-propionyl)amino)ethyl)phenylsulfonyl)amino)carbonyl]-pyridin-2-carbonsäure-1-(2,2-dimethylpropyl)ester

### Beispiel 654

5-[((4-(2-((2-Phenylacetyl)amino)ethyl)phenylsulfonyl)amino)carbonyl]-pyridin-2-carbonsäure-1-(2,2-dimethylpropyl)ester

### Beispiel 655

5-[((4-(2-((Phenoxyacetyl)amino)ethyl)phenylsulfonyl)amino)carbonyl]-pyridin-2-carbonsäure-1-(2,2-dimethylpropyl)ester

### Beispiel 656

5-[((4-(2-((4-Fluorbenzoyl)amino)ethyl)phenylsulfonyl)amino)carbonyl]-pyridin-2-carbonsäure-1-(2,2-dimethylpropyl)ester

### Beispiel 657

5-[((4-(2-((4-Ethoxybenzoyl)amino)ethyl)phenylsulfonyl)amino)carbonyl]-pyridin-2-carbonsäure-1-(2,2-dimethylpropyl)ester

### Beispiel 658

5-[((4-(2-((Cyclohexanoyl)amino)ethyl)phenylsulfonyl)amino)carbonyl]-pyridin-2-carbonsäure-1-(2,2-dimethylpropyl)ester

### Beispiel 659

5-[((4-(2-((Cyclohexylacetyl)amino)ethyl)phenylsulfonyl)amino)carbonyl]-pyridin-2-carbonsäure-1-(2,2-dimethylpropyl)ester

### Beispiel 660

5-[((3-(2-((2-Chlor-5-methoxybenzoyl)amino)ethyl)phenylsulfonyl)amino) carbonyl]-pyridin-2-carbonsäure-1-(2,2-dimethylpropyl)ester

## Patentansprüche

1. Acylsulfonamido- und Sulfonamidopyridin-2-carbonsäureester sowie ihre Pyridin-N-oxide der allgemeinen Formel I 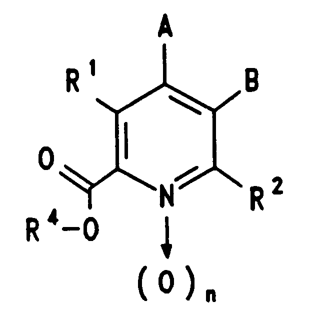 worin
A = R³ und B = X-NR⁵R⁶ oder
B = R³ und A = X-NR⁵R⁶ und
X eine Einfachbindung oder -CO-
bedeutet und
R¹, R² und R³ gleich oder verschieden sind und Wasserstoff, (C₁-C₆)-Alkyl, (C₁-C₆)-Alkoxy, Halogen, insbesondere Fluor, Chlor oder Brom, Nitril, Hydroxy, Amino, ggf. mono- oder disubstituiert mit (C₁-C₄)-Alkyl, Hydroxy-(C₁-C₄)-alkyl, (C₁-C₆)-Alkylcarbonyloxy,
R⁴ den Rest eines Alkohols R⁴OH bedeutet, worin R⁴ insbesondere (C₁-C₁₀)-Acyloxy-(C₁-C₆)-alkyl, vorzugsweise (C₁-C₁₀)-Alkanoyloxy-(C₁-C₆)-alkyl, Benzyloxy-(C₁-C₆)-alkyl, Benzyloxycarbonyloxy-(C₁-C₆)-alkyl oder Alkoxycarbonyloxy-(C₁-C₆)-alkyl, einen verzweigten oder unverzweigten oder cyclischen aliphatischen (C₁-C₁₆)-Alkyl-Rest, oder einen verzweigten oder unverzweigten ggf. cyclischen (C₁-C₁₆)-Alkenyl-Rest, einen (C₁-C₁₆)-Alkinyl-Rest, oder einen (C₁-C₁₆)-Alkeninyl-Rest bedeutet, die jeweils eine oder mehrere Mehrfachbindungen enthalten können,
einen (C₆-C₁₆)-Aryl-Rest, einen (C₇-C₁₆)-Aralkyl-Rest oder einen 5- oder 6-gliedrigen, vorzugsweise Stickstoff-enthaltenden Heteroaryl- oder einen 5- oder 6-gliedrigen, vorzugsweise Stickstoff-enthaltenden Heteroaralkyl-Rest bedeutet, wobei die vorstehenden Reste insbesondere einen oder mehrere Substituenten aus der Reihe
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]ₓC_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)-carbamoyl, N-(C₁-C₁₀)-Alkyl-N-((C₁-C₁₀)-Alkoxy-(C₁-C₁₀)-alkyl)carbamoyl, N-(C₁-C₁₀)-Alkyl-N-((C₆-C₁₆)-Aryloxy-(C₁-C₁₀)-alkyl)carbamoyl, N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-Alkoxy-(C₁-C₁₀)-alkyl)carbamoyloxy, N-((C₆-C₁₆)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-Alkoxy-(C₁-C₁₀)-alkyl)carbamoyloxy, N-(C₁-C₁₀)-Alkyl-N-((C₆-C₁₆)-Aryloxy-(C₁-C₁₀)-alkyl)carbamoyloxy, N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁ )-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl, (C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido, N-((C₁-C₁₀)-alkyl-(C₇-C₁₆)-Aralkylsulfonamido, tragen, wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₁-c₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkoy,
(C₁-C₁₂)Alkylcarbonyl, (C₃-C₈)-Cycloalkylcarbonyl, (C₆-C₁₂)-Arylcarbonyl,
(C₇-C₁₆)-Aralkylcarbonyl, (C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl,
N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy,
N-(C₇-C₁₆)-Aralkylcarbamoyloxy, N-(C₁-C₁₀)-Alkyl-
N-(C₆-C₁₂)-arylcarbamoyloxy,
N-(C₁-C₁₀)-Alkyl-N-(C₇-C₁₆)-Aralkylcarbamoyloxy,
N-((C₁-C₁₀)-Alkoxy-(C₁-C₁₀)-alkyl)carbamoyloxy,
N-((C₆-C₁₆)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-Alkoxy-(C₁-C₁₀)-alkyl)carbamoyloxy,
N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁ )-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
R⁵ Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1-3fach substituiert sein kann mit Hydroxy oder (C₁-C₄)-Alkoxy, oder ein Kation eines basischen Aminosäurederivates bedeutet
R⁶ einen Rest der Formel II ausgenommen -SO₂H bedeutet
Y-[C-U]ᵣ-D-W (II)
in welchem
Y -SO₂- oder -CO- bedeutet,
C eine Bindung oder
einen verzweigten oder unverzweigten aliphatischen (C₁-C₁₆)-Alkandiyl- oder cycloaliphatischen (C₃-C₁₀)-Alkandiylrest oder
einen verzweigten oder unverzweigten (C₂-C₁₆)-Alkendiyl- oder Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
U eine Bindung oder
Wasserstoff oder
einen Rest aus der Reihe folgender Heteroatomgruppierungen bedeutet
-CO-, -O(CO)-, -(CO)-O-, -(CO)NR-, -NR(CO)-, -O-, -SO-, -SO₂-, -NR, worin R (C₁-C₃)-Alkyl oder Wasserstoff bedeutet,
r 1, 2, 3 oder 4 ist,
D eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest bedeutet, der jeweils eine oder mehrere C-C-Mehrfachbindungen enthalten kann,
W eine Bindung oder
Wasserstoff oder
einen (C₃-C₁₀) cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest bedeutet, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C, D und/oder W, sofern diese keine Bindung oder Wasserstoff bedeuten, vorzugsweise ihrerseits substituiert sind durch eine Kombination von bis zu 5 gleichen oder verschiedenen Substituenten aus der Reihe Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl,
N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy,
N-(C₆-C₁₆)-Arylcarbamoyloxy, N-(C₇-C₁₆)-Aralkylcarbamoyloxy,
N-(C₁-C₁₀)-Alkyl-N-(C₆-C₁₂)-arylcarbamoyloxy,
N-(C₁-C₁₀)-Alkyl-N-(C₇-C₁₆)-Aralkylcarbamoyloxy
N-((C₁-C₁₀)-Alkoxy-(C₁-C₁₀)-alkyl)carbamoyloxy,
N-((C₆-C₁₆)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-Alkoxy-(C₁-C₁₀)-alkyl)carbamoyloxy,
N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₁₀)-Alkyl-(C₇-C₁₀)-Aralkylamino, N-(C₁-C₁₀)-Alkyl-N-(C₆-C₁₂)-Arylamino, (C₁-C₁₂)-Alkoxy-amino, (C₁-C₁₂)-Alkoxy-(C₁-C₁₀)-alkylamino, (C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-C₁₀)-alkylamino-(C₁-C₁₀)-alkyl, (C₃-C₈)-cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl,(C₃-C₈)-cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido,
N-((C₁-C₁₀)-alkyl-(C₇-C₁₆)-Aralkylsulfonamido,
wobei die Reste, die einen Arylrest enthalten, ihrerseits am Aryl substituiert sein können mit 1, 2, 3, 4 oder 5 gleichen oder verschiedenen Substituenten aus der Reihe, Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H(_{2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl,
N-(C₇-C₁₆)-Aralkylcarbamoyl, N-(C₁-C₁₀)-Alkyl-N(C₆-C₁₆)-arylcarbamoyl,
N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy,
N-(C₁-C₁₀)-Alkyl-N-(C₆-C₁₂)-arylcarbamoyloxy,
N-(C₁-C₁₀)-Alkyl-N-(C₇-C₁₆)-Aralkylcarbamoyloxy
N-((C₁-C₁₀)-Alkoxy-(C₁-C₁₀)-alkyl)carbamoyloxy,
N-((C₆-C₁₆)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-Alkoxy-(C₁-C₁₀)-alkyl)carbamoyloxy,
N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₁₀)-Alkyl-N-(C₇-C₁₆)-Aralkylamino, N-(C₁-C₁₀)-Alkyl-N-(C₆-C₁₂)-Arylamino, (C₁-C₁₂)-Alkoxy-amino,
(C₁-C₁₂)-Alkoxy-N-(C₁-C₁₀)-alkylamino, (C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-C₁₀)-alkylamino-(C₁-C₁₀)-alkyl, (C₃-C₈)-cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl,(C₃-C₈)-cycloalkylsulfamoyl,
N-(C₆-C₁₆)-Arylsulfamoyl,
N-(C₇-C₁₆)-Aralkylsulfamoyl,
N-(C₁-C₁₀)-Alkyl-N-(C₆-C₁₆)-Arylsulfamoyl,
N-(C₁-C₁₀)-Alkyl-N-(C₇-C₁₆)-Aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido,
N-((C₁-C₁₀)-alkyl-(C₇-C₁₆)-Aralkylsulfonamido,
und
n 0 oder 1,
f 1 bis 8, vorzugsweise 1 bis 5,
g 0, 1 bis (2f+1) und
x 0 bis 8, vorzugsweise 0 oder 1 bedeuten, ausgenommen
5-[((Methylsulfonyl)amino)carbonyl]-pyridin-2-carbonsäuremethylester,
5-[((2-Propylsulfonyl)amino)carbonyl]-pyridin-2-carbonsäuremethylester,
5-[((Phenylsulfonyl)amino)carbonyl]-pyridin-2-carbonsäuremethylester,
5-[((Benzylsulfonyl)amino)carbonyl]-pyridin-2-carbonsäuremethylester,
5-[((4-Methoxyphenylsulfonyl)amino)carbonyl]-pyridin-2-carbonsäuremethylester,
5-[((1-Naphthylsulfonyl)amino)carbonyl]-pyridin-2-carbonsäuremethylester,
5-[((4,5-Dibrom-2-thienylsulfonyl)amino)carbonyl]-pyridin-2-carbonsäuremethylester,
5-[((5-Chlor-2-thienylsulfonyl)amino)carbonyl]-pyridin-2-carbonsäuremethylester,
5-[((8-Chinolylsulfonyl)amino)carbonyl]-pyridin-2-carbonsäuremethylester,
5-[((4-(2-(4,7-Dichlorchinolyl))phenyl-sulfonyl)amino)carbonyl]-pyridin-2-carbonsäuremethylester.

2. Verbindungen nach Anspruch 1, in denen
X eine Einfachbindung oder -CO- ist,
R¹, R² und R³ gleich oder verschieden sind und Wasserstoff, (C₁-C₆)-Alkyl, (C₁-C₆)-Alkoxy, Halogen, insbesondere Fluor oder Chlor, Hydroxy, Amino,
R⁴ einen Rest eines Alkohols R⁴OH bedeutet, worin R⁴ (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl
oder (C₁-C₆)-Alkoxycarbonyloxy-(C₁-C₆)-alkyl, einen verzweigten oder unverzweigten aliphatischen oder cycloaliphatischen (C₁-C₁₂)-Alkyl-Rest,
einen verzweigten oder unverzweigten (C₁-C₁₂)-Alkenyl-Rest, einen (C₂-C₁₂)-Alkinyl-Rest, oder einen (C₂-C₁₂)-Alkeninyl-Rest bedeutet, der jeweils eine oder mehrere Mehrfachbindungen enthalten kann, oder
einen (C₆-C₁₆)-Aryl-Rest, einen (C₆-C₁₆)-Aralkyl-Rest, einen Heteroaryl- oder einen Heteroaralkyl-Rest bedeutet,
wobei die vorstehenden Reste einen oder zwei Substituenten aus der Reihe Hydroxy, Halogen, Cyano, Trifluormethyl, Nitro, Carboxy, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-Aryloxy, (C₇-C₁₆)-Aralkyloxy, (C₁-C₈)-Hydroxyalkyl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl,
N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy,
N-(C₇-C₁₆)-Aralkylcarbamoyloxy, N-(C₁-C₁₀)-Alkyl-
N-(C₆-C₁₂)-arylcarbamoyloxy,
N-(C₁-C₁₀)-Alkyl-N-(C₇-C₁₆)-Aralkylcarbamoyloxy,
N-((C₁-C₁₀)-Alkoxy-(C₁-C₁₀)-alkyl)carbamoyloxy,
N-((C₆-C₁₆)-Aryloxy-(C₁-C₁₀)-alkyl)carbamoyloxy,
N-((C₇-C₁₆)-Aralkyloxy-(C₁-C₁₀)-alkyl)carbamoyloxy,
N-(C₁-C₁₀)-Alkyl-N-((C₁-C₁₀)-Alkoxy-(C₁-C₁₀)-alkyl)carbamoyloxy,
N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, N-(C₇-C₁₁)-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino tragen können, und
wobei die Reste, die einen Arylrest enthalten, ihrerseits im Arylteil substituiert sein können durch 1 bis 5 gleiche oder verschiedene Reste der Reihe Hydroxy, Halogen, Cyano, Trifluormethyl, (C₁-C₁₂)-Alkyl, (C₃-C₈)-Cycloalkyl, (C₁-C₁₂)-Alkoxy, (C₁-C₁₂)-Alkoxy-(C₁-C₁₂)-alkoxy,
(C₁-C₁₂)-Alkylcarbonyl, (C₃-C₈)-Cycloalkylcarbonyl,
(C₁-C₁₂)-Alkoxycarbonyl, (C₁-C₁₂)-Alkoxy-(C₁-C₁₂)-alkoxycarbonyl, (C₃-C₈)-Cycloalkoxycarbonyl,
(C₁-C₁₂)-Alkylcarbonyloxy, (C₃-C₈)-Cycloalkylcarbonyloxy,
(C₁-C₁₂)-Alkoxycarbonyloxy, (C₁-C₁₂)-Alkoxy-(C₁-C₁₂)-alkoxycarbonyloxy, (C₃-C₈)-Cycloalkoxycarbonyloxy, Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl,
N-((C₁-C₁₀)-Alkoxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy,
Amino, (C₁-C₁₂)-Alkylamino, Di-(C₁-C₁₂)-alkylamino, (C₃-C₈)-Cycloalkylamino,
(C₁-C₁₂)-Alkanoylamino, (C₃-C₈)-Cycloalkanoylamino,
(C₁-C₁₂-Alkanoylamino-(C₁-C₈)-alkyl, (C₃-C₈)-Cycloalkanoylamino-(C₁-C₈)-alkyl,
(C₁-C₁₂)Alkylmercapto, (C₁-C₁₂)-Alkylsulfinyl, (C₁-C₁₂)-Alkylsulfonyl,
R⁵ Wasserstoff, (C₁-C₃)-Alkyl, (C₁-C₄)-Alkanoyl oder ein 1,2 oder 3-wertiges physiologisch verwendbares Kation, insbesondere Na⊕, K⊕, Mg²⊕, Ca²⊕ oder ein Ammoniumion, ggf. 1-3fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1-3fach substituiert sein kann mit Hydroxy und/oder (C₁-C₄)-Alkoxy, oder ein Kation eines basischen Aminosäurederivates bedeutet,
R⁶ einen Rest der Formel II ausgenommen -SO₂H bedeutet
-Y-[C-U]ᵣ-D-W (II)
in welchen
Y -SO₂- bedeutet,
C eine Bindung, oder
einen verzweigten oder unverzweigten aliphatischen (C₁-C₁₂)-Alkandiyl-rest, oder
einen verzweigten oder unverzweigten (C₂-C₁₂)-Alkendiyl-Rest, einen (C₂-C₁₂)-Alkindiyl-Rest oder einen (C₂-C₁₂)-Alkenindiyl-Rest bedeutet, der eine oder mehrere C-C-Mehrfachbindungen enthalten kann,
U eine Bindung oder
Wasserstoff oder einen Rest aus der Reihe folgender Heteroatomgruppierungen bedeutet -(CO)NR-, -NR(CO)-, -O-, -SO-, SO₂-, worin R (C₂-C₃)-Alkyl oder Wasserstoff bedeutet,
r 1 oder 2 ist,
D eine Bindung oder
Wasserstoff oder
einen verzweigten oder unverzweigten aliphatischen (C₁-C₈)-Alkandiyl-Rest bedeutet, oder
einen verzweigten oder unverzweigten (C₂-C₈)-Alkendiyl-Rest oder (C₂-C₈)-Alkindiyl-Rest und
W eine Bindung oder
Wasserstoff oder
einen (C₃-C₁₀) cycloaliphatischen Alkyl-, Alkenyl-, Alkinyl- oder Alkeninyl-Rest oder einen (C₆-C₁₆)-Arylrest oder einen 5- oder 6-gliedrigen Heteroarylrest bedeutet, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C, D und/oder W, sofern diese keine Bindung oder Wasserstoff bedeuten, vorzugsweise ihrerseits substituiert sind durch eine Kombination von bis zu 5 gleichen oder verschiedenen Substituenten aus der Reihe
Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f + 1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₅)-Alkyl-N(C₇-C₁₀)-Aralkylamino, N-(C₁-C₅)-Alkyl-N(C₆-C₁₂)-Arylamino, (C₁-C₁₂)-Alkoxy-amino,
(C₁-C₁₂)-Alkoxy-N-(C₁-C₁₀)-alkylamino, (C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)alkylamino-(C₁-C₁₀)alkyl, N,N-Di-(C₁-C₁₀)-alkylamino-(C₁-C₁₀)-alkyl, (C₃-C₈)-cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl,
(C₇-C₁₆)-Aralkylmercapto, (C₇-C₁₆)-Aralkylsulfinyl, (C₇-C₁₆)-Aralkylsulfonyl,
wobei die Reste, die einen Arylrest enthalten, ihrerseits am Aryl substituiert sein können mit 1 , 2, 3, 4 oder 5 gleichen oder verschiedenen Substituenten aus der Reihe Wasserstoff, Hydroxy, Halogen, Cyano, Trifluormethyl, Nitro, Carboxyl,
(C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f + 1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₅)-Alkyl-(C₇-C₁₀)-Aralkylamino, N-(C₁-C₅)-Alkyl-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)alkyl, N,N-Di(C₁-C₁₀)-alkylamino-(C₁-C₁₀)-alkyl, (C₃-C₈)-cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl ausgenommen die Verbindungen:
5-[((Methylsulfonyl)amino)carbonyl]-pyridin-2-carbonsäuremethylester,
5-[((2-Propylsulfonyl)amino)carbonyl]-pyridin-2-carbonsäuremethylester,
5-[((Phenylsulfonyl)amino)carbonyl]-pyridin-2-carbonsäuremethylester,
5-[((Benzylsulfonyl)amino)carbonyl]-pyridin-2-carbonsäuremethylester,
5-[((4-Methoxyphenylsulfonyl)amino)carbonyl]-pyridin-2-carbonsäuremethylester,
5-[((1-Naphthylsulfonyl)amino)carbonyl]-pyridin-2-carbonsäuremethylester,
5-[((4,5-Dibrom-2-thienylsulfonyl)amino)carbonyl]-pyridin-2-carbonsäuremethylester,
5-[((5-Chlor-2-thienylsulfonyl)amino)carbonyl]-pyridin-2-carbonsäuremethylester,
5-[((8-Chinolylsulfonyl)amino)carbonyl]-pyridin-2-carbonsäuremethylester,
5-[((4-(2-(4,7-Dichlorchinolyl))phenylsulfonyl)amino)carbonyl]-pyridin-2-carbonsäuremethylester.

3. Verbindungen nach Anspruch 1 oder 2, in denen
X eine Einfachbindung oder -CO- ist
R¹, R² und R³ gleich oder verschieden sind und Wasserstoff oder (C₁-C₃)-Alkyl, (C₁-C₃)-Alkoxy, Hydroxy, Fluor oder Chlor,
R⁴ den Rest eines Alkohols R⁴OH bedeutet, worin R⁴ (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder
(C₁-C₆)-Alkoxycarbonyloxy-(C₁-C₆)-alkyl,
einen verzweigten oder unverzweigten oder cyclischen aliphatischen (C₁-C₁₀)-Alkyl-Rest, oder einen verzweigten oder unverzweigten (C₂-C₁₀)-Alkenyl-Rest oder
einen (C₂-C₁₂)-Alkinyl-Rest, der jeweils eine oder mehrere C-C-Mehrfachbindungen enthalten kann, oder
einen (C₆-C₁₆)-Aryl-Rest, einen (C₇-C₁₁)-Aralkyl-Rest oder einen Heteroaryl- oder Heteroalkyl-Rest bedeutet,
wobei die vorstehenden Reste einen oder zwei Substituenten aus der Reihe
Hydroxy, (C₁-C₆)-Alkoxy, (C₁-C₆)-Alkoxy-(C₁-C₆)-alkoxy, (C₆-C₁₂)-Aryloxy, (C₇-C₁₂)-Aralkyloxy,
(C₁-C₈)-Alkylcarbonyl, (C₃-C₈)-Cycloalkylcarbonyl, (C₆-C₁₂)-Arylcarbonyl, (C₇-C₁₂)-Aralkylcarbonyl,
(C₁-C₈)-Alkoxycarbonyl, (C₁-C₆)-Alkoxy-(C₁-C₆)-alkoxycarbonyl, (C₆-C₁₂)- Aryloxycarbonyl, (C₇-C₁₂)-Aralkoxycarbonyl, (C₃-C₈)-Cycloalkoxycarbonyl,
(C₁-C₈)-Alkylcarbonyloxy, (C₃-C₈)-Cycloalkylcarbonyloxy, (C₆-C₁₂)-Arylcarbonyloxy, (C₇-C₁₂)-Aralkylcarbonyloxy,
(C₁-C₈)-Alkoxycarbonyloxy, (C₁-C₆)-Alkoxy-(C₁-C₆)-alkoxycarbonyloxy, (C₆-C₁₂)-Aryloxycarbonyloxy, (C₇-C₁₂)-Aralkyloxycarbonyloxy, (C₃-C₈)-Cycloalkoxycarbonyloxy,
Carbamoyl, N-(C₁-C₈)-Alkylcarbamoyl, N,N-Di-(C₁-C₈)-alkylcarbamoyl, N-(C₃-C₈)-Cycloalkylcarbamoyl,
N-((C₁-C₆)-Alkoxy-(C₁-C₆)alkyl)carbamoyl,
Amino, (C₁-C₆)-Alkylamino, Di-(C₁-C₆)alkylamino, (C₃-C₈)-Cycloalkylamino, N-(C₆-C₁₂)-Arylamino, N-(C₇-C₁₁)-Aralkylamino, N-(C₁-C₅)-Alkyl-(C₆-C₁₂)-Arylamino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₆)alkylamino, (C₇-C₁₁)-Aralkanoyl-N-(C₁-C₆)-alkylamino tragen können, und
wobei die Reste, die einen Arylrest enthalten, insbesondere substituiert sind mit bis zu 3 Substituenten aus der Reihe Hydroxy, Fluor, Chlor, Cyano, Trifluormethyl, (C₁-C₆)-Alkyl, (C₃-C₈)-Cycloalkyl, (C₁-C₈)-Alkoxy,
(C₁-C₆)-Alkylcarbonyl, (C₃-C₈)-Cycloalkylcarbonyl,
(C₁-C₆)-Alkoxycarbonyl, (C₃-C₈)-Cycloalkoxycarbonyl,
(C₁-C₆)-Alkylcarbonyloxy, (C₃-C₈)-Cycloalkylcarbonyloxy, (C₁-C₆)-Alkoxycarbonyloxy, (C₃-C₈)-
Cycloalkoxycarbamoyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl,
N-((C₁-C₆)-Alkoxy-(C₁-C₆)-alkyl)carbamoyl,
N-(C₁-C₆)-Alkyl-N-((C₁-C₆)-Alkoxy-(C₁-C₆)-alkyl)carbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyloxy, Amino, (C₁-C₆)-Alkylamino, Di-(C₁-C₆)-alkylamino, (C₃-C₈)-Cycloalkylamino,
(C₁-C₆)-Alkanoylamino, (C₃-C₈)-Cycloalkanoylamino,
(C₁-C₆)Alkylmercapto, (C₁-C₆)-Alkylsulfinyl, (C₁-C₆)-Alkylsulfonyl, und
R⁵ Wasserstoff, (C₁-C₃)-Alkyl, (C₁-C₄)-Alkanoyl oder ein 1,2 oder 3-wertiges physiologisch verwendbares Kation, insbesondere Na⊕, K⊕, Mg²⊕, Ca²⊕ oder ein Ammoniumion bedeutet, ggf. 1-3fach substituiert mit
(C₁-C₈)-Hydroxyalkyl, (C₁-C₄)-Alkoxy-(C₁-C₈)-alkyl, Phenyl, Benzyl oder (C₁-C₈)-Alkyl, welches 1-3fach substituiert sein kann mit Hydroxy und/oder (C₁-C₄)-Alkoxy,
R⁶ einen Rest der Formel II ausgenommen -SO₂H bedeutet,
-Y-[C-U]ᵣ-D-W (II)
in welchem
Y -SO₂- bedeutet,
C eine Bindung oder
einen (C₁-C₆)-Alkandiyl-Rest bedeutet,
U eine Bindung oder
Wasserstoff oder -O- bedeutet,
r 1 ist,
D eine Bindung oder
Wasserstoff oder
einen unverzweigten aliphatischen (C₁-C₈)-Alkandiyl-Rest bedeutet, und
W eine Bindung oder
Wasserstoff, einen (C₆-C₁₂)-Arylrest oder einen 5-oder 6-gliedrigen Heteroarylrest bedeutet, wobei mindestens eine der Variablen C oder D oder W nicht eine Bindung bedeuten und U nur dann in der Bedeutung einer
Heteratomgruppierung steht, wenn C nicht eine Bindung bedeutet oder wenn D und/oder W nicht eine Bindung bedeuten und
C, D und/oder W, sofern diese keine Bindung oder Wasserstoff bedeuten, vorzugsweise ihrerseits substituiert sind mit bis zu 3 gleichen oder verschiedenen Substituenten aus der Reihe Hydroxy, Halogen, Cyano, Trifluormethyl, Nitro, Carboxyl, (C₁-C₈)-Alkyl, (C₃-C₈)-Cycloalkyl, (C₆-C₁₂)-Aryl, (C₇-C₁₆)-Aralkyl, (C₃-C₈)-Alkenyl, (C₃-C₈)-Alkinyl, (C₁-C₈)-Alkoxy, (C₁-C₈)-Alkoxy-(C₁-C₈)-alkyl, (C₆-C₁₂)-Aryloxy, (C₇-C₁₆)-Aralkyloxy, (C₁-C₈)-Hydroxyalkyl, -O-[CH₂₋]_{X}C_{f}H_{(2f + 1-g})F_{g},
(C₁-C₈)-Alkoxycarbonyl, (C₆-C₁₂)-Aryloxycarbonyl, (C₇-C₁₄)-Aralkoxycarbonyl, (C₃-C₈)-Cycloalkoxycarbonyl,
(C₁-C₈)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl₁ N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl,
N-((C₇-C₁₆)-Aralkyloxy-(C₁-C₁₀)-alkyl)carbamoyl,
N-(C₁-C₁₀)-Alkyl-N-((C₁-C₁₀)-Alkoxy-(C₁-C₁₀)-alkyl)carbamoyl,
N-(C₁-C₁₀)-Alkyl-N-((C₆-C₁₆)-Aryloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl,
Amino, (C₁-C₈)-Alkylamino, Di-(C₁-C₈)-alkylamino, (C₃-C₈)-Cycloalkylamino, N-(C₆-C₁₂)-Arylamino, N-(C₇-C₁₁)-Aralkylamino, N-(C₁-C₃)-Alkyl-N-(C₇-C₁₁)-Aralkylamino,
(C₁-C₁₀)-Alkanoylamino, (C₃-C₈)-Cycloalkanoylamino, (C₆-C₁₂)-Aroylamino, (C₇-C₁₆)-Aralkanoylamino, (C₁-C₁₀)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₆)-alkyl, (C₃-C₈)-Cycloalkanoylamino-(C₁-C₈)alkyl, (C₆-C₁₆)-Aroylamino-(C₁-C₆)-alkyl, (C₇-C₁₆)-Aralkanoylamino-(C₁-C₆)-alkyl,
(C₁-C₈)-Alkylmercapto, (C₁-C₈)-Alkylsulfinyl, (C₁-C₈)-Alkylsulfonyl,
(C₆-C₁₂)-Arylmercapto, (C₆-C₁₂)-Arylsulfinyl, (C₆-C₁₂)-Arylsulfonyl,
(C₇-C₁₄)-Aralkylmercapto, (C₇-C₁₄)-Aralkylsulfinyl, (C₇-C₁₄)-Aralkylsulfonyl,
wobei die Reste, die einen Arylrest enthalten, ihrerseits am Aryl substituiert sein können mit 1, 2, 3, 4 oder 5 gleichen oder verschiedenen Substituenten aus der Reihe Wasserstoff, Hydroxy, Halogen, Cyano, Trifluormethyl, Nitro, Carboxyl, (C₁-C₈)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₈)-Alkoxy, (C₁-C₆)-Alkoxy-(C₁-C₆)alkyl, (C₆-C₁₂)-Aryloxy, (C₇-C₁₆)-Aralkyloxy, -O-[CH₂₋]_{X}C_{f}H_{(2f + 1-g)}F_{g},
(C₁-C₁₂)-Alkoxycarbonyl, (C₃-C₈)-Cycloalkoxycarbonyl,
(C₁-C₁₂)-Alkylcarbonyloxy, (C₃-C₈)-Cycloalkylcarbonyloxy,
Carbamoyl, N-(C₁-C₈)-Alkylcarbamoyl, N,N-Di-(C₁-C₈)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₈)-Alkyl-N-(C₆-C₁₆)-arylcarbamoyl, N-(C₁-C₈)-Alkyl-N-(C₇-C₆)-Aralkylcarbamoyl,
N-((C₁-C₆)-Alkoxy-(C₁-C₆)-alkyl)carbamoyl,
N-((C₆-C₆)-Aryloxy-(C₁-C₆)-alkyl)carbamoyl,
N-((C₇-C₁₆)-Aralkyloxy-(C₁-C₆)-alkyl)carbamoyl,
N-(C₁-C₈)-Alkyl-N-((C₁-C₆)-Alkoxy-(C₁-C₆)-alkyl)carbamoyl,
N-(C₁-C₈)-Alkyl-N-((C₆-C₁₆)-Aryloxy-(C₁-C₆)-alkyl)carbamoyl,
N-(C₁-C₈)-Alkyl-N-((C₇-C₁₆)-Aralkyloxy-(C₁-C₆)-alkyl)carbamoyl,
Amino, (C₁-C₆)-Alkylamino, Di-(C₁-C₆)-alkylamino, (C₃-C₈)-Cycloalkylamino, N-(C₆-C₁₂)-Arylamino, N-(C₇-C₁₁)-Aralkylamino, N-(C₁-C₃)-Alkyl-(C₇-C₁₁)-Aralkylamino, N-(C₁-C₃)-Alkyl-(C₆-C₁₂)-Arylamino, (C₁-C₈)-Alkoxy-amino,
(C₁-C₈)-Alkanoylamino, (C₃-C₈)-Cycloalkanoylamino, (C₆-C₁₂)-Aroylamino, (C₇-C₁₂)-Aralkanoylamino, (C₁-C₈)-Alkanoyl-N-(C₁-C₆)-alkylamino, (C₃-C₈)-Cycloalkanoyl-N-(C₁-C₆)-alkylamino, (C₆-C₁₂)-Aroyl-N-(C₁-C₁₀)-alkylamino, (C₇-C₁₁)-Aralkanoyl-N-(C₁-C₆)-alkylamino,
(C₁-C₈)-Alkanoylamino-(C₁-C₄)-alkyl, (C₃-C₈)-Cycloalkanoylamino-(C₁-C₄)alkyl, (C₆-C₁₂)-Aroylamino-(C₁-C₄)-alkyl, (C₇-C₁₂)-Aralkanoylamino-(C₁-C₄)-alkyl, ausgenommen die Verbindungen:
5-[((Methylsulfonyl)amino)carbonyl]-pyridin-2-carbonsäuremethylester,
5-[((2-Propylsulfonyl)amino)carbonyl]-pyridin-2-carbonsäuremethylester,
5-[((Phenylsulfonyl)amino)carbonyl]-pyridin-2-carbonsäuremethylester,
5-[((Benzylsulfonyl)amino)carbonyl]-pyridin-2-carbonsäuremethylester,
5-[((4-Methoxyphenylsulfonyl)amino)carbonyl]-pyridin-2-carbonsäuremethylester,
5-[((1-Naphthylsulfonyl)amino)carbonyl]-pyridin-2-carbonsäuremethylester,
5-[((4,5-Dibrom-2-thienylsulfonyl)amino)carbonyl]-pyridin-2-carbonsäuremethylester,
5-[((5-Chlor-2-thienylsulfonyl)amino)carbonyl]-pyridin-2-carbonsäuremethylester,
5-[((8-Chinolylsulfonyl)amino)carbonyl]-pyridin-2-carbonsäuremethylester,
5-[((4-(2-(4,7-Dichlorchinolyl))phenyl-sulfonyl)amino)carbonyl]-pyridin-2-carbonsäuremethylester.

4. Verbindungen nach einem oder mehreren der Ansprüche 1 bis 3, worin
X eine Einfachbindung oder -CO- ist,
R¹, R² und R³ Wasserstoff bedeutet,
R⁴ den Rest eines Alkohols R⁴OH bedeutet und für einen verzweigten oder unverzweigten oder cyclischen aliphatischen (C₁-C₉)-Alkyl-Rest,
einen verzweigten oder unverzweigten (C₁-C₈)-Alkenylrest oder (C₁-C₈)-Alkinylrest bedeutet, einen Phenyl-, Benzyl-, Phenethyl-, Phenylpropylrest,
wobei die vorstehenden Reste einen Substituenten aus der Reihe Wasserstoff, Hydroxy, (C₁-C₄)-Alkoxy,
(C₁-C₆)-Alkoxycarbonyl, (C₆-C₁₂)-Phenoxycarbonyl, (C₇-C₁₆)-Phenylalkylcarbonyl, (C₃-C₈)-Cycloalkoxycarbonyl,
(C₁-C₆)-Alkylcarbonyloxy, (C₃-C₈)-Cycloalkylcarbonyloxy, Benzoyloxy, (C₇-C₁₆)-Phenylalkylcarbonyloxy, (C₃-C₈)-Cycloalkoxycarbonyloxy enthalten,
(C₁-C₆)-Alkoxycarbonyloxy, Phenoxycarbonyloxy, (C₇-C₁₆)-Phenylalkylcarbonyloxy, (C₃-C₈)-Cycloalkoxycarbonyloxy,
R⁵ Wasserstoff oder ein 1, 2 oder 3-wertiges physiologisch verwendbares Kation bedeutet, insbesondere Na⊕, K⊕, Mg²⊕, Ca²⊕ oder H₃N^{⊕}C(CH₂OH)₃ (Tris-Salz),
R⁶ einen Rest der Formel II ausgenommen -SO₂H bedeutet
-Y-[C-U]ᵣ-D-W (II)
in welchem
Y -SO₂- bedeutet,
C eine Bindung oder
(C₁-C₄)-Alkandiyl bedeutet,
U eine Bindung, Wasserstoff oder -O- bedeutet,
r 1 ist,
D eine Bindung, Wasserstoff oder (C₁-C₄)-Alkandiyl,
W eine Bindung, Wasserstoff oder einen Phenylrest bedeutet, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C, D und/oder W substituiert sind durch Wasserstoff oder von 1 oder 2 Substituenten aus der folgenden Reihe stehen Fluor, Chlor, (C₁-C₆)-Alkyl, Phenyl, (C₁-C₆)-Alkoxy, Phenoxy, -O-[CH₂₋]_{X}C_{f}H_{(2f + 1-g)}F_{g},
Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl, N-(C₇-C₁₆)-Phenylalkylcarbamoyl, N-(C₁-C₈)-Alkyl-N-(C₆-C₁₆)-phenyl-carbamoyl, N-(C₁-C₈)-Alkyl-N-(C₇-C₁₆)-Phenylalkylcarbamoyl,
N-((C₁-C₄)-Alkoxy-(C₁-C₈)-alkyl)carbamoyl,
N-Phenoxy-(C₁-C₈)-alkyl)carbamoyl,
N-((C₇-C₁₆)-Phenylalkyloxy-(C₁-C₈)-alkyl)carbamoyl,
N-(C₁-C₈)-Alkyl-N-((C₁-C₆)-Alkoxy-(C₁-C₈)-alkyl)carbamoyl,
N-(C₁-C₈)-Alkyl-N-((C₆-C₁₂)-Phenoxy-(C₁-C₈)-alkyl)carbamoyl,
N-(C₁-C₈)-Alkyl-N-((C₇-C₁₆)-Phenylalkyloxy-(C₁-C₈)-alkyl)carbamoyl,
(C₁-C₈)-Alkanoylamino, (C₃-C₈)-Cycloalkanoylamino, (C₆-C₁₂)-Phenylmino, (C₇-C₁₁)-Phenylalkanoylamino, (C₁-C₈)-Alkanoyl-N-(C₁-C₁₀)-alkylamino, (C₃-C₈)-Cycloalkanoyl-N-(C₁-C₆)-alkylamino, Benzoyl-N-(C₁-C₁₀)-alkylamino, (C₇-C₁₁)-Phenylalkanoyl-N-(C₁-C₆)-alkylamino, (C₁-C₁₀)-Alkanoylamino-(C₁-C₂)-alkyl, (C₃-C₈)-Cycloalkanoylamino-(C₁-C₂)alkyl, Benzoylamino-(C₁-C₂)-alkyl, (C₇-C₁₄)-Phenylalkanoylamino-(C₁-C₂)-alkyl, wobei die Reste, die einen Arylrest enthalten, ihrerseits substituiert sind mit einem Substituenten aus der Reihe Wasserstoff, Hydroxy, Fluor, Chlor, Trifluormethyl, (C₁-C₆)-Alkyl, (C₁-C₈)-Alkoxy,
n 0,
f 1 bis 5,
g 0, 1 bis (2f + 1) und
x 0 oder 1
bedeuten, ausgenommen die Verbindungen
5-[((Methylsulfonyl)amino)carbonyl]-pyridin-2-carbonsäuremethylester,
5-[((2-Propylsulfonyl)amino)carbonyl]-pyridin-2-carbonsäuremethylester,
5-[((Phenylsulfonyl)amino)carbonyl]-pyridin-2-carbonsäuremethylester,
5-[((Benzylsulfonyl)amino)carbonyl]-pyridin-2-carbonsäuremethylester,
5-[((4-Methoxyphenylsulfonyl)amino)carbonyl]-pyridin-2-carbonsäuremethylester,
5-[((1-Naphthylsulfonyl)amino)carbonyl]-pyridin-2-carbonsäuremethylester,
5-[((4,5-Dibrom-2-thienylsulfonyl)amino)carbonyl]-pyridin-2-carbonsäuremethylester,
5-[((5-Chlor-2-thienylsulfonyl)amino)carbonyl]-pyridin-2-carbonsäuremethylester,
5-[((8-Chinolylsulfonyl)amino)carbonyl]-pyridin-2-carbonsäuremethylester,
5-[((4-(2-(4,7-Dichlorchinolyl))phenylsulfonyl)amino)carbonyl]-pyridin-2-carbonsäuremethylester.

5. Verbindungen nach einem oder mehreren der Ansprüche 1 bis 4 zur Anwendung als Arzneimittel.

6. Verfahren zur Herstellung der Verbindungen gemäß einem oder mehreren der Ansprüche 1 bis 5, in denen X eine Einfachbindung bedeutet, dadurch gekennzeichnet, daß
i) eine Verbindung der Formel 1 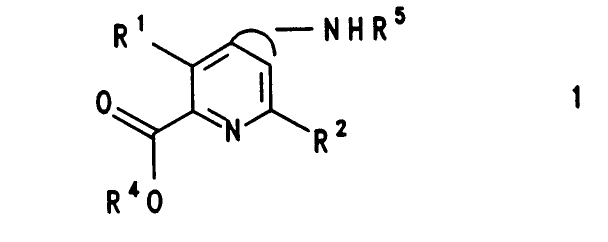 mit ZR⁶ zu einer Verbindung der Formel 3 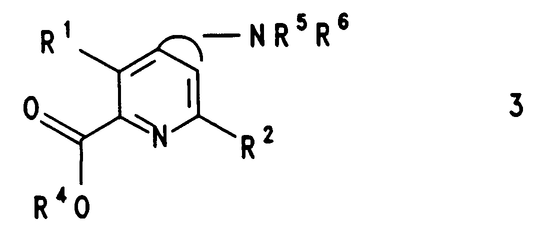 oder
ii) eine Verbindung der Formel 5 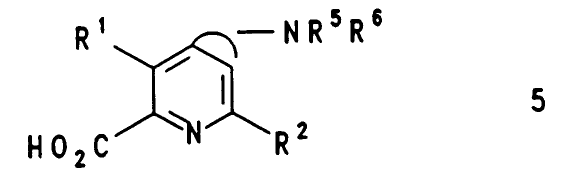 mit R⁴OH zu einer Verbindung der Formel 3 umgesetzt werden, worin R¹, R², R³, R⁴, R⁵ und R⁶ in den Ansprüchen 1 bis 4 definiert sind und Z für Hydroxy oder für eine Abgangsgruppe steht, die nukleophil ablösbar ist und insbesondere F, Cl, Br, J oder Tosulat bedeutet und die Verbindungen der Formel 3 anschließend ggf. zu ihren Pyridin-N-oxiden oxidiert werden.

7. Verfahren zur Herstellung der Verbindungen gemäß einem oder mehreren der Ansprüche 1 bis 5, in denen X für -CO- steht, dadurch gekennzeichnet, daß
i) eine Verbindung der Formel 6 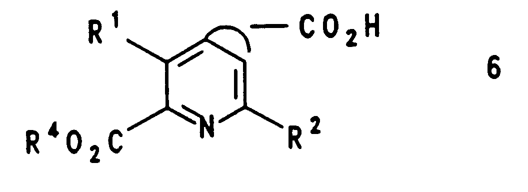 mit NHR⁵R⁶ zu einer Verbindung der Formel 8 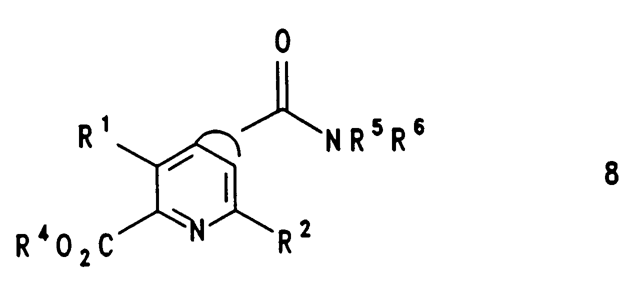 oder
ii) eine Verbindung der Formel 9 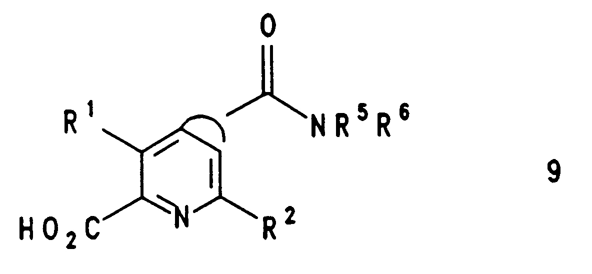 mit R⁴OH zu einer Verbindung der Formel 8
oder
iii) eine Verbindung der Formel 10 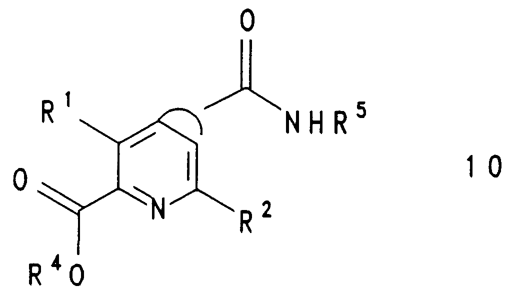 mit ZR⁶ zu einer Verbindung der Formel 8 umgesetzt werden, worin R¹, R², R³, R⁴, R⁵ und R⁶ in den Ansprüchen 1 bis 4 definiert sind und Z für Hydroxy oder für eine Abgangsgruppe steht, die nukleophil ablösbar ist und insbesondere F, Cl, Br, J oder Tosylat bedeutet und die Verbindungen der Formel 8 anschließend ggf. zu ihren Pyridin-N-oxiden oxidiert werden.

8. Verbindungen nach den Ansprüchen 1 bis 4 gegen fibrotische Erkrankungen.

9. Verbindungen nach den Ansprüchen 1 bis 4 zur Anwendung als Fibrosuppresiva.

10. Arzneimittel, enthaltend mindestens eine Verbindung der Formel I und ggf. einen verträglichen pharmazeutischen Träger.

11. Verbindungen der Formel I zur Anwendung in einem Verfahren zur Behandlung von Störungen des Stoffwechsels von Collagen und collagenähnlichen Stoffen.

12. Verbindungen der Formel I zur Anwendung in einem Verfahren zur Behandlung von fibrotischen Erkrankungen.

13. Verfahren zur Herstellung von Arzneimitteln zur Behandlung von fibrotischen Erkrankungen, dadurch gekennzeichnet, daß das Arzneimittel eine Verbindung der Formel I enthält.

## Claims

1. An acylsulfonamido- or sulfonamidopyridine-2-carboxylic acid ester or its pyridine N-oxide of the formula I 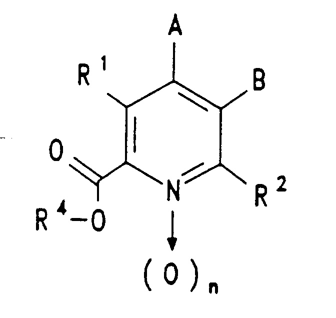 in which
A = R³ and B = X-NR⁵R⁶ or
B = R³ and A = X-NR⁵R⁶ and
X is a single bond or -CO- and
R¹, R² and R³ are identical or different and are hydrogen, (C₁-C₆)-alkyl, (C₁-C₆)-alkoxy, halogen, in particular fluorine, chlorine or bromine, nitrile, hydroxyl, amino, optionally mono- or disubstituted by (C₁-C₄)-alkyl, hydroxy-(C₁-C₄)-alkyl or (C₁-C₆)-alkylcarbonyloxy,
R⁴ is the radical of an alcohol R⁴OH, in which R⁴ is, in particular, (C₁-C₁₀)-acyloxy-(C₁-C₆)-alkyl, preferably (C₁-C₁₀)-alkanoyloxy-(C₁-C₆)-alkyl, benzyloxy-(C₁-C₆)-alkyl, benzyloxycarbonyloxy-(C₁-C₆)-alkyl or alkoxycarbonyloxy-(C₁-C₆)-alkyl, a branched or unbranched or cyclic aliphatic (C₁-C₁₆)-alkyl radical, or
a branched or unbranched, optionally cyclic (C₁-C₁₆)-alkenyl radical, a (C₁-C₁₆)-alkynyl radical or a (C₁-C₁₆)-alkenynyl radical, each of which can contain one or more multiple bonds, or
a (C₆-C₁₆)-aryl radical, a (C₇-C₁₆)-aralkyl radical or a 5- or 6-membered, preferably nitrogen-containing heteroaryl radical or a 5- or 6-membered, preferably nitrogen-containing heteroaralkyl radical, the above radicals carrying, in particular, one or more substituents from the series comprising hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₃-C₁₂)-alkenyloxycarbonyloxy, (C₃-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₆)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkoxy-(C₁-C₁₀)-alkyl)carbamoyloxy, N-((C₆-C₁₆)-aryloxy-(C₁-C₁₀)alkyl)carbamoyloxy, N-((C₇-C₁₆)-aralkyloxy-(C₁-C₁₀)alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₆)-aryloxy-(C₁-C₁₀)-alkyl)carbamoyloxy, N-(C₁-C₁₀)-alkyl-N-((C₇-C₁₆)-aralkyloxy-(C₁-C₁₀)alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl, (C₇-C₁₆)-aralkylsulfonyl,
sulfamoyl, N-(C₁-C₁₀)-alkylsulfamoyl, N,N-di-(C₁-C₁₀)-alkylsulfamoyl, (C₃-C₈)-cycloalkylsulfamoyl,
N-(C₆-C₁₆)-arylsulfamoyl,
N-(C₇-C₁₆)-aralkylsulfamoyl,
N-(C₁-C₁₀)-alkyl-N-(C₆-C₁₆)-arylsulfamoyl,
N-(C₁-C₁₀)-alkyl-N-(C₇-C₁₆)-aralkylsulfamoyl, (C₁-C₁₀)-alkyl-sulfonamido,
N-((C₁-C₁₀)-alkyl)-(C₁-C₁₀)-alkylsulfonamido, (C₇-C₁₆)-aralkylsulfonamido and N-((C₁-C₁₀)-alkyl-(C₇-C₁₆)-aralkylsulfonamido, it being possible for the radicals which contain an aryl radical to be substituted in turn on the aryl by 1 to 5 identical or different radicals from the series comprising:
hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkoxy,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₃-C₁₂)-alkenyloxycarbonyloxy, (C₃-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl,
N-((C₁-C₁₀)-alkoxy-(C₁-C₁₀)-alkyl)carbamoyl,
N-((C₆-C₁₆)-aryloxy-(C₁-C₁₀)-alkyl)carbamoyl,
N-((C₇-C₁₆)-aralkyloxy-(C₁-C₁₀)-alkyl)carbamoyl,
N-(C₁-C₁₀)-alkyl-N-((C₁-C₁₀)-alkoxy-(C₁-C₁₀)-alkyl)carbamoyl,
N-(C₁-C₁₀)-alkyl-N-((C₆-C₁₆)-aryloxy-(C₁-C₁₀)-alkyl)carbamoyl,
N-(C₁-C₁₀)-alkyl-N-((C₇-C₁₆)-aralkyloxy-(C₁-C₁₀)-alkyl)carbamoyl,
carbamoyloxy, N-(C₁-C₁₂)-alkylcarbamoyloxy,
N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy,
N-(C₁-C₁₀)-alkyl-N-(C₆-C₁₂)-arylcarbamoyloxy,
N-(C₁-C₁₀)-alkyl-N-(C₇-C₁₆)-aralkylcarbamoyloxy,
N-((C₁-C₁₀)-alkoxy-(C₁-C₁₀)-alkyl)carbamoyloxy,
N-((C₆-C₁₆)-aryloxy-(C₁-C₁₀)-alkyl)carbamoyloxy,
N-((C₇-C₁₆)-aralkyloxy-(C₁-C₁₀)-alkyl)carbamoyloxy,
N-(C₁-C₁₀)-alkyl-N-((C₁-C₁₀)-alkoxy-(C₁-C₁₀)-alkyl)carbamoyloxy,
N-(C₁-C₁₀)-alkyl-N-((C₆-C₁₆)-aryloxy-(C₁-C₁₀)-alkyl)carbamoyloxy,
N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkylaralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl and (C₇-C₁₆)-aralkylsulfonyl,
R⁵ is hydrogen, (C₁-C₆)-alkyl or an N-protective group, such as (C₁-C₈)-alkanoyl, (C₁-C₆)-alkylcarbamoyl, (C₁-C₆)-alkoxycarbonyl, benzyloxycarbonyl, (C₁-C₁₀)-acyloxy-(C₁-C₆)-alkyl, preferably (C₁-C₁₀)-alkanoyloxy-(C₁-C₆)-alkyl, benzoyloxy-(C₁-C₆)-alkyl, benzyloxycarbonyloxy-(C₁-C₆)-alkyl or (C₁-C₆)-alkoxycarbonyloxy-(C₁-C₆)-alkyl, a mono-, di-, tri- or tetravalent physiologically usable cation, in particular Na^{⊕}, K^{⊕}, Mg^{2⊕}, Ca^{2⊕}, Al^{3⊕} or an ammonium ion, optionally mono-, di- or trisubstituted by (C₁-C₈)-hydroxyalkyl, (C₁-C₄)-alkoxy-(C₁-C₈)-alkyl, phenyl, benzyl or (C₁-C₈)-alkyl, which can be mono-, di- or trisubstituted by hydroxyl or (C₁-C₄)-alkoxy, or a cation of a basic amino acid derivative,
R⁶ is a radical of the formula II, excluding -SO₂H
-Y-[C-U]ᵣ-D-W (II)
in which
Y is -SO₂- or -CO-,
C is a bond or a branched or unbranched aliphatic (C₁-C₁₆)-alkanediyl or cycloaliphatic (C₃-C₁₀)-alkanediyl radical or
a branched or unbranched (C₂-C₁₆)-alkenediyl or cycloalkenediyl radical, or a (C₂-C₁₆)-alkynediyl radical or a (C₂-C₁₆)-alkenynediyl radical, each of which can contain one or more C-C multiple bonds,
U is a bond or
hydrogen or
a radical from the following series of hetero-atom groupings
-CO-, -O(CO)-, -(CO)-O-, -(CO)NR-, -NR(CO)-, -O-, -SO-, -SO₂- and -NR, in which R is (C₁-C₃)-alkyl or hydrogen,
r is 1, 2, 3 or 4,
D is a bond or hydrogen or a branched or unbranched aliphatic (C₁-C₁₀)-alkanediyl radical, or a branched or unbranched (C₁-C₁₀)-alkenediyl radical, a (C₂-C₁₀)-alkynediyl radical or a (C₂-C₁₀)-alkenynediyl radical, each of which can contain one or more C-C multiple bonds,
W is a bond or
hydrogen or
a (C₃-C₁₀) cycloaliphatic alkyl, alkenyl, alkynyl or alkenynyl radical or
a (C₆-C₁₆)-aryl radical or a 5- or 6-membered heteroaryl radical, in which at least one of the variables C or D or W is not a bond and U only denotes a heteroatom grouping if C is not a bond or if D and/or W are not a bond and
C, D and/or W, if these are not a bond or hydrogen, are preferably substituted in turn by a combination of up to 5 identical or different substituents from the series comprising hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₃-C₁₂)-alkenyloxycarbonyloxy, (C₃-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl,
N-((C₁-C₁₀)-alkoxy-(C₁-C₁₀)-alkyl)carbamoyl,
N-((C₆-C₁₆)-aryloxy-(C₁-C₁₀)-alkyl)carbamoyl,
N-((C₇-C₁₆)-aralkyloxy-(C₁-C₁₀)-alkyl)carbamoyl,
N-(C₁-C₁₀)-alkyl-N-((C₁-C₁₀)-alkoxy-(C₁-C₁₀)-alkyl)carbamoyl,
N-(C₁-C₁₀)-alkyl-N-((C₆-C₁₆)-aryloxy-(C₁-C₁₀)-alkyl)carbamoyl,
N-(C₁-C₁₀)-alkyl-N-((C₇-C₁₆)-aralkyloxy-(C₁-C₁₀)-alkyl)carbamoyl,
carbamoyloxy, N-(C₁-C₁₂)-alkylcarbamoyloxy,
N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy,
N-(C₁-C₁₀)-alkyl-N-(C₇-C₁₆)-aralkylcarbamoyloxy,
N-((C₁-C₁₀)-alkoxy-(C₁-C₁₀)-alkyl)carbamoyloxy,
N-((C₆-C₁₆)-aryloxy-(C₁-C₁₀)-alkyl)carbamoyloxy,
N-((C₇-C₁₆)-aralkyloxy-(C₁-C₁₀)-alkyl)carbamoyloxy,
N-(C₁-C₁₀)-alkyl-N-((C₁-C₁₀)-alkoxy-(C₁-C₁₀)-alkyl)carbamoyloxy,
N-(C₁-C₁₀)-alkyl-N-((C₆-C₁₆)-aryloxy-(C₁-C₁₀)-alkyl)carbamoyloxy,
N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkylamino, N-(C₆-C₁₂)-arylamino, N-(C₇-C₁₁)-aralkylamino, N-(C₁-C₁₀)-alkyl-(C₇-C₁₀)-aralkylamino, N-(C₁-C₁₀)-alkyl-N-(C₆-C₁₂)-arylamino, (C₁-C₁₂)-alkoxyamino, (C₁-C₁₂)-alkoxy-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl, (C₇-C₁₆)-aralkylsulfonyl,
sulfamoyl, N-(C₁-C₁₀)-alkylsulfamoyl, N,N-di-(C₁-C₁₀)-alkylsulfamoyl, (C₃-C₈)-cycloalkylsulfamoyl,
N-(C₆-C₁₆)-arylsulfamoyl,
N-(C₇-C₁₆)-aralkylsulfamoyl,
N-(C₁-C₁₀)-alkyl-N-(C₆-C₁₆)-arylsulfamoyl,
N-(C₁-C₁₀)-alkyl-N-(C₇-C₁₆)-aralkylsulfamoyl,
(C₁-C₁₀)-alkyl-sulfonamido,
N-((C₁-C₁₀)-alkyl)-(C₁-C₁₀)-alkylsulfonamido, (C₇-C₁₆)-aralkylsulfonamido and N-((C₁-C₁₀)-alkyl-(C₇-C₁₆)-aralkylsulfonamido,
it being possible for the radicals which contain an aryl radical to be substituted in turn on the aryl by 1, 2, 3, 4 or 5 identical or different substituents from the series comprising hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₃-C₁₂)-alkenyloxycarbonyloxy, (C₃-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl,
N-((C₁-C₁₀)-alkoxy-(C₁-C₁₀)-alkyl)carbamoyl,
N-((C₆-C₁₆)-aryloxy-(C₁-C₁₀)-alkyl)carbamoyl,
N-((C₇-C₁₆)-aralkyloxy-(C₁-C₁₀)-alkyl)carbamoyl,
N-(C₁-C₁₀)-alkyl-N-((C₁-C₁₀)-alkoxy-(C₁-C₁₀)-alkyl)carbamoyl,
N-(C₁-C₁₀)-alkyl-N-((C₆-C₁₆)-aryloxy-(C₁-C₁₀)-alkyl)carbamoyl,
N-(C₁-C₁₀)-alkyl-N-((C₇-C₁₆)-aralkyloxy-(C₁-C₁₀)-alkyl)carbamoyl,
carbamoyloxy, N-(C₁-C₁₂)-alkylcarbamoyloxy,
N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy,
N-(C₁-C₁₀)-alkyl-N-(C₇-C₁₆)-aralkylcarbamoyloxy,
N-((C₁-C₁₀)-alkoxy-(C₁-C₁₀)-alkyl)carbamoyloxy,
N-((C₆-C₁₆)-aryloxy-(C₁-C₁₀)alkyl)carbamoyloxy,
N-((C₇-C₁₆)-aralkyloxy-(C₁-C₁₀)-alkyl)carbamoyloxy,
N-(C₁-C₁₀)-alkyl-N-((C₁-C₁₀)-alkoxy-(C₁-C₁₀)-alkyl)carbamoyloxy,
N-(C₁-C₁₀)-alkyl-N-((C₆-C₁₆)-aryloxy-(C₁-C₁₀)-alkyl)carbamoyloxy,
N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-(C₁-C₁₀)-alkyl-N-(C₇-C₁₆)-aralkylamino, N-(C₁-C₁₀)-alkyl-N-(C₆-C₁₂)-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₁-C₁₆)-aralkylsulfinyl, (C₇-C₁₆)-aralkylsulfonyl,
sulfamoyl, N-(C₁-C₁₀)-alkylsulfamoyl, N,N-di-(C₁-C₁₀)-alkylsulfamoyl, (C₃-C₈)-cycloalkylsulfamoyl,
N-(C₆-C₁₆)-arylsulfamoyl,
N-(C₇-C₁₆)-aralkylsulfamoyl,
N-(C₁-C₁₀)-alkyl-N-(C₆-C₁₆)-arylsulfamoyl,
N-(C₁-C₁₀)-alkyl-N-(C₇-C₁₆)-aralkylsulfamoyl,
(C₁-C₁₀)-alkyl-sulfonamido,
N-((C₁-C₁₀)-alkyl)-(C₁-C₁₀)-alkylsulfonamido, (C₇-C₁₆)-aralkylsulfonamido and N-((C₁-C₁₀)-alkyl-(C₇-C₁₆)-aralkylsulfonamido,
and
n is 0 or 1,
f is 1 to 8, preferably 1 to 5,
g is 0 or 1 to (2f + 1) and
x is 0 to 8, preferably 0 or 1, excluding
methyl 5-[((methylsulfonyl)amino)carbonyl]-pyridine-2-carboxylate,
methyl 5-[((2-propylsulfonyl)amino)carbonyl]-pyridine-2-carboxylate,
methyl 5-[((phenylsulfonyl)amino)carbonyl]-pyridine-2-carboxylate,
methyl 5-[((benzylsulfonyl)amino)carbonyl]-pyridine-2-carboxylate,
methyl 5-[((4-methoxyphenylsulfonyl)amino)carbonyl]-pyridine-2-carboxylate,
methyl 5-[((1-naphthylsulfonyl)amino)carbonyl]-pyridine-2-carboxylate,
methyl 5-[((4,5-dibromo-2-thienylsulfonyl)amino)carbonyl]-pyridine-2-carboxylate,
methyl 5-[((5-chloro-2-thienylsulfonyl)amino)carbonyl]-pyridine-2-carboxylate,
methyl 5-[((8-quinolylsulfonyl)amino)carbonyl]-pyridine-2-carboxylate,
methyl 5-[((4-(2-(4,7-dichloroquinolyl))phenylsulfonyl)amino)carbonyl]-pyridine-2-carboxylate.

2. A compound as claimed in claim 1, in which
X is a single bond or -CO-,
R¹, R² and R³ are identical or different and are hydrogen, (C₁-C₆)-alkyl, (C₁-C₆)-alkoxy, halogen, in particular fluorine or chlorine, hydroxyl or amino,
R⁴ is a radical of an alcohol R⁴OH, in which R⁴ is (C₁-C₁₀)-acyloxy-(C₁-C₆)-alkyl, preferably (C₁-C₁₀)-alkanoyloxy-(C₁-C₆)-alkyl, benzoyloxy-(C₁-C₆)-alkyl, benzyloxycarbonyloxy-(C₁-C₆)-alkyl or (C₁-C₆)-alkoxycarbonyloxy-(C₁-C₆)-alkyl,
a branched or unbranched aliphatic or cycloaliphatic (C₁-C₁₂)-alkyl radical,
a branched or unbranched (C₁-C₁₂)-alkenyl radical, a (C₂-C₁₂)-alkynyl radical or a (C₂-C₁₂)-alkenynyl radical, each of which can contain one or more multiple bonds, or
a (C₆-C₁₆)-aryl radical, a (C₆-C₁₆)-aralkyl radical or a heteroaryl or a heteroaralkyl radical,
it being possible for the above radicals to carry one or two substituents from the series comprising hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-aryloxy, (C₇-C₁₆)-aralkyloxy, (C₁-C₈)-hydroxyalkyl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₃-C₁₂)-alkenyloxycarbonyloxy, (C₃-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl,
N-((C₁-C₁₀)-alkoxy-(C₁-C₁₀)-alkyl)carbamoyl,
N-((C₆-C₁₆)-aryloxy-(C₁-C₁₀)-alkyl)carbamoyl,
N-((C₇-C₁₆)-aralkyloxy-(C₁-C₁₀)-alkyl)carbamoyl,
N-(C₁-C₁₀)-alkyl-N-((C₁-C₁₀)-alkoxy-(C₁-C₁₀)-alkyl)carbamoyl,
N-(C₁-C₁₀)-alkyl-N-((C₆-C₁₆)-aryloxy-(C₁-C₁₀)-alkyl)carbamoyl,
N-(C₁-C₁₀)-alkyl-N-((C₇-C₁₆)-aralkyloxy-(C₁-C₁₀)-alkyl)carbamoyl,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy,
N-((C₁-C₁₀)-alkoxy-(C₁-C₁₀)-alkyl)carbamoyloxy,
N-((C₆-C₁₆)-aryloxy-(C₁-C₁₀)-alkyl)carbamoyloxy,
N-((C₇-C₁₆)-aralkyloxy-(C₁-C₁₀)-alkyl)carbamoyloxy,
N-(C₁-C₁₀)-alkyl-N-((C₁-C₁₀)-alkoxy-(C₁-C₁₀)-alkyl)carbamoyloxy,
N-(C₁-C₁₀)-alkyl-N-((C₆-C₁₆)-aryloxy-(C₁-C₁₀)-alkyl)carbamoyloxy,
N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, N-(C₇-C₁₁)-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino and (C₇-C₁₁)-aralkanoyl-N-(C₁-C₁₀)-alkylamino, and
it being possible for the radicals which contain an aryl radical to be substituted in turn in the aryl part by 1 to 5 identical or different radicals from the series comprising hydroxyl, halogen, cyano, trifluoromethyl, (C₁-C₁₂)-alkyl, (C₃-C₈)-cycloalkyl, (C₁-C₁₂)-alkoxy, (C₁-C₁₂)-alkoxy-(C₁-C₁₂)-alkoxy,
(C₁-C₁₂)-alkylcarbonyl, (C₃-C₈)-cycloalkylcarbonyl,
(C₁-C₁₂)-alkoxycarbonyl, (C₁-C₁₂)-alkoxy-(C₁-C₁₂)-alkoxycarbonyl, (C₃-C₈)-cycloalkoxycarbonyl,
(C₁-C₁₂)-alkylcarbonyloxy, (C₃-C₈)-cycloalkylcarbonyloxy,
(C₁-C₁₂)-alkoxycarbonyloxy, (C₁-C₁₂)-alkoxy-(C₁-C₁₂)-alkoxycarbonyloxy, (C₃-C₈)-cycloalkoxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl,
N-((C₁-C₁₀)-alkoxy-(C₁-C₁₀)-alkyl)carbamoyl,
N-(C₁-C₁₀)-alkyl-N-((C₁-C₁₀)-alkoxy-(C₁-C₁₀)-alkyl)carbamoyl.
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy,
amino, (C₁-C₁₂)-alkylamino, di-(C₁-C₁₂)-alkylamino, (C₃-C₈)-cycloalkylamino,
(C₁-C₁₂)-alkanoylamino, (C₃-C₈)-cycloalkanoylamino,
(C₁-C₁₂)-alkanoylamino-(C₁-C₈)-alkyl, (C₃-C₈)-cycloalkanoylamino-(C₁-C₈)-alkyl,
(C₁-C₁₂)-alkylmercapto, (C₁-C₁₂)-alkylsulfinyl and (C₁-C₁₂)-alkylsulfonyl,
R⁵ is hydrogen, (C₁-C₃)-alkyl, (C₁-C₄)-alkanoyl or a mono-, di- or trivalent physiologically usable cation, in particular Na^{⊕}, K^{⊕}, Mg^{2⊕}, Ca^{2⊕} or an ammonium ion, optionally mono-, di- or trisubstituted by (C₁-C₈)-hydroxyalkyl, (C₁-C₄)-alkoxy-(C₁-C₈)-alkyl, phenyl, benzyl or (C₁-C₈)-alkyl, which can be mono-, di- or trisubstituted by hydroxyl and/or (C₁-C₄)-alkoxy, or a cation of a basic amino acid derivative,
R⁶ is a radical of the formula II, excluding -SO₂H
-Y-[C-U]ᵣ-D-W (II)
in which
Y is -SO₂-,
C is a bond or a branched or unbranched aliphatic (C₁-C₁₂)-alkanediyl radical or
a branched or unbranched (C₂-C₁₂)-alkenediyl radical, a (C₂-C₁₂)-alkynediyl radical or a (C₂-C₁₂)-alkenynediyl radical, which can contain one or more C-C multiple bonds,
U is a bond or
hydrogen or a radical from the following series of heteroatom groupings
-(CO)NR-, -NR(CO)-, -O-, -SO- or -SO₂-, in which R is (C₂-C₃)-alkyl or hydrogen,
r is 1 or 2,
D is a bond or
hydrogen or
a branched or unbranched aliphatic (C₁-C₈)-alkanediyl radical, or
a branched or unbranched (C₂-C₈)-alkenediyl radical or (C₂-C₈)-alkynediyl radical and
W is a bond or
hydrogen or
a (C₃-C₁₀) cycloaliphatic alkyl, alkenyl, alkynyl or alkenynyl radical or
a (C₆-C₁₆)-aryl radical or a 5- or 6-membered heteroaryl radical, at least one of the variables C or D or W not being a bond and U only being a heteroatom grouping if C is not a bond or if D and/or W are not a bond and
C, D and/or W, if these are not a bond or hydrogen, are preferably substituted in turn by a combination of up to 5 identical or different substituents from the series comprising
hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl,
N-((C₁-C₁₀)-alkoxy-(C₁-C₁₀)-alkyl)carbamoyl,
N-((C₆-C₁₆)-aryloxy-(C₁-C₁₀)-alkyl)carbamoyl,
N-((C₇-C₁₆)-aralkyloxy-(C₁-C₁₀)-alkyl)carbamoyl,
N-(C₁-C₁₀)-alkyl-N-((C₁-C₁₀)-alkoxy-(C₁-C₁₀)-alkyl)carbamoyl,
N-(C₁-C₁₀)-alkyl-N-((C₆-C₁₆)-aryloxy-(C₁-C₁₀)-alkyl)carbamoyl,
N-(C₁-C₁₀)-alkyl-N-((C₇-C₁₆)-aralkyloxy-(C₁-C₁₀)-alkyl)carbamoyl,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-(C₁-C₅)-alkyl-N-(C₇-C₁₀)-aralkylamino, N-(C₁-C₅)-alkyl-N-(C₆-C₁₂)-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl and (C₇-C₁₆)-aralkylsulfonyl,
it being possible for the radicals which contain an aryl radical to be substituted in turn on the aryl by 1, 2, 3, 4 or 5 identical or different substituents from the series comprising
hydrogen, hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl,
N-((C₁-C₁₀)-alkoxy-(C₁-C₁₀)-alkyl)carbamoyl,
N-((C₆-C₁₆)-aryloxy-(C₁-C₁₀)-alkyl)carbamoyl,
N-((C₇-C₁₆)-aralkyloxy-(C₁-C₁₀)-alkyl)carbamoyl,
N-(C₁-C₁₀)-alkyl-N-((C₁-C₁₀)-alkoxy-(C₁-C₁₀)-alkyl)carbamoyl,
N-(C₁-C₁₀)-alkyl-N-((C₆-C₁₆)-aryloxy-(C₁-C₁₀)-alkyl)carbamoyl,
N-(C₁-C₁₀)-alkyl-N-((C₇-C₁₆)-aralkyloxy-(C₁-C₁₀)-alkyl)carbamoyl,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-(C₁-C₅)-alkyl-(C₇-C₁₀)-aralkylamino, N-(C₁-C₅)-alkyl-(C₆-C₁₂)-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoyl-amino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl and (C₇-C₁₆)-aralkylsulfonyl, excluding the compounds: methyl 5-[((methylsulfonyl)amino)carbonyl]-pyridine-2-carboxylate,
methyl 5-[((2-propylsulfonyl)amino)carbonyl]-pyridine-2-carboxylate,
methyl 5-[((phenylsulfonyl)amino)carbonyl]-pyridine-2-carboxylate,
methyl 5-[((benzylsulfonyl)amino)carbonyl]-pyridine-2-carboxylate,
methyl 5-[((4-methoxyphenylsulfonyl)amino)carbonyl]-pyridine-2-carboxylate,
methyl 5-[((1-naphthylsulfonyl)amino)carbonyl]-pyridine-2-carboxylate,
methyl 5-[((4,5-dibromo-2-thienylsulfonyl)amino)carbonyl]-pyridine-2-carboxylate,
methyl 5-[((5-chloro-2-thienylsulfonyl)amino)carbonyl]-pyridine-2-carboxylate,
methyl 5-[((8-quinolylsulfonyl)amino)carbonyl]-pyridine-2-carboxylate and
methyl 5-[((4-(2-(4,7-dichloroquinolyl))phenylsulfonyl)amino)carbonyl]-pyridine-2-carboxylate.

3. A compound as claimed in claim 1 or 2, in which
X is a single bond or -CO-,
R¹, R² and R³ are identical or different and are hydrogen or (C₁-C₃)-alkyl, (C₁-C₃)-alkoxy, hydroxyl, fluorine or chlorine,
R⁴ is the radical of an alcohol R⁴OH, in which R⁴ is (C₁-C₁₀)-acyloxy-(C₁-C₆)-alkyl, preferably (C₁-C₁₀)-alkanoyloxy-(C₁-C₆)-alkyl, benzoyloxy-(C₁-C₆)-alkyl, benzyloxycarbonyloxy-(C₁-C₆)-alkyl or
(C₁-C₆)-alkoxycarbonyloxy-(C₁-C₆)-alkyl,
a branched or unbranched or cyclic aliphatic (C₁-C₁₀)-alkyl radical, or
a branched or unbranched (C₂-C₁₀)-alkenyl radical or a (C₂-C₁₂)-alkynyl radical, each of which can contain one or more C-C multiple bonds, or
a (C₆-C₁₆)-aryl radical, a (C₇-C₁₁)-aralkyl radical or a heteroaryl or heteroalkyl radical,
it being possible for the above radicals to carry one or two substituents from the series comprising
hydroxyl, (C₁-C₆)-alkoxy, (C₁-C₆)-alkoxy-(C₁-C₆)-alkoxy, (C₆-C₁₂)-aryloxy, (C₇-C₁₂)-aralkyloxy,
(C₁-C₈)-alkylcarbonyl, (C₃-C₈)-cycloalkylcarbonyl, (C₆-C₁₂)-arylcarbonyl, (C₇-C₁₂)-aralkylcarbonyl,
(C₁-C₈)-alkoxycarbonyl, (C₁-C₆)-alkoxy-(C₁-C₆)-alkoxycarbonyl, (C₆-C₁₂)-aryloxycarbonyl, (C₇-C₁₂)-aralkoxycarbonyl, (C₃-C₈)-cycloalkoxycarbonyl,
(C₁-C₈)-alkylcarbonyloxy, (C₃-C₈)-cycloalkylcarbonyloxy, (C₆-C₁₂)-arylcarbonyloxy, (C₇-C₁₂)-aralkylcarbonyloxy,
(C₁-C₈)-alkoxycarbonyloxy, (C₁-C₆)-alkoxy-(C₁-C₆)-alkoxycarbonyloxy, (C₆-C₁₂)-aryloxycarbonyloxy, (C₇-C₁₂)-aralkyloxycarbonyloxy, (C₃-C₈)-cycloalkoxycarbonyloxy,
carbamoyl, N-(C₁-C₈)-alkylcarbamoyl, N,N-di-(C₁-C₈)-alkylcarbamoyl, N-(C₃-C₈)-cycloalkylcarbamoyl,
N-((C₁-C₆)-alkoxy-(C₁-C₆)-alkyl)carbamoyl,
amino, (C₁-C₆)-alkylamino, di-(C₁-C₆)-alkylamino, (C₃-C₈)-cycloalkylamino, N-(C₆-C₁₂)-arylamino, N-(C₇-C₁₁)-aralkylamino, N-(C₁-C₅)-alkyl-(C₆-C₁₂)-arylamino,
(C₁-C₈)-alkanoylamino, (C₃-C₈)-cycloalkanoylamino, (C₆-C₁₂)-aroylamino, (C₇-C₁₂)-aralkanoylamino, (C₁-C₈)-alkanoyl-N-(C₁-C₆)-alkylamino, (C₃-C₈)-cycloalkanoyl-N-(C₁-C₆)-alkylamino, (C₆-C₁₂)-aroyl-N-(C₁-C₆)-alkylamino and (C₇-C₁₁)-aralkanoyl-N-(C₁-C₆)-alkylamino, and
the radicals which contain an aryl radical being substituted, in particular, by up to 3 substituents from the series comprising hydroxyl, fluorine, chlorine, cyano, trifluoromethyl, (C₁-C₆)-alkyl, (C₃-C₈)-cycloalkyl, (C₁-C₈)-alkoxy,
(C₁-C₆)-alkylcarbonyl, (C₃-C₈)-cycloalkylcarbonyl,
(C₁-C₆)-alkoxycarbonyl, (C₃-C₈)-cycloalkoxycarbonyl,
(C₁-C₆)-alkylcarbonyloxy, (C₃-C₈)-cycloalkylcarbonyloxy,
(C₁-C₆)-alkoxycarbonyloxy, (C₃-C₈)-cycloalkoxycarbamoyloxy,
carbamoyl, N-(C₁-C₆)-alkylcarbamoyl, N,N-di-(C₁-C₆)-alkylcarbamoyl, N-(C₃-C₈)-cycloalkylcarbamoyl,
N-((C₁-C₆)-alkoxy-(C₁-C₆)-alkyl)carbamoyl,
N-(C₁-C₆)-alkyl-N-((C₁-C₆)-alkoxy-(C₁-C₆)-alkyl)carbamoyl,
carbamoyloxy, N-(C₁-C₆)-alkylcarbamoyloxy, N,N-di-(C₁-C₆)-alkylcarbamoyloxy, N-(C₃-C₈)-cycloalkylcarbamoyloxy,
amino, (C₁-C₆)-alkylamino, di-(C₁-C₆)-alkylamino, (C₃-C₈)-cycloalkylamino,
(C₁-C₆)-alkanoylamino, (C₃-C₈)-cycloalkanoylamino,
(C₁-C₆)-alkylmercapto, (C₁-C₆)-alkylsulfinyl and (C₁-C₆)-alkylsulfonyl, and
R⁵ is hydrogen, (C₁-C₃)-alkyl, (C₁-C₄)-alkanoyl or a mono-, di- or trivalent physiologically usable cation, in particular Na^{⊕}, K^{⊕}, Mg^{2⊕}, Ca^{2⊕} or an ammonium ion, optionally mono-, di- or trisubstituted by
(C₁-C₈)-hydroxyalkyl, (C₁-C₄)-alkoxy-(C₁-C₈)-alkyl, phenyl, benzyl or (C₁-C₈)-alkyl, which can be mono-, di- or trisubstituted by hydroxyl and/or (C₁-C₄)-alkoxy,
R⁶ is a radical of the formula II, excluding -SO₂H
-Y-[C-U]ᵣ-D-W (II)
in which
Y is -SO₂-,
C is a bond or
a (C₁-C₁₆)-alkanediyl radical,
U is a bond or
hydrogen or -O-,
r is 1,
D is a bond or
hydrogen or
an unbranched aliphatic (C₁-C₈)-alkanediyl radical, and
W is a bond or
hydrogen, a (C₆-C₁₂)-aryl radical or a 5- or 6-membered heteroaryl radical, at least one of the variables C or D or W not being a bond and U only being a heteroatom grouping if C is not a bond or if D and/or W are not a bond and
C, D and/or W, if these are not a bond or hydrogen, are preferably substituted in turn by up to 3 identical or different substituents from the series comprising hydroxyl, halogen, cyano, trifluoromethyl, nitro, carboxyl, (C₁-C₈)-alkyl, (C₃-C₈)-cycloalkyl, (C₆-C₁₂)-aryl, (C₇-C₁₆)-aralkyl, (C₃-C₈)-alkenyl, (C₃-C₈)-alkynyl, (C₁-C₈)-alkoxy, (C₁-C₈)-alkoxy-(C₁-C₈)-alkyl, (C₆-C₁₂)-aryloxy, (C₇-C₁₆)-aralkyloxy, (C₁-C₈)-hydroxyalkyl, -O-[CH₂]ₓC_{f}H_{(2f+1-g)}F_{g},
(C₁-C₈)-alkoxycarbonyl, (C₆-C₁₂)-aryloxycarbonyl, (C₇-C₁₄)-aralkoxycarbonyl, (C₃-C₈)-cycloalkoxycarbonyl,
(C₁-C₈)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl,
N-((C₁-C₁₀)-alkoxy-(C₁-C₁₀)-alkyl)carbamoyl,
N-((C₆-C₁₆)-aryloxy-(C₁-C₁₀)-alkyl)carbamoyl,
N-((C₇-C₁₆)-aralkyloxy-(C₁-C₁₀)-alkyl)carbamoyl,
N-(C₁-C₁₀)-alkyl-N-((C₁-C₁₀)-alkoxy-(C₁-C₁₀)-alkyl)carbamoyl,
N-(C₁-C₁₀)-alkyl-N-((C₆-C₁₆)-aryloxy-(C₁-C₁₀)-alkyl)carbamoyl,
N-(C₁-C₁₀)-alkyl-N-((C₇-C₁₆)-aralkyloxy-(C₁-C₁₀)-alkyl)carbamoyl,
amino, (C₁-C₈)-alkylamino, di-(C₁-C₈)-alkylamino, (C₃-C₈)-cycloalkylamino, N-(C₆-C₁₂)-arylamino, N-(C₇-C₁₁)-aralkylamino, N-(C₁-C₃)-alkyl-N-(C₇-C₁₁)-aralkylamino,
(C₁-C₁₀)-alkanoylamino, (C₃-C₈)-cycloalkanoylamino, (C₆-C₁₂)-aroylamino, (C₇-C₁₆)-aralkanoylamino, (C₁-C₁₀)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₆)-alkyl, (C₃-C₈)-cycloalkanoylamino-(C₁-C₈)-alkyl, (C₆-C₁₆)-aroylamino-(C₁-C₆)-alkyl, (C₇-C₁₆)-aralkanoylamino-(C₁-C₆)-alkyl,
(C₁-C₈)-alkylmercapto, (C₁-C₈)-alkylsulfinyl, (C₁-C₈)-alkylsulfonyl,
(C₆-C₁₂)-arylmercapto, (C₆-C₁₂)-arylsulfinyl, (C₆-C₁₂)-arylsulfonyl,
(C₇-C₁₄)-aralkylmercapto, (C₇-C₁₄)-aralkylsulfinyl and (C₇-C₁₄)-aralkylsulfonyl,
it being possible for the radicals which contain an aryl radical to be substituted in turn on the aryl by 1, 2, 3, 4 or 5 identical or different substituents from the series comprising
hydrogen, hydroxyl, halogen, cyano, trifluoromethyl, nitro, carboxyl, (C₁-C₈)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₈)-alkoxy, (C₁-C₆)-alkoxy-(C₁-C₆)-alkyl, (C₆-C₁₂)-aryloxy, (C₇-C₁₆)-aralkyloxy, -O-[CH₂]ₓC_{f}H_{(2f+1-g)}F_{g},
(C₁-C₁₂)-alkoxycarbonyl, (C₃-C₈)-cycloalkoxycarbonyl,
(C₁-C₁₂)-alkylcarbonyloxy, (C₃-C₈)-cycloalkylcarbonyloxy,
carbamoyl, N-(C₁-C₈)-alkylcarbamoyl, N,N-di-(C₁-C₈)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₈)-alkyl-N-(C₆-C₁₆)-arylcarbamoyl, N-(C₁-C₈)-alkyl-N-(C₇-C₁₆)-aralkylcarbamoyl,
N-((C₁-C₆)-alkoxy-(C₁-C₆)-alkyl)carbamoyl,
N-((C₆-C₆)-aryloxy-(C₁-C₆)-alkyl)carbamoyl,
N-((C₇-C₁₆)-aralkyloxy-(C₁-C₆)-alkyl)carbamoyl,
N-(C₁-C₈)-alkyl-N-((C₁-C₆)-alkoxy-(C₁-C₆)-alkyl)carbamoyl,
N-(C₁-C₈)-alkyl-N-((C₆-C₁₆)-aryloxy-(C₁-C₆)-alkyl)carbamoyl,
N-(C₁-C₈)-alkyl-N-((C₇-C₁₆)-aralkyloxy-(C₁-C₆)-alkyl)carbamoyl,
amino, (C₁-C₆)-alkylamino, di-(C₁-C₆)-alkylamino, (C₃-C₈)-cycloalkylamino, N-(C₆-C₁₂)-arylamino, N-(C₇-C₁₁)-aralkylamino, N-(C₁-C₃)-alkyl-(C₇-C₁₁)-aralkylamino, N-(C₁-C₃)-alkyl-(C₆-C₁₂)-arylamino, (C₁-C₈)-alkoxy-amino,
(C₁-C₈)-alkanoylamino, (C₃-C₈)-cycloalkanoylamino, (C₆-C₁₂)-aroylamino, (C₇-C₁₂)-aralkanoylamino, (C₁-C₈)-alkanoyl-N-(C₁-C₆)-alkylamino, (C₃-C₈)-cycloalkanoyl-N-(C₁-C₆)-alkylamino, (C₆-C₁₂)-aroyl-N-(C₁-C₁₀)-alkylamino, (C₇-C₁₁)-aralkanoyl-N-(C₁-C₆)-alkylamino,
(C₁-C₈)-alkanoylamino-(C₁-C₄)-alkyl, (C₃-C₈)-cycloalkanoylamino-(C₁-C₄)-alkyl, (C₆-C₁₂)-aroylamino-(C₁-C₄)-alkyl and (C₇-C₁₂)-aralkanoylamino-(C₁-C₄)-alkyl,
excluding the compounds:
methyl 5-[((methylsulfonyl)amino)carbonyl]-pyridine-2-carboxylate,
methyl 5-[((2-propylsulfonyl)amino)carbonyl]-pyridine-2-carboxylate,
methyl 5-[((phenylsulfonyl)amino)carbonyl]-pyridine-2-carboxylate,
methyl 5-[((benzylsulfonyl)amino)carbonyl]-pyridine-2-carboxylate,
methyl 5-[((4-methoxyphenylsulfonyl)amino)carbonyl]-pyridine-2-carboxylate,
methyl 5-[((1-naphthylsulfonyl)amino)carbonyl]-pyridine-2-carboxylate,
methyl 5-[((4,5-dibromo-2-thienylsulfonyl)amino)carbonyl]-pyridine-2-carboxylate,
methyl 5-[((5-chloro-2-thienylsulfonyl)amino)carbonyl]-pyridine-2-carboxylate,
methyl 5-[((8-quinolylsulfonyl)amino)carbonyl]-pyridine-2-carboxylate,
methyl 5-[((4-(2-(4,7-dichloroquinolyl))phenylsulfonyl)amino)carbonyl]-pyridine-2-carboxylate.

4. A compound as claimed in one or more of claims 1 to 3, in which
X is a single bond or -CO-,
R¹, R² and R³ are hydrogen,
R⁴ is the radical of an alcohol R⁴OH and is a branched or unbranched or cyclic aliphatic (C₁-C₉)-alkyl radical, or
a branched or unbranched (C₁-C₈)-alkenyl radical or (C₁-C₈)-alkynyl radical, or a phenyl, benzyl, phenethyl or phenylpropyl radical,
the above radicals containing a substituent from the series comprising hydrogen, hydroxyl, (C₁-C₄)-alkoxy,
(C₁-C₆)-alkoxycarbonyl, (C₆-C₁₂)-phenoxycarbonyl, (C₇-C₁₆)-phenylalkylcarbonyl, (C₃-C₈)-cycloalkoxycarbonyl,
(C₁-C₆)-alkylcarbonyloxy, (C₃-C₈)-cycloalkylcarbonyloxy, benzoyloxy, (C₇-C₁₆)-phenylalkylcarbonyloxy and (C₃-C₈)-cycloalkoxycarbonyloxy, or
(C₁-C₆)-alkoxycarbonyloxy, phenoxycarbonyloxy, (C₇-C₁₆)-phenylalkylcarbonyloxy or (C₃-C₈)-cycloalkoxycarbonyloxy,
R⁵ is hydrogen or a mono-, di- or trivalent physiologically usable cation, in particular Na^{⊕}, K^{⊕}, Mg^{2⊕}, Ca^{2⊕} or H₃N^{⊕}C(CH₂OH)₃ (tris salt),
R⁶ is a radical of the formula II, excluding -SO₂H
-Y-[C-U]ᵣ-D-W (II)
in which
Y is -SO₂-,
C is a bond or
(C₁-C₄)-alkanediyl,
U is a bond, hydrogen or
-O-,
r is 1,
D is a bond, hydrogen or
(C₁-C₄)-alkanediyl,
W is a bond, hydrogen or
a phenyl radical, at least one of the variables C or D or W not being a bond and U only being a heteroatom grouping if C is not a bond or if D and/or W are not a bond, and
C, D and/or W are substituted by hydrogen or by 1 or 2 substituents from the following series fluorine, chlorine, (C₁-C₆)-alkyl, phenyl, (C₁-C₆)-alkoxy, phenoxy, -O-[CH₂]ₓC_{f}H_{(2f+1-g)}F_{g},
carbamoyl, N-(C₁-C₁₀)-alkylcarbamoyl, N,N-di-(C₁-C₈)-alkylcarbamoyl, N-(C₃-C₈)-cycloalkylcarbamoyl, N-phenylcarbamoyl, N-(C₇-C₁₆)-phenylalkylcarbamoyl, N-(C₁-C₈)-alkyl-N-(C₆-C₁₆)-phenylcarbamoyl, N-(C₁-C₈)-alkyl-N-(C₇-C₁₆)-phenylalkylcarbamoyl,
N-((C₁-C₄)-alkoxy-(C₁-C₈)-alkyl)carbamoyl,
N-phenoxy-(C₁-C₈)-alkyl)carbamoyl,
N-((C₇-C₁₆)-phenylalkyloxy-(C₁-C₈)-alkyl)carbamoyl,
N-(C₁-C₈)-alkyl-N-((C₁-C₆)-alkoxy-(C₁-C₈)-alkyl)carbamoyl,
N-(C₁-C₈)-alkyl-N-((C₆-C₁₂)-phenoxy-(C₁-C₈)-alkyl)-carbamoyl,
N-(C₁-C₈)-alkyl-N-((C₇-C₁₆)-phenylalkyloxy-(C₁-C₈)-alkyl)carbamoyl,
(C₁-C₈)-alkanoylamino, (C₃-C₈)-cycloalkanoylamino, (C₆-C₁₂)-phenylamino, (C₇-C₁₁)-phenylalkanoylamino, (C₁-C₈)-alkanoyl-N-(C₁-C₁₀)-alkylamino, (C₃-C₈)-cycloalkanoyl-N-(C₁-C₆)-alkylamino, benzoyl-N-(C₁-C₁₀)-alkylamino, (C₇-C₁₁)-phenylalkanoyl-N-(C₁-C₆)-alkylamino,
(C₁-C₁₀)-alkanoylamino-(C₁-C₂)-alkyl, (C₃-C₈)-cycloalkanoylamino-(C₁-C₂)-alkyl, benzoylamino-(C₁-C₂)-alkyl and (C₇-C₁₄)-phenylalkanoylamino- (C₁-C₂)-alkyl, the radicals which contain an aryl radical being substituted in turn by a substituent from the series comprising
hydrogen, hydroxyl, fluorine, chlorine, trifluoromethyl, (C₁-C₆)-alkyl and (C₁-C₈)-alkoxy,
n is 0,
f is 1 to 5,
g is 0 or 1 to (2f + 1) and
x is 0 or 1,
excluding the compounds
methyl 5-[((methylsulfonyl)amino)carbonyl]-pyridine-2-carboxylate,
methyl 5-[((2-propylsulfonyl)amino)carbonyl]-pyridine-2-carboxylate,
methyl 5-[((phenylsulfonyl)amino)carbonyl]-pyridine-2-carboxylate,
methyl 5-[((benzylsulfonyl)amino)carbonyl]-pyridine-2-carboxylate,
methyl 5-[((4-methoxyphenylsulfonyl)amino)carbonyl]-pyridine-2-carboxylate,
methyl 5-[((1-naphthylsulfonyl)amino)carbonyl]-pyridine-2-carboxylate,
methyl 5-[((4,5-dibromo-2-thienylsulfonyl)amino)carbonyl]-pyridine-2-carboxylate,
methyl 5-[((5-chloro-2-thienylsulfonyl)amino)carbonyl]-pyridine-2-carboxylate,
methyl 5-[((8-quinolylsulfonyl)amino)carbonyl]-pyridine-2-carboxylate and
methyl 5-[((4-(2-(4,7-dichloroquinolyl))phenylsulfonyl)amino)carbonyl]-pyridine-2-carboxylate.

5. A compound as claimed in one or more of claims 1 to 4 for use as a medicament.

6. A process for the preparation of a compound as claimed in one or more of claims 1 to 5, in which X is a single bond, which comprises
i) reacting a compound of the formula 1 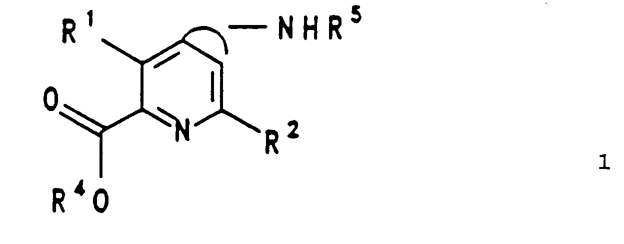 with ZR⁶ to give a compound of the formula 3 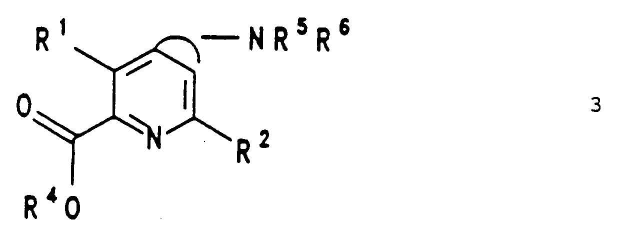 or
ii) reacting a compound of the formula 5 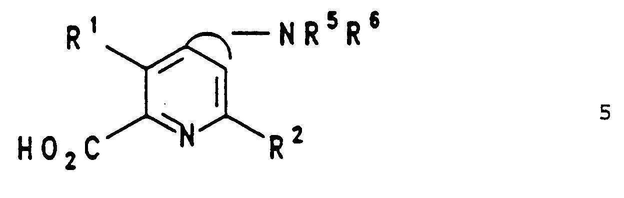 with R⁴OH to give a compound of the formula 3, in which R¹, R², R³, R⁴, R⁵ and R⁶ are defined in claims 1 to 4 and Z is hydroxyl or a leaving group which can be detached nucleophilically, and in particular is F, Cl, Br, I or tosylate, and, if appropriate, subsequently oxidizing the compounds of the formula 3 to give their pyridine N-oxides.

7. A process for the preparation of a compound as claimed in one or more of claims 1 to 5, in which X is -CO-, which comprises
i) reacting a compound of the formula 6 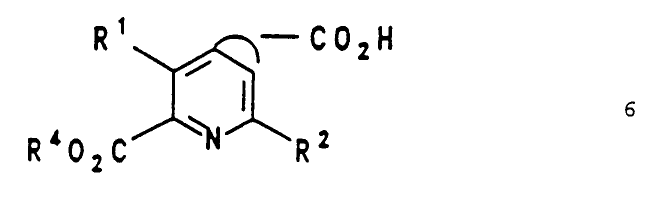 with NHR⁵R⁶ to give a compound of the formula 8 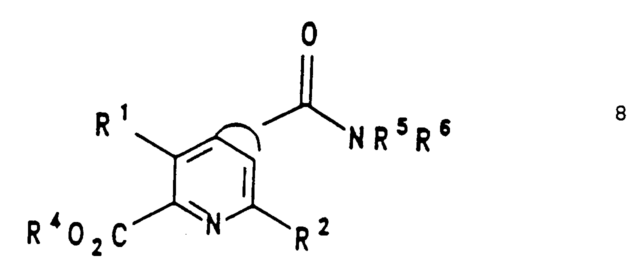 or
ii) reacting a compound of the formula 9 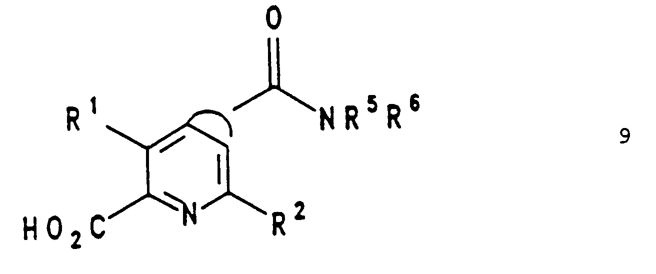 with R⁴OH to give a compound of the formula 8 or
iii) reacting a compound of the formula 10 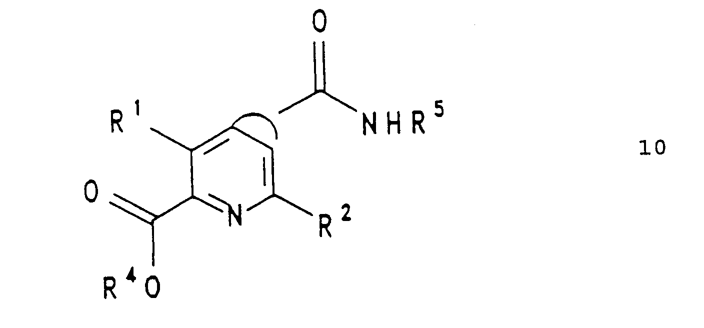 with ZR⁶ to give a compound of the formula 8, in which R¹, R², R³, R⁴, R⁵ and R⁶ are defined in claims 1 to 4 and Z is hydroxyl or a leaving group which can be detached nucleophilically, and in particular is F, Cl, Br, I or tosylate, and, if appropriate, subsequently oxidizing the compounds of the formula 8 to their pyridine N-oxides.

8. A compound as claimed in claims 1 to 4 against fibrotic diseases.

9. A compound as claimed in claims 1 to 4 for use as a fibrosuppressant.

10. A medicament comprising at least one compound of the formula I and if appropriate a tolerated pharmaceutical excipient.

11. A compound of the formula I for use in a process for the treatment of disturbances in the metabolism of collagen and collagen-like substances.

12. A compound of the formula I for use in a process for the treatment of fibrotic diseases.

13. A process for the preparation of a medicament for the treatment of fibrotic diseases, wherein the medicament comprises a compound of the formula I.

## Revendications

1. Esters d'acides acylsulfonamido- et sulfonamidopyridine-2-carboxyliques et leurs pyridine-N-oxydes de formule générale I 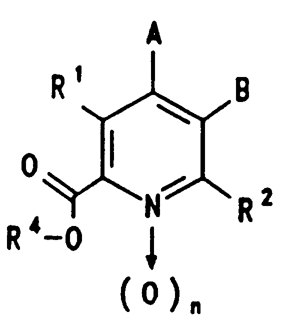 dans laquelle
A = R³ et B = X-NR⁵R⁶ ou
B = R³ et A = X-NR⁵R⁶ et
X représente une liaison simple ou -CO-, et
R¹, R² et R³ sont identiques ou différents et représentent un atome d'hydrogène ou d'halogène, en particulier de fluor, de chlore ou de brome, ou un groupe alkyle en C₁-C₆, alcoxy en C₁-C₆, nitrile, hydroxy, amino éventuellement mono- ou disubstitué par un ou des radicaux alkyle en C₁-C₄, hydroxyalkyle en C₁-C₄, alkyl(C₁-C₆)carbonyloxy,
R⁴ représente le reste d'un alcool R⁴OH, dans lequel R⁴ représente en particulier un groupe acyloxy(C₁-C₁₀)-alkyle(C₁-C₆), de préférence un groupe alcanoyloxy(C₁-C₁₀)-alkyle(C₁-C₆), benzyloxy-alkyle(C₁-C₆), benzyloxycarbonyloxy-alkyle(C₁-C₆) ou alcoxycarbonyloxyalkyle(C₁-C₆), un radical alkyle en C₁-C₁₆ ramifié ou non ramifié ou cyclique aliphatique, ou un radical alcényle en C₂-C₁₆ ramifié ou non ramifié, éventuellement cyclique, un radical alcynyle en C₂-C₁₆ ou un radical alcénynyle en C₂-C₁₆, qui peuvent comporter chacun une ou plusieurs liaisons multiples,
un radical aryle en C₆-C₁₆, un radical aralkyle en C₇-C₁₆ ou un radical hétéroaryle à 5 ou 6 chaînons, de préférence azoté, ou un radical hétéroaralkyle à 5 ou 6 chaînons, de préférence azoté, les radicaux précédents portant en particulier un ou plusieurs substituants choisis parmi
des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)-alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl,
alkyl(C₁-C₁₂)carbonyle, cycloalkyl(C₃-C₈)carbonyle, aryl(C₆-C₁₂)carbonyle, aralkyl(C₇-C₁₆)carbonyle, cinnamoyle, alcényl(C₃-C₁₂)carbonyle, alcynyl(C₃-C₁₂)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₃-C₁₂)carbonyle, alcynyloxy(C₃-C₁₂)carbonyle,
alkyl(C₁-C₁₂)carbonyloxy, cycloalkyl(C₃-C₈)carbonyloxy, aryl(C₆-C₁₂)carbonyloxy, aralkyl(C₇-C₁₆)carbonyloxy, cinnamoyloxy, alcényl(C₃-C₁₂)carbonyloxy, alcynyl(C₃-C₁₂)carbonyloxy,
alcoxy(C₁-C₁₂)carbonyloxy, alcoxy(C₁-C₁₂)alcoxy(C₁-C₁₂)carbonyloxy, aryloxy(C₆-C₁₂)carbonyloxy, aralkyloxy(C₇-C₁₆)carbonyloxy, cycloalcoxy(C₃-C₈)carbonyloxy, alcényloxy(C₃-C₁₂)carbonyloxy, alcynyloxy(C₃-C₁₂)carbonyloxy,
carbamoyle, N-alkyl(C₁-C₁₂)carbamoyle, N,N-dialkyl(C₁-C₁₂)carbamoyle, N-cycloalkyl(C₃-C₈)carbamoyle, N-aryl(C₆-C₁₆)carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)alkyl(C₁-C₁₀)]carbamoyle, N-alkyl(C₁-C₁₀)-N-[aryloxy(C₆-C₁₆)-alkyl(C₁-C₁₀)]carbamoyle, N-alkyl(C₁-C₁₀)-N-[aralkyloxy(C₇-C₁₆)-alkyl(C₁-C₁₀)]carbamoyle,
carbamoyloxy, N-alkyl(C₁-C₁₂)carbamoyloxy, N,N-di-alkyl(C₁-C₁₂)carbamoyloxy, N-cycloalkyl(C₃-C₈)carbamoyloxy, N-aryl(C₆-C₁₆)carbamoyloxy, N-aralkyl(C₇-C₁₆)carbamoyloxy, N-alkyl(C₁-C₁₀)-N-aryl(C₆-C₁₂)carbamoyloxy, N-alkyl(C₁-C₁₀)-N-aralkyl(C₇-C₁₆)carbamoyloxy, N-[alcoxy(C₁-C₁₀)-alkyl(C₁-C₁₀)]carbamoyloxy, N-[aryloxy(C₆-C₁₆)alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₆)-alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
sulfamoyle, N-alkyl(C₁-C₁₀)sulfamoyle, N,N-dialkyl(C₁-C₁₀)sulfamoyle, cycloalkyl(C₃-C₈)sulfamoyle, N-aryl(C₆-C₁₆)sulfamoyle, N-aralkyl(C₇-C₁₆)sulfamoyle, N-alkyl(C₁-C₁₀)-N-aryl(C₆-C₁₆)sulfamoyle, N-alkyl(C₁-C₁₀)-N-aralkyl(C₇-C₁₆)sulfamoyle,
alkyl(C₁-C₁₀)sulfonamido, N-[alkyl(C₁-C₁₀)]-alkyl(C₁-C₁₀)sulfonamido, aralkyl(C₇-C₁₆)sulfonamido, N-[alkyl(C₁-C₁₀)]-aralkyl(C₇-C₁₆) sulfonamido, les radicaux qui contiennent un reste aryle pouvant pour leur part être substitués sur le fragment aryle par 1 à 5 groupes identiques ou différents, choisis parmi
des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcoxy en C₁-C₁₂, alcoxy(C₁-C₁₂)-alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈,
alkyl(C₁-C₁₂)carbonyle, cycloalkyl(C₃-C₈)carbonyle, aryl(C₆-C₁₂)carbonyle, aralkyl(C₇-C₁₆)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₃-C₁₂)carbonyle, alcynyloxy(C₃-C₁₂)carbonyle,
alkyl(C₁-C₁₂)carbonyloxy, cycloalkyl(C₃-C₈)carbonyloxy, aryl(C₆-C₁₂)carbonyloxy, aralkyl(C₇-C₁₆)carbonyloxy, cinnamoyloxy, alcényl(C₃-C₁₂)carbonyloxy, alcynyl(C₃-C₁₂)carbonyloxy,
alcoxy(C₁-C₁₂)carbonyloxy, alcoxy(C₁-C₁₂)alcoxy(C₁-C₁₂)carbonyloxy, aryloxy(C₆-C₁₂)carbonyloxy, aralkyloxy(C₇-C₁₆)carbonyloxy, cycloalcoxy(C₃-C₈)carbonyloxy, alcényloxy(C₃-C₁₂)carbonyloxy, alcynyloxy(C₃-C₁₂)carbonyloxy,
carbamoyle, N-alkyl(C₁-C₁₂)carbamoyle, N,N-dialkyl(C₁-C₁₂)carbamoyle, N-cycloalkyl(C₃-C₈)carbamoyle, N-aryl(C₆-C₁₆)carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₆)-alkyl(C₁-C₁₀)]carbamoyle, N-alkyl(C₁-C₁₀)-N-[aralkyloxy(C₇-C₁₆)-alkyl(C₁-C₁₀)]carbamoyle,
carbamoyloxy, N-alkyl(C₁-C₁₂)carbamoyloxy, N,N-dialkyl(C₁-C₁₂)carbamoyloxy, N-cycloalkyl(C₃-C₈)carbamoyloxy, N-aryl(C₆-C₁₆)carbamoyloxy, N-aralkyl(C₇-C₁₆)carbamoyloxy, N-alkyl(C₁-C₁₀)-N-aryl(C₆-C₁₂)carbamoyloxy, N-alkyl(C₁-C₁₀)-N-aralkyl(C₇-C₁₆)carbamoyloxy, N-[alcoxy(C₁-C₁₀)-alkyl(C₁-C₁₀)]carbamoyloxy, N-[aryloxy(C₆-C₁₆)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₆)-alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle et aralkyl(C₇-C₁₆)sulfonyle,
R⁵ représente un atome d'hydrogène ou un groupe alkyle en C₁-C₆ ou un groupe protecteur de N, tel qu'un groupe alcanoyle en C₁-C₈, alkyl(C₁-C₆)carbamoyle, alcoxy(C₁-C₆)carbonyle, benzyloxycarbonyle, acyloxy(C₁-C₁₀)alkyle(C₁-C₆), de préférence un groupe alcanoyloxy(C₁-C₁₀)-alkyle(C₁-C₆), benzoyloxy-alkyle(C₁-C₆), benzyloxycarbonyloxy-alkyle(C₁-C₆) ou alcoxy(C₁-C₆)carbonyloxy-alkyle(C₁-C₆), un cation mono-, di-, tri- ou tétravalent, physiologiquement utilisable, en particulier Na^{⊕}, K^{⊕}, Mg^{2⊕}, Ca^{2⊕}, Al^{3⊕} ou un ion ammonium éventuellement 1 à 3 fois substitué par un ou des groupes hydroxyalkyle en C₁-C₈, alcoxy(C₁-C₄)-alkyle(C₁-C₈), phényle, benzyle ou alkyle en C₁-C₈, qui peut être 1 à 3 fois substitué par un ou des groupes hydroxy ou alcoxy en C₁-C₄, ou un cation d'un dérivé d'aminoacide basique,
R⁶ représente un radical de formule II, à l'exception de -SO₂H,
-Y-(C-U)ᵣ-D-W (II)
dans laquelle
Y représente -SO₂- ou -CO-,
C représente une liaison ou un radical alcanediyle en C₁-C₁₆ aliphatique, ramifié ou non ramifié, ou un radical alcanediyle en C₃-C₁₀ cycloaliphatique, ou un radical alcènediyle en C₂-C₁₆ ramifié ou non ramifié ou cycloalcènediyle, ou un radical alcynediyle en C₂-C₁₆, ou un radical alcénynediyle en C₂-C₁₆, qui peut dans chaque cas comporter une ou plusieurs liaisons multiples C-C,
U représente une liaison ou un atome d'hydrogène ou un radical de la série des groupements d'hétéroatomes suivants: -CO-, -O(CO)-, -(CO)-O-, -(CO)NR-, -NR(CO)-, -O-, -SO-, -SO₂-, -NR, dans lequel R représente un atome d'hydrogène ou un groupe alkyle en C₁-C₃,
r est 1, 2, 3 ou 4,
D représente une liaison ou un atome d'hydrogène ou un radical alcanediyle en C₁-C₁₀ aliphatique, ramifié ou non ramifié, ou
un radical alcènediyle en C₁-C₁₀ ramifié ou non ramifié,
un radical alcynediyle en C₂-C₁₀ ou un radical alcénynediyle en C₂-C₁₀, qui peut dans chaque cas comporter une ou plusieurs multiples C-C,
W représente une liaison ou
un atome d'hydrogène ou
un groupe alkyle, alcényle, alcynyle ou alcénynyle cycloaliphatique en C₃-C₁₀ ou
un radical aryle en C₆-C₁₆ ou un radical hétéroaryle à 5 ou 6 chaînons, au moins l'une des variables C, D et W ne représentant pas une liaison et U n'ayant la signification d'un groupement d'hétéroatomes que lorsque C ne représente pas une liaison ou lorsque D et/ou W ne représentent pas une liaison, et
C, D et/ou W, lorsqu'ils ne représentent pas une liaison ni un atome d'hydrogène, sont pour leur part de préférence substitués par une combinaison de jusqu'à 5 substituants identiques ou différents, choisis parmi
des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)- alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl,
alkyl(C₁-C₁₂)carbonyle, cycloalkyl(C₃-C₈)carbonyle, aryl(C₆-C₁₂)carbonyle, aralkyl(C₇-C₁₆)carbonyle, cinnamoyle, alcényl(C₃-C₁₂)carbonyle, alcynyl(C₃-C₁₂)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₃-C₁₂)carbonyle, alcynyloxy(C₃-C₁₂)carbonyle,
alkyl(C₁-C₁₂)carbonyloxy, cycloalkyl(C₃-C₈)carbonyloxy, aryl(C₆-C₁₂)carbonyloxy, aralkyl(C₇-C₁₆)carbonyloxy, cinnamoyloxy, alcényl(C₃-C₁₂)carbonyloxy, alcynyl(C₃-C₁₂)carbonyloxy,
alcoxy(C₁-C₁₂)carbonyloxy, alcoxy(C₁-C₁₂)alcoxy(C₁-C₁₂)carbonyloxy, aryloxy(C₆-C₁₂)carbonyloxy, aralkyloxy(C₇-C₁₆)carbonyloxy, cycloalcoxy(C₃-C₈)carbonyloxy, alcényloxy(C₃-C₁₂)carbonyloxy, alcynyloxy(C₃-C₁₂)carbonyloxy,
carbamoyle, N-alkyl(C₁-C₁₂)carbamoyle, N,N-dialkyl(C₁-C₁₂)carbamoyle, N-cycloalkyl(C₃-C₈)carbamoyle, N-aryl(C₆-C₁₆)carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₆)-alkyl(C₁-C₁₀)]carbamoyle, N-alkyl(C₁-C₁₀)-N-[aralkyloxy(C₇-C₁₆)-alkyl(C₁-C₁₀)]carbamoyle,
carbamoyloxy, N-alkyl(C₁-C₁₂)carbamoyloxy, N,N-dialkyl(C₁-C₁₂)carbamoyloxy, N-cycloalkyl(C₃-C₈)carbamoyloxy, N-aryl(C₆-C₁₆)carbamoyloxy, N-aralkyl(C₇-C₁₆)carbamoyloxy, N-alkyl(C₁-C₁₀)-N-aryl(C₆-C₁₂)carbamoyloxy, N-alkyl(C₁-C₁₀)-N-aralkyl(C₇-C₁₆)carbamoyloxy, N-[alcoxy(C₁-C₁₀)-alkyl(C₁-C₁₀)]carbamoyloxy, N-[aryloxy(C₆-C₁₆)alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)alkyl(C₁-C₁₀)]carbamoyloxy, N-alkyl(C₁-C₁₀)-N-[aryloxy(C₆-C₁₆)-alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl(C₁-C₁₀)-aralkyl(C₇-C₁₀)amino, N-alkyl(C₁-C₁₀)-N-aryl(C₆-C₁₂)amino, alcoxy(C₁-C₁₂)amino, alcoxy(C₁-C₁₂)-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
sulfamoyle, N-alkyl(C₁-C₁₀)sulfamoyle, N,N-dialkyl(C₁-C₁₀)sulfamoyle, cycloalkyl(C₃-C₈)sulfamoyle, N-aryl(C₆-C₁₆)sulfamoyle, N-aralkyl(C₇-C₁₆)sulfamoyle, N-alkyl(C₁-C₁₀)-N-aryl(C₆-C₁₆)sulfamoyle, N-alkyl(C₁-C₁₀)-N-aralkyl(C₇-C₁₆)sulfamoyle,
alkyl(C₁-C₁₀)sulfonamido, N-[alkyl(C₁-C₁₀)]-alkyl(C₁-C₁₀)sulfonamido, aralkyl(C₇-C₁₆)sulfonamido, N-[alkyl(C₁-C₁₀)]-aralkyl(C₇-C₁₆)sulfonamido, les radicaux qui contiennent un reste aryle pouvant pour leur part être substitués sur le fragment aryle par 1, 2, 3, 4 ou 5 substituants identiques ou différents, choisis parmi
des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)alkyle(C₁-C₁₂), alcoxy(C₁-C₁₂)alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl,
alkyl(C₁-C₁₂)carbonyle, cycloalkyl(C₃-C₈)carbonyle, aryl(C₆-C₁₂)carbonyle, aralkyl(C₇-C₁₆)carbonyle, cinnamoyle, alcényl(C₃-C₁₂)carbonyle, alcynyl(C₃-C₁₂)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₃-C₁₂)carbonyle, alcynyloxy(C₃-C₁₂)carbonyle,
alkyl(C₁-C₁₂)carbonyloxy, cycloalkyl(C₃-C₈)carbonyloxy, aryl(C₆-C₁₂)carbonyloxy, aralkyl(C₇-C₁₆)carbonyloxy, cinnamoyloxy, alcényl(C₃-C₁₂)carbonyloxy, alcynyl(C₃-C₁₂)carbonyloxy,
alcoxy(C₁-C₁₂)carbonyloxy, alcoxy(C₁-C₁₂)alcoxy(C₁-C₁₂)carbonyloxy, aryloxy(C₆-C₁₂)carbonyloxy, aralkyloxy(C₇-C₁₆)carbonyloxy, cycloalcoxy(C₃-C₈)carbonyloxy, alcényloxy(C₃-C₁₂)carbonyloxy, alcynyloxy(C₃-C₁₂)carbonyloxy,
carbamoyle, N-alkyl(C₁-C₁₂)carbamoyle, N,N-dialkyl(C₁-C₁₂)carbamoyle, N-cycloalkyl(C₃-C₈)carbamoyle, N-aryl(C₆-C₁₆)carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₆)-alkyl(C₁-C₁₀)]carbamoyle, N-alkyl(C₁-C₁₀)-N-[aralkyloxy(C₇-C₁₆)-alkyl(C₁-C₁₀)]carbamoyle,
carbamoyloxy, N-alkyl(C₁-C₁₂)carbamoyloxy, N,N-dialkyl(C₁-C₁₂)carbamoyloxy, N-cycloalkyl(C₃-C₈)carbamoyloxy, N-aryl(C₆-C₁₆)carbamoyloxy, N-aralkyl(C₇-C₁₆)carbamoyloxy, N-alkyl(C₁-C₁₀)-N-aryl(C₆-C₁₂)carbamoyloxy, N-alkyl(C₁-C₁₀)-N-aralkyl(C₇-C₁₆)carbamoyloxy, N-[alcoxy(C₁-C₁₀)-alkyl(C₁-C₁₀)]carbamoyloxy, N-[aryloxy(C₆-C₁₆)alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)alkyl(C₁-C₁₀)]carbamoyloxy, N-alkyl(C₁-C₁₀)-N-[aryloxy(C₆-C₁₆)-alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl(C₁-C₁₀)-N-aralkyl(C₇-C₁₆)amino, N-alkyl(C₁-C₁₀)-N-aryl(C₆-C₁₂)amino, alcoxy(C₁-C₁₂)amino, alcoxy(C₁-C₁₂)-N-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
sulfamoyle, N-alkyl(C₁-C₁₀)sulfamoyle, N,N-dialkyl(C₁-C₁₀)sulfamoyle, cycloalkyl(C₃-C₈)sulfamoyle, N-aryl(C₆-C₁₆)sulfamoyle, N-aralkyl(C₇-C₁₆)sulfamoyle, N-alkyl(C₁-C₁₀)-N-aryl(C₆-C₁₆)sulfamoyle, N-alkyl(C₁-C₁₀)-N-aralkyl(C₇-C₁₆)sulfamoyle,
alkyl(C₁-C₁₀)sulfonamido, N-[alkyl(C₁-C₁₀)]-alkyl(C₁-C₁₀)sulfonamido, aralkyl(C₇-C₁₆)sulfonamido, N-[alkyl(C₁-C₁₀)]-aralkyl(C₇-C₁₆)sulfonamido,
et
n est 0 ou 1,
f va de 1 à 8, de préférence de 1 à 5,
g est 0, 1 à (2f+1) et
x représente 0 à 8, de préférence 0 ou 1,
à l'exception des composés suivants:
5-[((méthylsulfonyl)amino)carbonyl]pyridine-2-carboxylate de méthyle,
5-[((2-propylsulfonyl)amino)carbonyl]pyridine-2-carboxylate de méthyle,
5-[((phénylsulfonyl)amino)carbonyl]pyridine-2-carboxylate de méthyle,
5-[((benzylsulfonyl)amino)carbonyl]pyridine-2-carboxylate de méthyle,
5-[((4-méthoxyphénylsulfonyl)amino)carbonyl]pyridine-2-carboxylate de méthyle,
5-[((1-naphtylsulfonyl)amino)carbonyl]pyridine-2-carboxylate de méthyle,
5-[((4,5-dibromo-2-thiénylsulfonyl)amino)carbonyl]pyridine-2-carboxylate de méthyle,
5-[((5-chloro-2-thiénylsulfonyl)amino)carbonyl]pyridine-2-carboxylate de méthyle,
5-[((8-quinolylsulfonyl)amino)carbonyl]pyridine-2-carboxylate de méthyle,
5-[((4-(2-(4,7-dichloroquinolyl))phénylsulfonyl)amino)carbonyl]pyridine-2-carboxylate de méthyle.

2. Composés selon la revendication 1, dans lesquels
X est une liaison simple ou -CO-,
R¹, R² et R³ sont identiques ou différents et représentent un atome d'hydrogène ou d'halogène, en particulier de fluor ou de chlore, ou un groupe alkyle en C₁-C₆, alcoxy en C₁-C₆, hydroxy, amino,
R⁴ représente le reste d'un alcool R⁴OH dans lequel R⁴ représente un groupe acyloxy(C₁-C₁₀)-alkyle(C₁-C₆), de préférence un groupe alcanoyloxy(C₁-C₁₀)-alkyle(C₁-C₆), benzoyloxy-alkyle(C₁-C₆), benzyloxycarbonyloxy-alkyle(C₁-C₆) ou alcoxy(C₁-C₆)carbonyloxy-alkyle(C₁-C₆),
un radical alkyle en C₁-C₁₂ aliphatique ramifié ou non ramifié, ou cycloaliphatique,
un radical alcényle en C₂-C₁₂ ramifié ou non ramifié, un radical alcynyle en C₂-C₁₂ ou un radical alcénynyle en C₂-C₁₂, qui peut dans chaque cas comporter une ou plusieurs liaisons multiples, ou
un radical aryle en C₆-C₁₆, un radical aralkyle en C₆-C₁₆, un radical hétéroaryle ou un radical hétéroaralkyle,
les radicaux précédents pouvant porter 1 ou 2 substituants choisis parmi
des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)-aralkyle(C₁-C₁₂), alcoxy(C₁-C₁₂)-aryloxy(C₁-C₁₂), aralkyloxy en C₇-C₁₆, hydroxyalkyle en C₁-C₈,
alkyl(C₁-C₁₂)carbonyle, cycloalkyl(C₃-C₈)carbonyle, aryl(C₆-C₁₂)carbonyle, aralkyl(C₇-C₁₆)carbonyle, cinnamoyle, alcényl(C₃-C₁₂)carbonyle, alcynyl(C₃-C₁₂)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₃-C₁₂)carbonyle, alcynyloxy(C₃-C₁₂)carbonyle,
alkyl(C₁-C₁₂)carbonyloxy, cycloalkyl(C₃-C₈)carbonyloxy, aryl(C₆-C₁₂)carbonyloxy, aralkyl(C₇-C₁₆)carbonyloxy, cinnamoyloxy, alcényl(C₃-C₁₂)carbonyloxy, alcynyl(C₃-C₁₂)carbonyloxy,
alcoxy(C₁-C₁₂)carbonyloxy, alcoxy(C₁-C₁₂)alcoxy(C₁-C₁₂)carbonyloxy, aryloxy(C₆-C₁₂)carbonyloxy, aralkyloxy(C₇-C₁₆)carbonyloxy, cycloalcoxy(C₃-C₈)carbonyloxy, alcényloxy(C₃-C₁₂)carbonyloxy, alcynyloxy(C₃-C₁₂)carbonyloxy,
carbamoyle, N-alkyl(C₁-C₁₂)carbamoyle, N,N-dialkyl(C₁-C₁₂)carbamoyle, N-cycloalkyl(C₃-C₈)carbamoyle, N-aryl(C₆-C₁₆)carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₆)-alkyl(C₁-C₁₀)]carbamoyle, N-alkyl(C₁-C₁₀)-N-[aralkyloxy(C₇-C₁₆)-alkyl(C₁-C₁₀)]carbamoyle,
carbamoyloxy, N-alkyl(C₁-C₁₂)carbamoyloxy, N,N-dialkyl(C₁-C₁₂)carbamoyloxy, N-cycloalkyl(C₃-C₈)carbamoyloxy, N-aryl(C₆-C₁₆)carbamoyloxy, N-aralkyl(C₇-C₁₆)carbamoyloxy, N-alkyl(C₁-C₁₀)-N-aryl(C₆-C₁₂)carbamoyloxy, N-alkyl(C₁-C₁₀)-N-aralkyl(C₇-C₁₆)carbamoyloxy, N-[alcoxy(C₁-C₁₀)-alkyl(C₁-C₁₀)]carbamoyloxy, N-[aryloxy(C₆-C₁₆)alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₆)-alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
amino, alkyl(C₁-C₁₂)amino, dialkyl(C₁-C₁₂)amino, cycloalkyl(C₃-C₈)amino, N-aralkyl(C₇-C₁₁)amino, N-alkyl-arylamino, alcoxy(C₁-C₁₂)amino, alcoxy(C₁-C₁₂)-N-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino et aralcanoyl(C₇-C₁₁)-N-alkyl(C₁-C₁₀)amino, et les radicaux qui contiennent un reste aryle pouvant pour leur part être substitués sur le fragment aryle par 1 à 5 radicaux identiques ou différents, choisis parmi
des atomes d'halogène et des groupes hydroxy, cyano, trifluorométhyle, alkyle en C₁-C₁₂, cycloalkyle en C₃-C₈, alcoxy en C₁-C₁₂, alcoxy(C₁-C₁₂)-alcoxy(C₁-C₁₂),
alkyl(C₁-C₁₂)carbonyle, cycloalkyl(C₃-C₈)carbonyle,
alcoxy(C₁-C₁₂)carbonyle, alcoxy(C₁-C₁₂)-alcoxy(C₁-C₁₂)carbonyle, cycloalcoxy(C₃-C₈)carbonyle,
alkyl(C₁-C₁₂)carbonyloxy, cycloalkyl(C₃-C₈)carbonyloxy, alcoxy(C₁-C₁₂)carbonyloxy, alcoxy(C₁-C₁₂)-alcoxy (C₁-C₁₂)carbonyloxy, cycloalcoxy(C₃-C₈)carbonyloxy,
carbamoyle, N-alkyl(C₁-C₁₂)carbamoyle, N,N-dialkyl(C₁-C₁₂)carbamoyle, N-cycloalkyl(C₃-C₈)carbamoyle, N-[alcoxy(C₁-C₁₀)-alkyl(C₁-C₁₀)]carbamoyle, N-alkylC₁-C₁₀)-N-[alcoxy(C₁-C₁₀)-alkyl(C₁-C₁₀)]carbamoyle,
carbamoyloxy, N-alkyl(C₁-C₁₂)carbamoyloxy, N,N-dialkyl(C₁-C₁₂)carbamoyloxy, N-cycloalkyl(C₃-C₈)carbamoyloxy,
amino, alkyl(C₁-C₁₂)amino, dialkyl(C₁-C₁₂)amino, cycloalkyl(C₃-C₈)amino,
alcanoyl(C₁-C₁₂)amino, cycloalcanoyl(C₃-C₈)amino, alcanoyl(C₁-C₁₂)amino-alkyle(C₁-C₈), cycloalcanoyl(C₃-C₈)amino-alkyle(C₁-C₈),
alkyl(C₁-C₁₂)mercapto, alkyl(C₁-C₁₂)sulfinyle et alkyl(C₁-C₁₂)sulfonyle,
R⁵ représente un atome d'hydrogène ou un groupe alkyle en C₁-C₃, alcanoyle en C₁-C₄ ou un cation mono-, di- ou trivalent, physiologiquement utilisable, en particulier Na^{⊕}, K^{⊕}, Mg^{2⊕}, Ca^{2⊕} ou un ion ammonium éventuellement 1 à 3 fois substitué par un ou des groupes hydroxyalkyle en C₁-C₈, alxoxy(C₁-C₄)-alkyle(C₁-C₈), phényle, benzyle ou alkyle en C₁-C₈, qui peut être 1 à 3 fois substitué par un ou des radicaux hydroxy et/ou alcoxy en C₁-C₄, ou un cation d'un dérivé d'aminoacide basique,
R⁶ représente un radical de formule II, à l'exception de -SO₂H
-Y-(C-U)ᵣ-D-W (II)
dans laquelle
Y représente -SO₂-,
C représente une liaison ou un radical alcanediyle en C₁-C₁₂ aliphatique, ramifié ou non ramifié, ou un radical alcènediyle en C₂-C₁₂ ramifié ou non ramifié, un radical alcynediyle en C₂-C₁₂ ou un radical alcénynediyle en C₂-C₁₂, qui peut comporter une ou plusieurs liaisons multiples C-C,
U représente une liaison ou
un atome d'hydrogène ou un radical choisi dans la série des groupements d'hétéroatomes suivants: -(CO)NR-, -NR(CO)-, -O-, -SO-, -SO₂-, R représentant un atome d'hydrogène ou un groupe alkyle en C₂-C₃,
r est 1 ou 2,
D représente une liaison ou un atome d'hydrogène ou un radical alcanediyle en C₁-C₈ aliphatique, ramifié ou non ramifié, ou
un radical alcynediyle en C₂-C₈ ou alcènediyle en C₂-C₈ ramifié ou non ramifié, et
W représente une liaison ou
un atome d'hydrogène ou
un radical alkyle, alcényle, alcynyle ou alcénynyle cycloaliphatique en C₃-C₁₀ ou
un radical aryle en C₆-C₁₆ ou un radical hétéroaryle à 5 ou 6 chaînons, au moins l'une des variables C, D et W ne représentant pas une liaison et U n'ayant la signification d'un groupement d'hétéroatomes que lorsque C ne représente pas une liaison ou lorsque D et/ou W ne représentent pas une liaison, et
C, D et/ou W, lorsqu'ils ne représentent pas un atome d'hydrogène ni une liaison, sont pour leur part de préférence substitués par une combinaison de jusqu'à 5 substituants identiques ou différents, choisis parmi
des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)-alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl,
alkyl(C₁-C₁₂)carbonyle, cycloalkyl(C₃-C₈)carbonyle, aryl(C₆-C₁₂)carbonyle, aralkyl(C₇-C₁₆)carbonyle, cinnamoyle, alcényl(C₃-C₁₂)carbonyle, alcynyl(C₃-C₁₂)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₃-C₁₂)carbonyle, alcynyloxy(C₃-C₁₂)carbonyle,
alkyl(C₁-C₁₂)carbonyloxy, cycloalkyl(C₃-C₈)carbonyloxy, aryl(C₆-C₁₂)carbonyloxy, aralkyl(C₇-C₁₆)carbonyloxy, cinnamoyloxy, alcényl(C₃-C₁₂)carbonyloxy, alcynyl(C₃-C₁₂)carbonyloxy,
carbamoyle, N-alkyl(C₁-C₁₂)carbamoyle, N,N-dialkyl(C₁-C₁₂)carbamoyle, N-cycloalkyl(C₃-C₈)carbamoyle, N-aryl(C₆-C₁₆)carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₆)-alkyl(C₁-C₁₀)]carbamoyle, N-alkyl(C₁-C₁₀)-N-[aralkyloxy(C₇-C₁₆)-alkyl(C₁-C₁₀)]carbamoyle,
amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl(C₁-C₅)-N-aralkyl(C₇-C₁₀)amino, N-alkyl(C₁-C₅)-N-aryl(C₆-C₁₂)amino, alcoxy(C₁-C₁₂)amino, alcoxy(C₁-C₁₂)-N-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
les radicaux qui contiennent un reste aryle pouvant pour leur part être substitués sur le fragment aryle par 1, 2, 3, 4 ou 5 substituants identiques ou différents, choisis parmi
des atomes d'hydrogène ou d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl,
alkyl(C₁-C₁₂)carbonyle, cycloalkyl(C₃-C₈)carbonyle, aryl(C₆-C₁₂)carbonyle, aralkyl(C₇-C₁₆)carbonyle, cinnamoyle, alcényl(C₃-C₁₂)carbonyle, alcynyl(C₃-C₁₂)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₃-C₁₂)carbonyle, alcynyloxy(C₃-C₁₂)carbonyle, alkyl(C₁-C₁₂)carbonyloxy, cycloalkyl(C₃-C₈)carbonyloxy, aryl(C₆-C₁₂)carbonyloxy, aralkyl(C₇-C₁₆)carbonyloxy, cinnamoyloxy, alcényl(C₃-C₁₂)carbonyloxy, alcynyl(C₃-C₁₂)carbonyloxy,
carbamoyle, N-alkyl(C₁-C₁₂)carbamoyle, N,N-dialkyl(C₁-C₁₂)carbamoyle, N-cycloalkyl(C₃-C₈)carbamoyle, N-aryl(C₆-C₁₆)carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₆)-alkyl(C₁-C₁₀)]carbamoyle, N-alkyl(C₁-C₁₀)-N-[aralkyloxy(C₇-C₁₆)-alkyl(C₁-C₁₀)]carbamoyle,
amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl(C₁-C₅)-aralkyl(C₇-C₁₀)amino, N-alkyl(C₁-C₅)-aryl(C₆-C₁₂)amino, alcoxy(C₁-C₁₂)amino, alcoxy(C₁-C₁₂)-N-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
à l'exception des composés suivants:
5-[((méthylsulfonyl)amino)carbonyl]pyridine-2-carboxylate de méthyle,
5-[((2-propylsulfonyl)amino)carbonyl]pyridine-2-carboxylate de méthyle,
5-[((phénylsulfonyl)amino)carbonyl]pyridine-2-carboxylate de méthyle,
5-[((benzylsulfonyl)amino)carbonyl]pyridine-2-carboxylate de méthyle,
5-[((4-méthoxyphénylsulfonyl)amino)carbonyl]pyridine-2-carboxylate de méthyle,
5-[((1-naphtylsulfonyl)amino)carbonyl]pyridine-2-carboxylate de méthyle,
5-[((4,5-dibromo-2-thiénylsulfonyl)amino)carbonyl]pyridine-2-carboxylate de méthyle,
5-[((5-chloro-2-thiénylsulfonyl)amino)carbonyl]pyridine-2-carboxylate de méthyle,
5-[((8-quinolylsulfonyl)amino)carbonyl]pyridine-2-carboxylate de méthyle,
5-[((4-(2-(4,7-dichloroquinolyl))phénylsulfonyl)amino)carbonyl]pyridine-2-carboxylate de méthyle.

3. Composés selon la revendication 1 ou 2, dans lesquels
X est une liaison simple ou -CO-,
R¹, R² et R³ sont identiques ou différents et représentent un atome d'hydrogène, de fluor ou de chlore, ou un groupe alkyle en C₁-C₃, alcoxy en C₁-C₃ ou hydroxy,
R⁴ représente le reste d'un alcool R⁴OH dans lequel R⁴ représente un groupe acyloxy(C₁-C₁₀)-alkyle(C₁-C₆), de préférence un groupe alcanoyloxy(C₁-C₁₀)-alkyle(C₁-C₆), benzoyloxy-alkyle(C₁-C₆), benzyloxycarbonyloxy-alkyle(C₁-C₆) ou alcoxy(C₁-C₆)carbonyloxy-alkyle(C₁-C₆),
un radical alkyle en C₁-C₁₀ ramifié ou non ramifié ou cyclique aliphatique, ou
un radical alcényle en C₂-C₁₀ ou alcynyle en C₂-C₁₂, ramifié ou non ramifié, qui peut dans chaque cas comporter une ou plusieurs liaisons multiples C-C, ou
un radical aryle en C₆-C₁₆, un radical aralkyle en C₇-C₁₁ ou un radical hétéroaryle ou hétéroaralkyle, les radicaux précédents pouvant porter 1 ou 2 substituants choisis parmi
des groupes hydroxy, alcoxy en C₁-C₆, alcoxy(C₁-C₆)alcoxy(C₁-C₆), aryloxy en C₆-C₁₂, aralkyloxy en C₇-C₁₂,
alkyl(C₁-C₈)carbonyle, cycloalkyl(C₃-C₈)carbonyle, aryl(C₆-C₁₂)carbonyle, aralkyl(C₇-C₁₂)carbonyle,
alcoxy(C₁-C₈)carbonyle, alcoxy(C₁-C₆)-alcoxy(C₁-C₆)carbonyle, aryloxy(C₆-C₁₂)carbonyle, aralcoxy(C₇-C₁₂)carbonyle, cycloalcoxy(C₃-C₈)carbonyle,
alkyl(C₁-C₈)carbonyloxy, cycloalkyl(C₃-C₈)carbonyloxy, aryl(C₆-C₁₂)carbonyloxy, aralkyl(C₇-C₁₂)carbonyloxy,
alcoxy(C₁-C₈)carbonyloxy, alcoxy(C₁-C₆)-alcoxy(C₁-C₆)carbonyloxy, aryloxy(C₆-C₁₂)carbonyloxy, aralkyloxy(C₇-C₁₂)carbonyloxy, cycloalcoxy(C₃-C₈)carbonyloxy,
carbamoyle, N-alkyl(C₁-C₈)carbamoyle, N,N-dialkyl(C₁-C₈)carbamoyle, N-cycloalkyl(C₃-C₈)carbamoyle, N-[alcoxy(C₁-C₆)-alkyl(C₁-C₆)]carbamoyle,
amino, alkyl(C₁-C₆)amino, dialkyl(C₁-C₆)amino, cycloalkyl(C₃-C₈)amino, N-aryl(C₆-C₁₂)amino, N-aralkyl(C₇-C₁₁)amino, N-alkyl(C₁-C₅)-aryl(C₆-C₁₂)amino,
alcanoyl(C₁-C₈)amino, cycloalcanoyl(C₃-C₈)amino, aroyl(C₆-C₁₂)amino, aralcanoyl(C₇-C₁₂)amino, alcanoyl(C₁-C₈)-N-alkyl(C₁-C₆)amino, cycloalcanoyl(C₃-C₈)-N-alkyl(C₁-C₆)amino, aroyl(C₆-C₁₂)-N-alkyl(C₁-C₆)amino, aralcanoyl(C₇-C₁₁)-N-alkyl(C₁-C₆)amino, et les radicaux qui comportent un reste aryle étant en particulier substitués par jusqu'à 3 substituants choisis parmi
des atomes de fluor et de chlore et des groupes hydroxy, cyano, trifluorométhyle, alkyle en C₁-C₆, cycloalkyle en C₃-C₈, alcoxy en C₁-C₈,
alkyl(C₁-C₆)carbonyle, cycloalkyl(C₃-C₈)carbonyle, alcoxy(C₁-C₆)carbonyle, cycloalcoxy(C₃-C₈)carbonyle,
alkyl(C₁-C₆)carbonyloxy, cycloalkyl(C₃-C₈)carbonyloxy, alcoxy(C₁-C₆)carbonyloxy,
cycloalcoxy(C₃-C₈)carbamoyloxy,
carbamoyle, N-alkyl(C₁-C₆)carbamoyle, N,N-dialkyl(C₁-C₆)carbamoyle, N-cycloalkyl(C₃-C₈)carbamoyle, N-[alcoxy(C₁-C₆)-alkyl(C₁-C₆)]carbamoyle, N-alkyl(C₁-C₆)-N-[alcoxy(C₁-C₆)-alkyl(C₁-C₆)]carbamoyle,
carbamoyloxy, N-alkyl(C₁-C₆)carbamoyloxy, N,N-dialkyl(C₁-C₆)carbamoyloxy, N-cycloalkyl(C₃-C₈)carbamoyloxy,
amino, alkyl(C₁-C₆)amino, dialkyl(C₁-C₆)amino, cycloalkyl(C₃-C₈)amino,
alcanoyl(C₁-C₆)amino, cycloalcanoyl(C₃-C₈)amino,
alkyl(C₁-C₆)mercapto, alkyl(C₁-C₆)sulfinyle, alkyl(C₁-C₆)sulfonyle, et
R⁵ représente un atome d'hydrogène ou un groupe alkyle en C₁-C₃, alcanoyle en C₁-C₄ ou un cation mono-, di- ou trivalent, physiologiquement utilisable, en particulier Na^{⊕}, K^{⊕}, Mg^{2⊕}, Ca^{2⊕} ou un ion ammonium éventuellement 1 à 3 fois substitué par un ou des groupes hydroxyalkyle en C₁-C₈, alcoxy(C₁-C₄)-alkyl(C₁-C₈), phényle, benzyle ou alkyle en C₁-C₈ qui peut être 1 à 3 fois substitué par un ou des radicaux hydroxy et/ou alcoxy en C₁-C₄,
R⁶ représente un radical de formule II, à l'exception de -SO₂H
-Y-(C-U)ᵣ-D-W (II)
dans laquelle
Y représente -SO₂-,
C représente une liaison ou un radical alcanediyle en C₁-C₆,
U représente une liaison ou un atome d'hydrogène ou un -O-,
r est 1,
D représente une liaison ou
un atome d'hydrogène ou
un radical alcanediyle en C₁-C₈ aliphatique non ramifié, et
W représente une liaison ou un atome d'hydrogène ou un radical aryle en C₆-C₁₂, ou un radical hétéroaryle à 5 ou 6 chaînons, au moins l'une des variables C, D et W ne représentant pas une liaison et U n'ayant la signification d'un groupement d'hétéroatomes que lorsque C ne représente pas une liaison ou lorsque D et/ou W ne représentent pas une liaison, et
C, D et/ou W, lorsqu'ils ne représentent pas une liaison ni un atome d'hydrogène, sont pour leur part de préférence substitués par jusqu'à 3 substituants identiques ou différents, choisis parmi
des atomes d'halogène et des groupes hydroxy, cyano, trifluorométhyle, nitro, carboxy, alkyle en C₁-C₈, cycloalkyle en C₃-C₈, aryle en C₆-C₁₂, aralkyle en C₇-C₁₆, alcényle en C₃-C₈, alcynyle en C₃-C₈, alcoxy en C₁-C₈, alcoxy(C₁-C₈)-alkyle(C₁-C₈), aryloxy en C₆-C₁₂, aralkyloxy en C₇-C₁₆, hydroxyalkyle en C₁-C₈, -O-[CH₂]ₓC_{f}H_{(2f+1-g)}F_{g},
alcoxy(C₁-C₈)carbonyle, aryloxy(C₆-C₁₂)carbonyle, aralcoxy(C₇-C₁₄)carbonyle, cycloalcoxy(C₃-C₈)carbonyle,
alkyl(C₁-C₈)carbonyloxy, cycloalkyl(C₃-C₈)carbonyloxy, aryl(C₆-C₁₂)carbonyloxy, aralkyl(C₇-C₁₆)carbonyloxy, cinnamoyloxy,
carbamoyle, N-alkyl(C₁-C₁₂)-carbamoyle, N,N-dialkyl(C₁-C₁₂)-carbamoyle, N-cycloalkyl(C₃-C₈)carbamoyle, N-aryl(C₆-C₁₆)-carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₆)-alkyl(C₁-C₁₀)]carbamoyle, N-alkyl(C₁-C₁₀)-N-[aralkyloxy(C₇-C₁₆)-alkyl(C₁-C₁₀)]carbamoyle,
amino, alkyl(C₁-C₈)amino, dialkyl(C₁-C₈)amino, cycloalkyl(C₃-C₈)amino, N-aryl(C₆-C₁₂)amino, N-aralkyl(C₇-C₁₁)amino, N-alkyl(C₁-C₃)-N-aralkyl(C₇-C₁₁)amino,
alcanoyl(C₁-C₁₀)amino, cycloalcanoyl(C₃-C₈)amino, aroyl(C₆-C₁₂)amino, aralcanoyl(C₇-C₁₆)amino, alcanoyl(C₁-C₁₀)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₆), cycloalcanoyl(C₃-C₈)amino-alkyle(C₁-C₈), aroyl(C₆-C₁₆)amino-alkyle(C₁-C₆), aralcanoyl(C₇-C₁₆)amino-alkyle(C₁-C₆),
alkyl(C₁-C₈)mercapto, alkyl(C₁-C₈)sulfinyle, alkyl(C₁-C₈)sulfonyle, aryl(C₆-C₁₂)mercapto, aryl(C₆-C₁₂)sulfinyle, aryl(C₆-C₁₂)sulfonyle, aralkyl(C₇-C₁₄)mercapto, aralkyl(C₇-C₁₄)sulfinyle, aralkyl(C₇-C₁₄)sulfonyle,
les radicaux qui contiennent un reste aryle pouvant pour leur part être substitués sur le fragment aryle par 1, 2, 3, 4 ou 5 substituants identiques ou différents, choisis parmi
des atomes d'hydrogène et d'halogène et des groupes hydroxy, cyano, trifluorométhyle, nitro, carboxy, alkyle en C₁-C₈, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₈, alcoxy en C₁-C₆-alkyle en C₁-C₆, aryloxy en C₆-C₁₂, aralkyloxy en C₇-C₁₆, -O-[CH₂]ₓC_{f}H_{(2f+1-g)}F_{g},
alcoxy(C₁-C₁₂)carbonyle, cycloalcoxy(C₃-C₈)carbonyle,
alkyl(C₁-C₁₂)carbonyloxy, cycloalkyl(C₃-C₈)carbonyloxy,
carbamoyle, N-alkyl(C₁-C₈)carbamoyle, N,N-dialkyl(C₁-C₈)carbamoyle, N-cycloalkyl(C₃-C₈)carbamoyle, N-aryl(C₆-C₁₆)carbamoyle, N-aralkyl(C₇-C₁₆)carbamoyle, N-alkyl(C₁-C₈)-N-aryl(C₆-C₁₆)carbamoyle, N-alkyl(C₁-C₈)-N-aralkyl(C₇-C₁₆)carbamoyle, N-[alcoxy(C₁-C₆)-alkyl(C₁-C₆)]carbamoyle, N-[aryloxy(C₆-C₁₆)-alkyl(C₁-C₆)]carbamoyle, N-[aralkyloxy(C₇-C₁₆)-alkyl(C₁-C₆)]carbamoyle, N-alkyl(C₁-C₈)-N-[alcoxy(C₁-C₆)-alkyl(C₁-C₆)]carbamoyle, N-alkyl(C₁-C₈)-N-[aryloxy(C₆-C₁₆)-alkyl(C₁-C₆)]carbamoyle, N-alkyl(C₁-C₈)-N-[aralkyloxy(C₇-C₁₆)-alkyl(C₁-C₆)]carbamoyle,
amino, alkyl(C₁-C₆)amino, dialkyl(C₁-C₆)amino, cycloalkyl(C₃-C₈)amino, N-aryl(C₆-C₁₂)amino, N-aralkyl
(C₇-C₁₁)amino, N-alkyl(C₁-C₃)-aralkyl(C₇-C₁₁)amino, N-alkyl(C₁-C₃)-aryl-(C₆-C₁₂)amino, alcoxy(C₁-C₈)amino,
alcanoyl(C₁-C₈)amino, cycloalcanoyl(C₃-C₈)amino, aroyl(C₆-C₁₂)amino, aralcanoyl(C₇-C₁₂)amino, alcanoyl(C₁-C₈)-N-alkyl(C₁-C₆)amino, cycloalcanoyl(C₃-C₈)-N-alkyl(C₁-C₆)amino, aroyl(C₆-C₁₂)-N-alkyl(C₁-C₁₀)amino, aralcanoyl(C₇-C₁₁)-N-alkyl(C₁-C₆)amino,
alcanoyl(C₁-C₈)amino-alkyle(C₁-C₄), cycloalcanoyl(C₃-C₈)amino-alkyle(C₁-C₄), aroyl(C₆-C₁₂)amino-alkyle(C₁-C₄) et aralcanoyl(C₇-C₁₂)amino-alkyle(C₁-C₄),
à l'exception des composés suivants:
5-[((méthylsulfonyl)amino)carbonyl]pyridine-2-carboxylate de méthyle,
5-[((2-propylsulfonyl)amino)carbonyl]pyridine-2-carboxylate de méthyle,
5-[((phénylsulfonyl)amino)carbonyl]pyridine-2-carboxylate de méthyle,
5-[((benzylsulfonyl)amino)carbonyl]pyridine-2-carboxylate de méthyle,
5-[((4-méthoxyphénylsulfonyl)amino)carbonyl]pyridine-2-carboxylate de méthyle,
5-[((1-naphtylsulfonyl)amino)carbonyl]pyridine-2-carboxylate de méthyle,
5-[((4,5-dibromo-2-thiénylsulfonyl)amino)carbonyl]pyridine-2-carboxylate de méthyle,
5-[((5-chloro-2-thiénylsulfonyl)amino)carbonyl]pyridine-2-carboxylate de méthyle,
5-[((8-quinolylsulfonyl)amino)carbonyl]pyridine-2-carboxylate de méthyle,
5-[((4-(2-(4,7-dichloroquinolyl))phénylsulfonyl)amino)carbonyl]pyridine-2-carboxylate de méthyle.

4. Composés selon une ou plusieurs des revendications 1 à 3, dans lesquels
X est une liaison simple ou -CO-,
R¹, R² et R³ représentent des atomes d'hydrogène,
R⁴ représente le reste d'un alcool R⁴OH ou un radical alkyle en C₁-C₉ ramifié ou non ramifié ou cyclique aliphatique, un radical alcényle en C₂-C₈ ramifié ou non ramifié ou un radical alcynyle en C₂-C₈, le radical phényle, benzyle, phénéthyle, phénylpropyle, les radicaux précédents contenant un substituant choisi parmi un atome d'hydrogène et un groupe hydroxy, alcoxy en C₁-C₄,
alcoxy(C₁-C₆)carbonyle, phénoxy(C₆-C₁₂)carbonyle, phénylalkyl(C₇-C₁₆)carbonyle, cycloalcoxy(C₃-C₈)carbonyle,
alkyl(C₁-C₆)carbonyloxy, cycloalkyl(C₃-C₈)carbonyloxy, benzoyloxy, phénylalkyl(C₇-C₁₆)carbonyloxy, cycloalcoxy(C₃-C₈)carbonyloxy,
ou représente un radical alcoxy(C₁-C₆)carbonyloxy, phénoxycarbonyloxy, phénylalkyl(C₇-C₁₆)carbonyloxy ou cycloalcoxy(C₃-C₈)carbonyloxy,
R⁵ représente un atome d'hydrogène ou un cation mono-, di- ou trivalent, physiologiquement acceptable, en particulier Na^{⊕}, K^{⊕}, Mg^{2⊕}, Ca^{2⊕} ou H₃N^{⊕}C(CH₂OH)₃ (sel Tris),
R⁶ représente un radical de formule II, à l'exception de -SO₂H
-Y-(C-U)ᵣ-D-W (II)
dans laquelle
Y représente -SO₂-,
C représente une liaison ou un groupe alcanediyle en C₁-C₄,
U représente une liaison, un atome d'hydrogène ou -O-,
r est 1,
D représente une liaison, un atome d'hydrogène ou un groupe alcanediyle en C₁-C₄,
W représente une liaison, un atome d'hydrogène ou le radical phényle, au moins l'une des variables C, D et W ne représentant pas une liaison et U n'ayant la signification d'un groupement d'hétéroatomes que lorsque C ne représente pas une liaison ou lorsque D et/ou W ne représentent pas une liaison, et
C, D et/ou W, sont substitués par un atome d'hydrogène ou par 1 ou 2 substituants choisis parmi des atomes de fluor et de chlore, des groupes alkyle en C₁-C₆, phényle, alcoxy en C₁-C₆, phénoxy, -O-[CH₂]ₓC_{f}H_{(2f+1-g)}F_{g},
carbamoyle, N-alkyl(C₁-C₁₀)carbamoyle, N,N-dialkyl(C₁-C₈)carbamoyle, N-cycloalkyl(C₃-C₈)carbamoyle, N-phénylcarbamoyle, N-phénylalkyl(C₇-C₁₆)carbamoyle, N-alkyl(C₁-C₈)-N-phényl(C₆-C₁₆)carbamoyle, N-alkyl(C₁-C₈)-N-phénylalkyl(C₇-C₁₆)carbamoyle, N-[alcoxy(C₁-C₄)alkyl(C₁-C₈)]carbamoyle, N-[phénoxy-alkyl(C₁-C₈)]carbamoyle, N-[phénylalkyloxy(C₇-C₁₆)-alkyl(C₁-C₈)]carbamoyle, N-alkyl(C₁-C₈)-N-[alcoxy(C₁-C₆)-alkyl(C₁-C₈)]carbamoyle, N-alkyl(C₁-C₈)-N-[phénoxy(C₆-C₁₂)-alkyl(C₁-C₈)]carbamoyle, N-alkyl(C₁-C₈)-N-[phénylalkyloxy(C₇-C₁₆)-alkyl(C₁-C₈)]carbamoyle,
alcanoyl(C₁-C₈)amino, cycloalcanoyl(C₃-C₈)amino, phényl(C₆-C₁₂)amino, phénylalcanoyl(C₇-C₁₁)amino, alcanoyl(C₁-C₈)-N-alkyl(C₁-C₁₀)amino, cycloalcanoyl(C₃-C₈)-N-alkyl(C₁-C₆)amino, benzoyl-N-alkyl(C₁-C₁₀)amino, phénylalcanoyl(C₇-C₁₁)-N-alkyl(C₁-C₆)amino,
alcanoyl(C₁-C₁₀)amino-alkyle(C₁-C₂), cycloalcanoyl(C₃-C₈)amino-alkyle(C₁-C₂), benzoylamino-alkyle(C₁-C₂), phénylalcanoyl(C₇-C₁₄)amino-alkyle(C₁-C₂), les radicaux qui contiennent un reste aryle étant pour leur part substitués par un substituant choisi parmi un atome d'hydrogène, de fluor ou de chlore, et des groupes hydroxy, trifluorométhyle, alkyle en C₁-C₆, alcoxy en C₁-C₈,
n = 0
f va de 1 à 5,
g représente 0, 1 à (2f+1) et
x est 0 ou 1,
à l'exception des composés suivants
5-[((méthylsulfonyl)amino)carbonyl]pyridine-2-carboxylate de méthyle,
5-[((2-propylsulfonyl)amino)carbonyl]pyridine-2-carboxylate de méthyle,
5-[((phénylsulfonyl)amino)carbonyl]pyridine-2-carboxylate de méthyle,
5-[((benzylsulfonyl)amino)carbonyl]pyridine-2-carboxylate de méthyle,
5-[((4-méthoxyphénylsulfonyl)amino)carbonyl]pyridine-2-carboxylate de méthyle,
5-[((1-naphtylsulfonyl)amino)carbonyl]pyridine-2-carboxylate de méthyle,
5-[((4,5-dibromo-2-thiénylsulfonyl)amino)carbonyl]pyridine-2-carboxylate de méthyle,
5-[((5-chloro-2-thiénylsulfonyl)amino)carbonyl]pyridine-2-carboxylate de méthyle,
5-[((8-quinolylsulfonyl)amino)carbonyl]pyridine-2-carboxylate de méthyle,
5-[((4-(2-(4,7-dichloroquinolyl))phénylsulfonyl)amino)carbonyl]pyridine-2-carboxylate de méthyle.

5. Composés selon une ou plusieurs des revendications 1 à 4, pour utilisation en tant que médicament.

6. Procédé pour la préparation des composés selon une ou plusieurs des revendications 1 à 5, dans lesquels X représente une liaison simple, caractérisé en ce que
I) on fait réagir un composé de formule 1 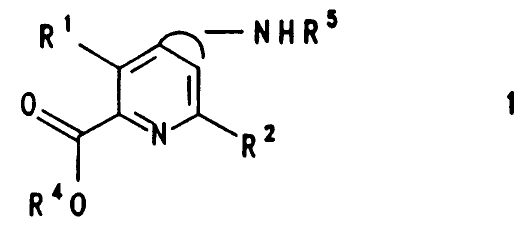 avec ZR⁶, pour aboutir à un composé de formule 3 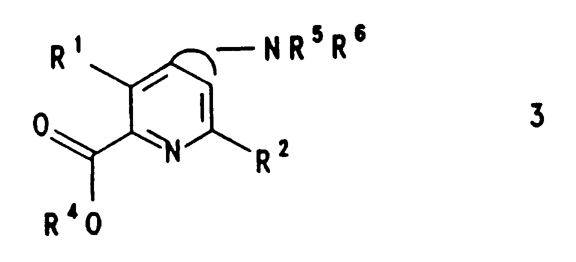 ou
II) on fait réagir un composé de formule 5 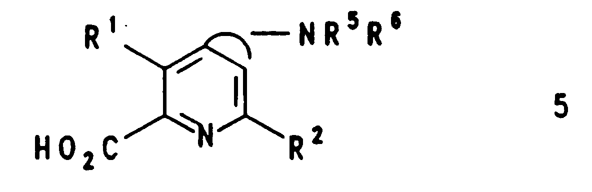 avec R⁴OH, pour aboutir à un composé de formule 3, formules dans lesquelles R¹, R², R³, R⁴, R⁵ et R⁶ sont définis dans les revendications 1 à 4 et Z représente le groupe hydroxy ou un groupe partant qui est séparable par substitution nucléophile, et représente en particulier F, Cl, Br, I ou le groupe tosylate, et éventuellement on oxyde ensuite les composé de formule 3 en leurs pyridine-N-oxydes.

7. Procédé pour la préparation des composés selon un ou plusieurs des revendications 1 à 5, dans lesquels X représente -CO-, caractérisé en ce que
I) on fait réagir un composé de formule 6 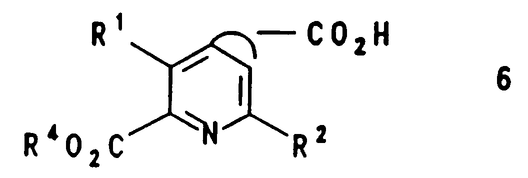 avec NHR⁵R⁶, pour aboutir à un composé de formule 8 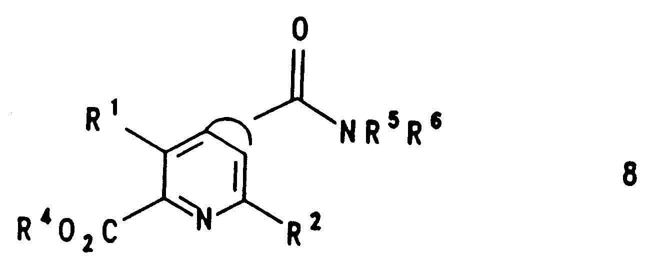 ou
II) on fait réagir un composé de formule 9 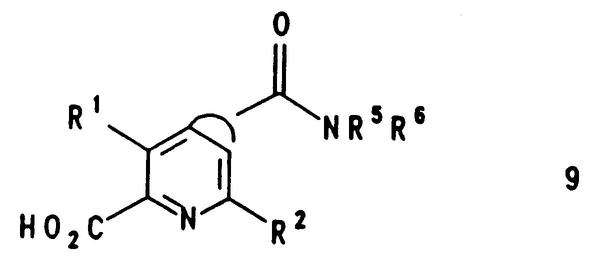 avec R⁴OH, pour aboutir à un composé de formule 8
ou
III) on fait réagir un composé de formule 10 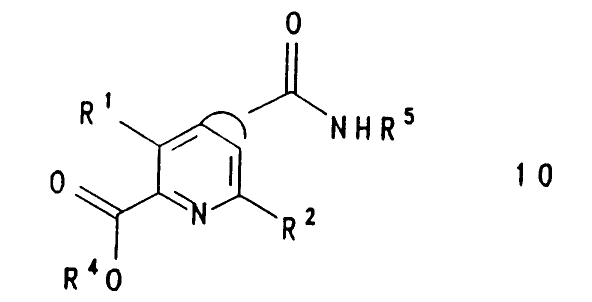 avec ZR⁶, pour aboutir à un composé de formule 8, formules dans lesquelles R¹, R², R³, R⁴, R⁵ et R⁶ sont définis dans les revendications 1 à 4, et Z représente le groupe hydroxy ou un groupe partant qui est séparable par substitution nucléophile, et représente en particulier F, Cl, Br, I ou le groupe tosylate, et éventuellement on oxyde ensuite les composé de formule 8 en leurs pyridine-N-oxydes.

8. Composés selon les revendications 1 à 4, contre des maladies de type fibrose.

9. Composés selon les revendications 1 à 4, pour utilisation en tant que fibrosuppresseurs.

10. Médicament, contenant au moins un composé de formule I et éventuellement un véhicule pharmaceutiquement acceptable.

11. Composés de formule I pour utilisation dans un procédé pour le traitement de troubles du métabolisme du collagène et de substances de type collagène.

12. Composés de formule I pour utilisation dans un procédé pour le traitement de maladies de type fibrose.

13. Procédé pour la fabrication de médicaments destinés au traitement de maladies fibreuses, caractérisé en ce que le médicament contient un composé de formule I.
